# EUROPEAN PATENT APPLICATION

(11) **EP 4 047 021 A1**
(43) Date of publication of application: **24.08.2022**
(21) Application number: 20877358.0
(22) Date of filing: 16.10.2020
(51) Int. Cl.: C07K 16/28, A61P 35/00, A61K 39/00

(54) **OX40/PD-L1 BISPECIFIC ANTIBODY**

(30) Priority: 17.10.2019 CN 201910987489; 17.10.2019 CN 201910987510
(71) Applicant: Jiangsu Alphamab Biopharmaceuticals Co., Ltd., Suzhou, Jiangsu 215125 (CN)
(72) Inventor: XU, Ting, Suzhou, Jiangsu 215125 (CN); WANG, Pilin, Suzhou, Jiangsu 215125 (CN); GUO, Kangping, Suzhou, Jiangsu 215125 (CN); JIN, Yuhao, Suzhou, Jiangsu 215125 (CN); CHEN, Ting, Suzhou, Jiangsu 215125 (CN); GAO, Li, Suzhou, Jiangsu 215125 (CN); ZHANG, Qingqing, Suzhou, Jiangsu 215125 (CN)
(74) Representative: Böhm, Brigitte
(86) International application number: PCT/CN2020/121579
(87) International publication number: WO 2021/073611

(57) **Abstract**

Provided is an isolated antigen-binding protein, wherein the drug is used for the treatment of tumor, and the isolated antigen-binding protein comprises a PD-L1 binding moiety and an OX40 binding moiety, wherein: the OX40 binding moiety is capable of recognizing and/or binding amino acid residues G70 and/or F71 in a human OX40 extracellular domain; and the PD-L1 binding moiety is capable of recognizing and/or binding amino acid residues I54, Y56, E58, Q66 and/or R113 in an N-terminal IgV domain of human PD-L1. Further provided is a use of the isolated antigen-binding protein in preparing a drug, wherein the drug is used for the treatment of tumors.

## Description

### Field of the Invention

The present application relates to the field of biomedicine, and specifically to an isolated antigen-binding protein. The isolated antigen-binding protein may comprise a PD-L1 binding moiety and an OX40 binding moiety, and can be used for inhibiting the growth of tumors or tumor cells.

### Background of the Invention

Programmed Death Ligand 1 (PD-L1) is a member of B7 family, which can be expressed in various immune cells. PD-L1 binds to Programmed Death-1 receptor (PD-1) to activate negatively regulated signaling pathways, thereby inhibiting proliferation and activity of T cells. PD1/PD-L1 are important immune checkpoints for regulating immune responses. Modulators of activity against PD1 or PD-L1 can be used, for example, in cancer immunotherapy and treatment of viral infection conditions. Therapeutic blockade of PD1/PD-L1 pathways helps to overcome the immune tolerance, and contributes to the immunity during the treatment of cancers or viral infections. OX40 is a member of tumor necrosis factor receptor superfamily, which can provide co-stimulatory signals to T cells so as to promote proliferation and survival of the cells.

The role of the immune system, especially T cells-mediated cytotoxicity, in the control of tumors is well established. More and more evidences demonstrate that T cells control the growth and survival of tumors in cancer patients, both in early and late stages of the disease. However, tumor-specific T cell responses are difficult to rise and maintain in cancer patients. The continued progress and success of cancer immunotherapies that stimulate or enhance the innate immune responses against cancers makes these therapies attractive treatment options when compared to therapies utilizing non-specific chemotherapy and/or radiation. Meanwhile, studies have shown that, for some viral, acute bacterial or fungal infections (e.g., sepsis caused by infection), after an early excessive inflammatory response, the body will enter into a subsequent persistent immunosuppressive state, which will lead to aggravation or death of the patient. Experimental results show that, for persistent immunosuppressive states occurring in sepsis, especially T cell depletion, the use of immune checkpoint inhibitors comprising anti-PD1/PDL1 antibodies can lead to effective antagonism, and they can significantly improve the survival rate of experimental animals. Therefore, combining co-stimulatory signaling with other signaling pathways of the immune system can provide an effective approach for the immunotherapy of tumors or microbial infections.

Recently, concurrent therapies with PD1/PD-L1 antibody and OX40 antibody administered intravenously alone have been reported. However, there are a number of drawbacks in these concurrent therapies, e.g., patient inconvenience, increased pain and increased difficulties in the preparation and characterization of multiple formulations. In addition, poor efficacy and safety issues have also been reported. Therefore, there is an unmet medical need for formulations which are more effective and less toxic, especially those capable of acting on multiple targets simultaneously.

### Summary of the Invention

The present application provides an isolated antigen-binding protein, comprising a PD-L1 binding moiety and an OX40 binding moiety, wherein: the OX40 binding moiety is capable of recognizing and/or binding amino acid residues G70 and/or F71 in a human OX40 extracellular domain; the PD-L1 binding moiety is capable of recognizing and/or binding amino acid residues I54, Y56, E58, Q66 and/or R113 in an N-terminal IgV domain of human PD-L1. The present application further provides a use of the isolated antigen-binding protein in preparing a drug, wherein the drug may be used for the treatment of tumor, viral infection, and/or bacterial and/or fungal infection. The present application further provides a method for enhancing immune effects in a subject in need thereof. The isolated antigen-binding protein of the present application has one or more of the following properties: (1) capable of binding to PD-L1 and OX40 on the cell surface with high affinity, and the location and manner of attachment of the PD-L1 binding moiety to the OX-40 binding moiety do not affect the ability to bind PD-L1; (2) activating T cells; (3) effectively bridging two cells expressing PDL1 and OX40 respectively; (4) having in vitro agonist activity; (5) effectively blocking the interaction between PDL1 and PD1, (5) the location and manner of attachment of the PD-L1 binding moiety to the OX-40 binding moiety do not affect the ADCC activity; (6) activating PBMCs, (7) having a half life of greater than 60 h, and (7) inhibiting the growth of tumors.

In one aspect, the present application provides an isolated antigen-binding protein, comprising a PD-L1 binding moiety and an OX40 binding moiety, wherein: the OX40 binding moiety is capable of recognizing and/or binding amino acid residues G70 and/or F71 in a human OX40 extracellular domain, wherein the human OX40 extracellular domain comprises an amino acid sequence as set forth in SEQ ID NO: 1; the PD-L1 binding moiety is capable of recognizing and/or binding amino acid residues I54, Y56, E58, Q66 and/or R113 in an N-terminal IgV domain of human PD-L1, and wherein the N-terminal IgV domain of human PD-L1 comprises an amino acid sequence as set forth in SEQ ID NO: 2.

In some embodiments, the PD-L1 binding moiety is also capable of binding amino acid residues D61, N63, V68, M115, S117, Y123 and/or R125 in an N-terminal IgV domain of human PD-L1, and wherein the N-terminal IgV domain of human PD-L1 comprises an amino acid sequence as set forth in SEQ ID NO: 2.

In some embodiments, the PD-L1 binding moiety is capable of binding a conformational epitope of the N-terminal IgV domain of human PD-L1, the conformational epitope comprises amino acid residues I54, Y56, E58, Q66 and R113 in the N-terminal IgV domain of human PD-L1, and wherein the N-terminal IgV domain of human PD-L1 comprises an amino acid sequence as set forth in SEQ ID NO: 2.

In some embodiments, the PD-L1 binding moiety is capable of binding a conformational epitope of the N-terminal IgV domain of human PD-L1, the conformational epitope comprises amino acid residues I54, Y56, E58, Q66, R113, D61, N63, V68, M115, S117, Y123 and R125 in the N-terminal IgV domain of human PD-L1, and wherein the N-terminal IgV domain of human PD-L1 comprises an amino acid sequence as set forth in SEQ ID NO: 2.

In some embodiments, the OX40 binding moiety is capable of binding a conformational epitope of the human OX40 extracellular domain, the conformational epitope comprises amino acid residues G70 and F71 in the human OX40 extracellular domain, and wherein the human OX40 extracellular domain comprises an amino acid sequence as set forth in SEQ ID NO: 1.

In some embodiments, the PD-L1 binding moiety is capable of competing with a PD-L1 reference antibody for binding to human PD-L1, wherein the PD-L1 reference antibody comprises a heavy chain variable domain VH2, and the VH2 comprises an H2CDR3, the H2CDR3 comprises an amino acid sequence as set forth in SEQ ID NO: 62.

In some embodiments, wherein the H2CDR3 of the PD-L1 reference antibody comprises an amino acid sequence as set forth in any one of SEQ ID NOs: 39-45.

In some embodiments, wherein the VH2 of the PD-L1 reference antibody comprises an H2CDR1, the H2CDR1 comprises an amino acid sequence as set forth in SEQ ID NO: 60.

In some embodiments, wherein the H2CDR1 of the PD-L1 reference antibody comprises an amino acid sequence as set forth in any one of SEQ ID NOs: 21-29.

In some embodiments, wherein the VH2 of the PD-L1 reference antibody comprises an H2CDR2, the H2CDR2 comprises an amino acid sequence as set forth in SEQ ID NO: 61.

In some embodiments, wherein the H2CDR2 of the PD-L1 reference antibody comprises an amino acid sequence as set forth in any one of SEQ ID NOs: 30-38.

In some embodiments, wherein the VH2 of the PD-L1 reference antibody comprises an amino acid sequence as set forth in SEQ ID NO: 63.

In some embodiments, wherein the VH2 of the PD-L1 reference antibody comprises an amino acid sequence as set forth in any one of SEQ ID NOs: 46-59.

In some embodiments, wherein the OX40 binding moiety competes with an OX40 reference antibody for binding to human OX40, wherein the OX40 reference antibody comprises a heavy chain H1CDR3, the heavy chain H1CDR3 comprises an amino acid sequence as set forth in SEQ ID NO: 5.

In some embodiments, wherein the OX40 reference antibody comprises a heavy chain H1CDR1, the heavy chain H1CDR1 comprises an amino acid sequence as set forth in SEQ ID NO: 3.

In some embodiments, wherein the OX40 reference antibody comprises a heavy chain H1CDR2, the heavy chain H1CDR2 comprises an amino acid sequence as set forth in SEQ ID NO: 4.

In some embodiments, wherein the OX40 reference antibody comprises a heavy chain variable domain VH1, the VH1 comprises an amino acid sequence as set forth in SEQ ID NO: 6.

In some embodiments, wherein the OX40 reference antibody comprises a light chain L1CDR1, the L1CDR1 comprises an amino acid sequence as set forth in SEQ ID NO: 9.

In some embodiments, wherein the OX40 reference antibody comprises a light chain L1CDR2, the L1CDR2 comprises an amino acid sequence as set forth in SEQ ID NO: 10.

In some embodiments, wherein the OX40 reference antibody comprises a light chain L1CDR3, the L1CDR3 comprises an amino acid sequence as set forth in SEQ ID NO: 11.

In some embodiments, wherein the OX40 reference antibody comprises a light chain variable domain VL1, the VL1 comprises an amino acid sequence as set forth in SEQ ID NO: 12.

In some embodiments, wherein the PD-L1 binding moiety comprises a heavy chain variable domain VH2, and the VH2 comprises an H2CDR3, and the H2CDR3 comprises an amino acid sequence as set forth in SEQ ID NO: 62.

In some embodiments, wherein the H2CDR3 of the PD-L1 binding moiety comprises an amino acid sequence as set forth in any one of SEQ ID NOs: 39-45.

In some embodiments, wherein the VH2 of the PD-L1 binding moiety comprises an H2CDR1, the H2CDR1 comprises an amino acid sequence as set forth in SEQ ID NO: 60.

In some embodiments, wherein the H2CDR1 of the PD-L1 binding moiety comprises an amino acid sequence as set forth in any one of SEQ ID NOs: 21-29.

In some embodiments, wherein the VH2 of the PD-L1 binding moiety comprises an H2CDR2, the H2CDR2 comprises an amino acid sequence as set forth in SEQ ID NO: 61.

In some embodiments, wherein the H2CDR2 of the PD-L1 binding moiety comprises an amino acid sequence as set forth in any one of SEQ ID NOs: 30-38.

In some embodiments, wherein the VH2 of the PD-L1 binding moiety comprises an amino acid sequence as set forth in SEQ ID NO: 63.

In some embodiments, wherein the VH2 of the PD-L1 binding moiety comprises an amino acid sequence as set forth in any one of SEQ ID NOs: 46-59.

In some embodiments, wherein the PD-L1 binding moiety is a single-domain antibody.

In some embodiments, wherein the PD-L1 binding moiety comprises an amino acid sequence as set forth in SEQ ID NO: 63.

In some embodiments, wherein the PD-L1 binding moiety comprises an amino acid sequence as set forth in any one of SEQ ID NOs: 46-59.

In some embodiments, wherein the OX40 binding moiety comprises a heavy chain H1CDR3, the heavy chain H1CDR3 comprises an amino acid sequence as set forth in SEQ ID NO: 5.

In some embodiments, wherein the OX40 binding moiety comprises a heavy chain H1CDR1, the heavy chain H1CDR1 comprises an amino acid sequence as set forth in SEQ ID NO: 3.

In some embodiments, wherein the OX40 binding moiety comprises a heavy chain H1CDR2, the heavy chain H1CDR2 comprises an amino acid sequence as set forth in SEQ ID NO: 4.

In some embodiments, wherein the OX40 binding moiety comprises a heavy chain variable domain VH1, the VH1 comprises an amino acid sequence as set forth in SEQ ID NO: 6.

In some embodiments, wherein the OX40 binding moiety comprises a light chain L1CDR1, the L1CDR1 comprises an amino acid sequence as set forth in SEQ ID NO: 9.

In some embodiments, wherein the OX40 binding moiety comprises a light chain L1CDR2, the L1CDR2 comprises an amino acid sequence as set forth in SEQ ID NO: 10.

In some embodiments, wherein the OX40 binding moiety comprises a light chain L1CDR3, the L1CDR3 comprises an amino acid sequence as set forth in SEQ ID NO: 11.

In some embodiments, wherein the OX40 binding moiety comprises a light chain variable domain VL1, the VL1 comprises an amino acid sequence as set forth in SEQ ID NO: 12.

In some embodiments, wherein the OX40 binding moiety comprises a light chain constant region, the light chain constant region is derived from Igκ or Igλ.

In some embodiments, wherein the light chain constant region comprises an amino acid sequence as set forth in SEQ ID NO: 13.

In some embodiments, wherein the OX40 binding moiety comprises a Fab domain.

In some embodiments, the isolated antigen-binding protein further comprises a heavy chain constant region, and the heavy chain constant region is derived from IgG.

In some embodiments, wherein the heavy chain constant region is derived from human IgG1 or human IgG4.

In some embodiments, wherein the heavy chain constant region comprises an amino acid sequence as set forth in SEQ ID NO: 7.

In some embodiments, wherein the PD-L1 binding moiety is located at N-terminus of the OX40 binding moiety.

In some embodiments, wherein the PD-L1 binding moiety is located at C-terminus of the OX40 binding moiety.

In some embodiments, wherein the OX40 binding moiety comprises an antibody heavy chain H1, and the PD-L1 binding moiety is fused with N-terminus of the H1 directly or indirectly.

In some embodiments, wherein the OX40 binding moiety comprises an antibody heavy chain H1, and the PD-L1 binding moiety is fused with C-terminus of the H1 directly or indirectly.

In some embodiments, the isolated antigen-binding protein comprises a first polypeptide chain and a second polypeptide chain, wherein, the first polypeptide chain comprises the light chain variable domain VL1 of the OX40 binding moiety, the second polypeptide chain comprises the heavy chain variable domain VH1 of the OX40 binding moiety and the PD-L1 binding moiety.

In some embodiments, wherein the first polypeptide chain comprises a light chain L1 of the OX40 binding moiety.

In some embodiments, wherein the first polypeptide chain comprises an amino acid sequence as set forth in SEQ ID NO: 16.

In some embodiments, wherein the second polypeptide chain comprises the heavy chain H1 of the OX40 binding moiety and the PD-L1 binding moiety.

In some embodiments, wherein in the second polypeptide chain, the N-terminus of the heavy chain H1 is linked to the C-terminus of the PD-L1 binding moiety directly or indirectly.

In some embodiments, wherein in the second polypeptide chain, the C-terminus of the heavy chain H1 is linked to the N-terminus of the PD-L1 binding moiety directly or indirectly.

In some embodiments, wherein the heavy chain H1 is linked to the PD-L1 binding moiety through a linker.

In some embodiments, wherein the linker is a peptide linker.

In some embodiments, wherein the linker comprises an amino acid sequence as set forth in any one of SEQ ID NOs: 14-15.

In some embodiments, wherein the second polypeptide chain comprises an amino acid sequence as set forth in any one of SEQ ID NOs: 17-20.

In some embodiments, the isolated antigen-binding protein comprises two first polypeptide chains and two second polypeptide chains.

In some embodiments, the isolated antigen-binding protein has a symmetrical structure.

In some embodiments, the isolated antigen-binding protein is capable of blocking the binding of PD-1 and PD-L1.

In some embodiments, the isolated antigen-binding protein is capable of blocking the binding of PD-L1 and CD80.

In some embodiments, the isolated antigen-binding protein has an OX40 agonist activity.

In another aspect, the present application provides an immunoconjugate, comprising the isolated antigen-binding protein.

In another aspect, the present application provides one or more isolated nucleic acid molecules, encoding the isolated antigen-binding protein or the immunoconjugate.

In another aspect, the present application provides a vector, comprising the one or more isolated nucleic acid molecules.

In another aspect, the present application provides a cell, comprising the nucleic acid molecules or the vector.

In another aspect, the present application provides a pharmaceutical composition, comprising the isolated antigen-binding protein, the immunoconjugate, the isolated nucleic acid molecules, thevector, and/or the cell, and optionally a pharmaceutically acceptable carrier.

In another aspect, the present application provides a use of the isolated antigen-binding protein or the immunoconjugate in preparing a drug, wherein the drug is used for the treatment of tumor.

In some embodiments, the tumor is selected from a group consisting of: blood tumor and solid tumor.

In another aspect, the present application provides a use of the isolated antigen-binding protein or the, the drug is used for the treatment of viral infection in a subject.

In some embodiments, the viral infection comprises chronic viral infection.

In some embodiments, the viral infection is infection caused by a virus selected from a group consisting of: hepatitis B virus (HBV), influenza virus and EBV.

In another aspect, the present application provides a use of the isolated antigen-binding protein or the immunoconjugate in preparing a drug, the drug is used for the treatment of bacterial and/or fungal infection in a subject.

In some embodiments, the bacterial and/or fungal infection comprises sepsis.

In the present application, the drug used for the treatment of tumor, the drug used for the treatment of viral infection in a subject and/or the drug used for the treatment of bacterial and/or fungal infection in a subject increases the production of cytokines in the subject.

In some embodiments, the drug increases the production of cytokines from CD4⁺ T cells and/or CD8⁺ T cells in the subject.

In some embodiments, the cytokines comprise interleukins and/or interferons.

In some embodiments, the cytokines comprise IL2, IL21 and/or IFN-γ.

In some embodiments, the drug enhances immune responses of T cells in the subject.

In some embodiments, the immune response comprises increased production of cytokines.

In some embodiments, the T cells comprise CD4⁺T cells and/or CD8⁺T cells.

In some embodiments, the drug upregulates the expression level of transcription factors in the subject.

In some embodiments, the transcription factors comprise T-bet and/or Bcl6.

In some embodiments, the subject is an HBeAg negative subject.

In another aspect, the present application provides a pharmaceutical composition for treating tumors, comprising the isolated antigen-binding protein and optionally a pharmaceutically acceptable carrier.

In another aspect, the present application provides a pharmaceutical composition for treating viral infection, comprising the isolated antigen-binding protein and optionally a pharmaceutically acceptable carrier.

In another aspect, the present application provides a pharmaceutical composition for treating bacterial and/or fungal infection, comprising the isolated antigen-binding protein and optionally a pharmaceutically acceptable carrier.

In another aspect, the present application provides a method for treating tumors, comprising a step of: administering the isolated antigen-binding protein.

In another aspect, the present application provides a method for treating viral infection, comprising a step of: administering the isolated antigen-binding protein to a subject in need thereof.

In another aspect, the present application provides a method for treating bacterial and/or fungal infection, comprising a step of: administering the isolated antigen-binding protein to a subject in need thereof.

In another aspect, the present application provides a method for enhancing immune effects in a subject in need thereof, comprising a step of: administering the isolated antigen-binding protein of the present application to the subject.

Those skilled in the art can easily perceive other aspects and advantages of the present application from the detailed description below. In the following detailed description, only exemplary embodiments of the present application are shown and described. As those skilled in the art will recognize, the content of the present application enables those skilled in the art to make changes to the disclosed specific embodiments without departing from the spirit and scope of the invention involved in the present application. Correspondingly, the drawings and descriptions in the specification of the present application are merely exemplary, rather than restrictive.

### Brief Description of the Drawings

The specific features of the invention involved in the present application are shown in the appended claims. The characteristics and advantages of the invention involved in the present application can be better understood by referring to the exemplary embodiments and the accompanying drawings described in detail below. A brief description of the drawings is as follows:
Fig. 1 shows the structure of the isolated antigen-binding protein of the present application;
Fig. 2 shows the binding activity of the isolated antigen-binding protein of the present application to PD-L1 on the cell surface;
Fig. 3 shows the binding activity of the isolated antigen-binding protein of the present application to OX40 on the cell surface;
Figs. 4A-4B show the binding activity of the isolated antigen-binding protein of the present application to activated T cells;
Fig. 5 shows the cell-bridging assay of the isolated antigen-binding protein of the present application;
Fig. 6 shows the test results of OX40 receptor agonist cells;
Fig. 7 shows the blocking activity of the isolated antigen-binding protein of the present application to PD1 and PD-L1;
Fig. 8 shows the results of the isolated antigen-binding protein of the present application binding to soluble PD-L1 protein molecules;
Figs. 9A-9B show that the isolated antigen-binding protein of the present application has ADCC activity;
Figs. 10A-10D show the results of the isolated antigen-binding protein of the present application stimulating cytokine secretion from PBMCs;
Figs. 11A-11D show the results of the isolated antigen-binding protein of the present application inhibiting the growth of tumors in an hPD-L1-MC38 mouse model;
Figs. 12A-12F show the results of the isolated antigen-binding protein of the present application inhibiting the growth of tumors in a wild-type MC38 mouse model.

### Detailed Description of the Embodiments

The implementation of the present application will be illustrated in the following specific examples, and other advantages and effects of the present application will be easily known by those familiar with this technology from the content disclosed in the specification.

The following is a further description of the present application: In the present invention, unless otherwise specified, the scientific and technical terms used herein have the meanings commonly understood by those skilled in the art. In addition, protein and nucleic acid chemistry, molecular biology, cell and tissue culture, microbiology, immunology related terms and laboratory procedures used herein are all terms and routine procedures widely used in the corresponding fields. At the same time, in order to better understand the present invention, definitions and explanations of related terms are provided below.

In the present application, the term "isolated" generally refers to artificially obtained from the natural state. If a certain "isolated" substance or component occurs in nature, it may be due to a change in its natural environment, or the substance may be isolated from its natural environment, or both. For example, a certain unisolated polynucleotide or polypeptide naturally exists in a living animal, and the same polynucleotide or polypeptide with a high purity isolated from this natural state is called isolated. The term "isolated" does not exclude the mixing of artificial or synthetic substances, nor does it exclude the presence of other impure substances that do not affect the activity of the substance.

In the present application, the term "isolated antigen-binding protein" generally refers to a protein with antigen-binding ability that is artificially obtained from the natural state. The "isolated antigen-binding protein" may comprise one or more antigen-binding moieties. For example, in the present application, the "isolated antigen-binding protein" may comprise a PD-L1 binding moiety and an OX40 binding moiety.

In the present application, the term "PD-1" generally refers to Programmed Death 1 receptor (also referred to as CD279), functional variants thereof and/or functional fragments thereof. PD-1 is usually expressed on T cells, B cells, natural killer T cells, activated monocytes and dendritic cells (DC). PD-1 can bind to its ligands PD-L1 and PD-L2. For example, the term "PD-1" may comprise polypeptides or fragments thereof having at least about 85% amino acid sequence identity with NCBI Accession No. P42081 and specifically binding PD-L1. Also comprised within the definition of PD-1 are variants that differ from the amino acid sequence of naturally occurring PD-1 but retain the ability to specifically bind PD-L1. Further comprised within the definition of PD-1 are variants that enhance the biological activity of PD-L1. The sequence of PD-1 is known in the art, and provided in, e.g., GenBank Accession No. Q15116.3. The term "PD-1" as used herein comprises human PD-1 (hPD-1), variants, isomers and species homologues of hPD-1, as well as analogues with at least one common hPD-1-containing epitope. For example, the term "PD-1" also covers PD-1 from other species, such as other mammals (e.g., rats, mice, rabbits, non-human primates, pigs or cows). The complete hPD-1 sequence can be found in GenBank Accession No. Q15116.3.

In the present application, the term "PD-L1" generally refers to Programmed death ligand 1 protein, functional variants thereof and/or functional fragments thereof. PD-L1 is also referred to as cluster of differentiation 274 (CD274) or B7 homologue 1 (B7-H1), and it is a protein encoded by the CD274 gene (in human). PD-L1 binds its receptors, e.g., Programmed death 1 (PD-1), and the PD-1 is expressed in activated T cells, B cells and macrophages (Ishida et al., 1992 EMBO J, 11:3887-3395; Okazaki et al., Autoimmune dilated cardiomyopathy in PD-1 receptor-deficient mice. Science, 2001; 291: 319-22). The complexation of PD-L1 and PD-1 exerts immunosuppressive effects by inhibiting T cell proliferation and production of cytokines IL-2 and IFN-γ (Freeman et al., Engagement of PD-1 immunoinhibitory receptor by a novel B7 family member leads to negative regulation of lymphocyte activation, J. Exp.Med. 2000, 192:1027-1034; Carter et al., PD-1: PD-L inhibitory pathway affects both CD4(+) and CD8(+) T cells and is overcome by IL-2. Eur. J. Immunol. 2002, 32:634-643). Also comprised within the definition of PD-L1 are variants that differ from the amino acid sequence of naturally occurring PD-L1 but retain the ability to specifically bind the receptor PD-1. Further comprised within the definition of PD-L1 are variants that enhance the biological activity of PD-1. The sequence of PD-L1 is known in the art, and provided in, e.g., GenBank Accession No. 29126. The term "PD-L1" as used herein comprises human PD-L1 (hPD-L1), variants, isomers and species homologues of hPD-L1 as well as analogues with at least one common hPD-L1-containing epitope. For example, the term "PD-L1" also covers PD-L1 from other species, such as other mammals (e.g., rats, mice, rabbits, non-human primates, pigs or cows). The complete hPD-L1 sequence can be found in GenBank Accession No. 29126.

In the present application, the term "OX40" generally refers to tumor necrosis factor receptors primarily present on activated CD4+ and CD8+ T cells, regulatory T (Treg) cells and natural killer (NK) cells (Croft et al., 2009, Immunol Rev 229:173-91). The aggregation of OX40 can generate efficient cell signaling events within T cells. OX40 signaling on activated CD4+ and CD8+ T cells leads to enhanced production of cytokines, release of granzyme and perforin, and expansion of effector and memory T cell pools (Jensen et al., 2010, Semin Oncol, 37:524-32). Moreover, OX40 signaling on Treg cells inhibits the expansion of Treg, stops the induction of Treg and blocks the inhibitory function of Treg (Voo et al., 2013, J Immunol. 191:3641-50; Vu et al., 2007, Blood. 110: 2501-10). For example, the term "OX40" may comprise polypeptides or fragments thereof having at least 80% amino acid sequence identity with UniProt Accession No. P43489.1 and specifically binding OX40L. The term "OX40" in the present application comprises human OX40 (hOX40), variants, isoforms and interspecies homologues of hOX40, and analogues thereof having at least one common epitope with hOX40.

In the present application, the term "N-terminal IgV domain of human PD-L1" generally refers to the extracellular domain located at the N-terminus of human PD-L1. The term "N-terminal IgV domain of human PD-L1" may also refer to an epitope within the domain. The N-terminal IgV domain of human PD-L1 protein (comprising signal peptides) may comprise an amino acid sequence as set forth in SEQ ID NO: 2.

In the present application, the term "extracellular domain" generally refers to the extracellular domain of a protein. For example, the extracellular domain of OX40 protein may generally be in an OX40 polypeptide form substantially free of transmembrane and cytoplasmic domains. Where, the definition of transmembrane is identified according to the conventional criteria used in the field for identifying the types of hydrophobic domains. The exact boundaries of transmembrane domains may be different, but most of them are similar. For example, the extracellular domain of the OX40 protein may contain about 5 (or 4, 3, 2 or 1) amino acids on either side of the boundary of the transmembrane domain or the extracellular domain. For example, the extracellular domain of the OX40 protein may carry associated signal peptides. In the present application, the term "human OX40 extracellular domain" generally refers to the extracellular domain located on human OX40. For example, the amino acid sequence of the extracellular domain of human OX40 protein may be shown in SEQ ID NO: 43 (P43489, positions 1 to 170). The term "human OX40 extracellular domain" may also refer to an epitope within the domain. For example, the extracellular domain of human OX40 protein (comprising signal peptides) may comprise an amino acid sequence as set forth in SEQ ID NO: 1.

In the present application, the term "extracellular domain" generally refers to a part of proteins (e.g., membrane proteins, such as receptors) protruding from the organelle and/or the outer membrane of the cell. If a polypeptide chain crosses the bilayer several times, the extracellular domain comprises a ring wound through the membrane. The extracellular domain can recognize and respond to specific antibodies.

In the present application, the term "epitope" generally refers to the site of antibody binding on the antigen. Epitopes can be formed by continuous amino acids (linear epitopes) or non-continuous amino acids that are spatially adjacent due to the tertiary folding of the protein (conformational epitopes). When exposed to a denaturing solvent, the epitopes formed by continuous amino acids are usually retained, but when treated with a denaturing solvent, the epitopes formed by tertiary folding are usually lost. Epitopes generally comprise at least 3, usually more, at least 5 or 8-10 amino acids in a unique spatial conformation. The methods for determining the spatial conformation of an epitope comprise, for example, X-ray crystal diffraction and two-dimensional NMR. See, e.g., Epitope Mapping Protocols in Methods in Molecular Biology, Vol. 66, Glenn E. Morris, Ed (1996).

In the present application, the term "conformational epitope" generally refers to non-continuous amino acid residues of an antigen (such as, PD-L1 antigen) that are spatially adjacent due to the tertiary folding of the protein. When the polypeptide chain is folded to form a natural protein, these non-continuous amino acid residues can accumulate on the surface. Conformational epitopes comprise, but not limited to, functional epitopes.

In the present application, the term "variable domain" generally refers to the amino-terminal domain of an antibody heavy chain or light chain. The heavy chain and light chain variable domains may be referred to as "VH" and "VL", respectively. These domains are usually the most variable part of the antibody (relative to other antibodies of the same type), and contain antigen-binding sites.

In the present application, the term "variable" generally refers to the fact that there is a great difference in the sequences of some segments of the variable domains between antibodies. V domain mediates the binding of antigen and determines the specificity of a specific antibody to its specific antigen. However, variability is not evenly distributed throughout the variable domain. Instead, it is concentrated in three segments called hypervariable regions (CDRs or HVRs) in the light chain and heavy chain variable domains. The more highly conserved part of the variable domain is referred to as framework region (FR). The variable domains of natural heavy chains and light chains each comprises four FR regions, most of which are in β-folded configuration in which they are connected by three CDRs to form a circular connection and in some cases form a part of a β-folded structure. The CDRs in each chain are held together closely by the FR region, and promote the formation of the antigen-binding site of the antibody together with the CDRs from another chain (see Kabat et al, Sequences of Immunological Interest, Fifth Edition, National Institute of Health, Bethesda, Md. (1991)). The constant domains are not directly involved in the binding of antibodies to antigens, but exhibit various effector functions, for example, antibodies are involved in the antibody-dependent cytotoxicity.

In the present application, the term "CD80" generally refers to a ligand of CD28/CTLA4 (also referred to as B7.1), functional variants thereof and/or functional fragments thereof. CD80 is generally expressed on specialized antigen-presenting cells (APCs). For example, the term "CD80" may comprise polypeptides or fragments thereof having at least about 85% amino acid sequence identity with NCBI Accession No. P33681 and specifically binding CTLA4. Also comprised within the definition of CD80 are variants that differ from the amino acid sequence of naturally occurring CD80 but retain the ability to specifically bind CTLA4. Further comprised within the definition of CD80 are variants that enhance the biological activity of CTLA4. The sequence of CD80 is known in the art, and provided in, e.g., GenBank Accession No. P33681. The term " CD80" as used herein comprises human CD80 (hCD80), variants, isomers and species homologues of hCD80, as well as analogues with at least one common hCD80-containing epitope. For example, the term "CD80" also covers CD80 from other species, such as other mammals (e.g., rats, mice, rabbits, non-human primates, pigs or cows). The complete hCD80 sequence can be found in GenBank Accession No. P33681.

In the present application, the term "antibody" generally refers to an immunoglobulin or a fragment or derivative thereof, encompassing any polypeptides that comprise an antigen binding site, no matter whether it is produced in vitro o in vivo. The term comprises, but is not limited to, polyclonal, monoclonal, mono-specific, multi-specific, non-specific, humanized, single-chain, chimeric, synthetic, recombinant, hybrid, mutated and grafted antibodies. Unless otherwise modified by a term "complete", as in "complete antibody", for the purposes of the present invention, the term "antibody" also comprises antibody fragments, such as Fab, F(ab')₂, Fv, scFv, Fd, dAb and other antibody fragments that retain the antigen binding functions (i.e., specifically binding to, e.g., OX40 or PD-LI). In general, such fragments should comprise antigen-binding domains. The basic 4-chain antibody unit is a heterotetrameric glycoprotein composed of two identical light (L) chains and two identical heavy (H) chains. The IgM antibody is composed of 5 basic heterotetrameric units and another polypeptide called J chain, and contains 10 antigen-binding sites; while the IgA antibody comprises 2-5 basic 4-chain units that can be polymerized with the J chain to form a multivalent combination. In terms of IgG, the 4-chain unit is generally about 150,000 Daltons. Each L chain is linked to the H chain through a covalent disulfide bond, while two H chains are linked to each other through one or more disulfide bonds depending on the isotype of the H chain. Each H and L chain also has regularly spaced intra-chain disulfide bridges. Each H chain has a variable domain (VH) at the N-terminus, which is followed by three constant domains (CHs) for each of α and γ chains, and followed by four CH domains for µ and ε isotypes. Each L chain has a variable domain (VL) at the N-terminus, and has a constant domain at the other terminus. VL corresponds to VH, and CL corresponds to the first constant domain (CH1) of the heavy chain. Specific amino acid residues are considered to form an interface between the light chain and heavy chain variable domains. VH is paired with VL to form a single antigen-binding site. For the structures and properties of different kinds of antibodies, see for example Basic and Clinical Immunology, 8th Edition, Daniel P. Sties, Abba I. Terr and Tristram G. Parsolw (eds), Appleton & Lange, Norwalk, Conn., 1994, Page 71 and Chapter 6. L chains from any vertebrate species can be classified into one of two distinct types based on the amino acid sequence of their constant domains, called kappa and lambda. Depending on the amino acid sequence of its heavy chain (CH) constant domain, immunoglobulin can be classified into different types or isotypes. There are five types of immunoglobulin: IgA, IgD, IgE, IgG and IgM, which have heavy chains named α, δ, ε, γ and µ, respectively. Based on the relatively small differences in terms of CH sequence and function, the γ and α types are further divided into subtypes. For example, human expresses the following subtypes: IgG1, IgG2A, IgG2B, IgG3, IgG4, IgA1 and IgK1.

In the present application, the term "CDR" generally refers to an area of an antibody variable domain, of which the sequence is highly variable and/or forms a structure-defining ring. In general, an antibody comprises six CDRs; three in VH (HCDR1, HCDR2, HCDR3), and three in VL (LCDR1, LCDR2, LCDR3). In native antibodies, HCDR3 and LCDR3 exhibit the most diversity of the six CDRs, and in particular, HCDR3 is considered to play a special role in conferring a fine specificity to the antibody. See, for example, Xu et al, Immunity 13:37-45 (2000); Johnson and Wu, in Methods in Molecular Biology 248:1-25 (Lo, ed., Human Press, Totowa, N.J., 2003). In fact, naturally occurring camel antibodies only composed of heavy chains function normally and are stable in the absence of light chains. See, for example, Hamers-Casterman et al., Nature 363:446-448 (1993); Sheriff et al, Nature Struct. Biol. 3:733-736 (1996). For example, in the present application, the OX40 binding moiety may comprise a heavy chain variable domain VH1 which may comprise 3 CDRs (e.g., H1CDR1, H1CDR2 and H1CDR3), and it may also comprise a light chain variable domain VL1 which may comprise 3 CDRs (e.g., L1CDR1, L1CDR2 and L1CDR3). Further for example, in the present application, the PD-L1 binding moiety may comprise a heavy chain variable domain VH2 which may comprise 3 CDRs (e.g., H2CDR1, H2CDR2 and H2CDR3).

In this field, the CDRs of an antibody can be classified by many methods, for example: 1) Kabat definition rule based on sequence variability (Wu and Kabat, J Exp Med 132:211-50, 1970; Kabat et al., Sequences of Proteins of Immunological Interest, Ed. 5, Public Health Service, National Institutes of Health, Bethesda, Md., 1991), 2) Chothia definition rule based on the location of the structural ring region (A1-Lazikani et al., J Mol Biol 273:927-48, 1997), 3) AbM definition rule that weigh the above two rules using an AbM antibody model software of Oxford Molecular, 4) Contact definition rule based on the crystal structure analysis of the obtained complex. These methods for labelling CDRs can be summarized in Table 1 below.

**Table 1 CDR Classification Method**

| | CCG Definition | Kabat Definition | AbM Definition | Chothia Definition | Contact Definition |
|---|---|---|---|---|---|
| Light chain CDR1 | L24-L34 | L24-L34 | L24-L34 | L24-L34 | L30-L36 |
| Light chain CDR2 | L50-L56 | L50-L56 | L50-L56 | L50-L56 | L45-L55 |
| Light chain CDR3 | L89-L97 | L89-L97 | L89-L97 | L89-L97 | L89-L96 |
| Heavy chain CDR1 | H26-H35 | H31-H35 | H26-H35 | H26-H32 | H30-H35 |
| Heavy chain CDR2 | H50-H65 | H50-H65 | H50-H58 | H52-H56 | H47-H58 |
| Heavy chain CDR3 | H95-H102 | H95-H102 | H95-H102 | H95-H102 | H93-H101 |

where, the position numbers of amino acid residues of Laa-Lbb are obtained following the Kabat numbering system.

In the present application, the term "competitive binding" generally refers to that an antibody or a fragment thereof interferes with the ability of another antibody (e.g., reference antibody) to directly or indirectly bind to a target/antigen (e.g., may be PD-L1 or OX40, respectively) through allosterically modulating the another antibody. For example, in the present application, the PD-L1 binding moiety is capable of competing with a PD-L1 reference antibody for binding to human PD-L1. Further for example, the OX40 binding moiety competes with an OX40 reference antibody for binding to human OX40. In addition, an antibody or a fragment thereof can interfere with the extent to which another antibody or fragment thereof binds to a target. Therefore, no matter whether it can be regarded as the blocking or competition according to the present invention, it can be determined by using a competitive binding assay. A particularly suitable quantitative competition assay uses a FACS-based or AlphaScreen-based method to measure the competition between a labeled (for example, His-labeled, biotinylated, or radiolabeled) antibody or a fragment thereof and another antibody or a fragment thereof in terms of binding to a target. Generally, a competitive antibody or a fragment thereof is, for example, one of the following: binding to a target in a competition test, so that during the test and in the presence of a second antibody or a fragment thereof, the recorded substitution of the isolated antigen-binding protein of the present invention reaches up to 100% of the maximum theoretical substitution obtained from the detected potential blocking antibody or fragment thereof present in a given amount (e.g., being substituted by a cold (e.g., unlabeled) antibody or fragment thereof that needs to be blocked) (for example, in the FACS-based competition test). For example, the competitive antibody or the fragment thereof has 10% to 100%, for example, 50% to 100% of the recorded substitution.

In the present application, the term "single-domain antibody", commonly also known as nanobody or heavy chain antibody (hcAb), is an antibody isolated from the serum of camelid and shark, which has only one heavy chain variable domain (VHH). The individually cloned and expressed VHH region has comparable structural stability and equivalent antigen-binding activity to the original heavy chain antibody, and VHH is the minimum unit that is currently known to bind the target antigen. For example, in the present application, the PD-L1 binding moiety may be a single-domain antibody.

In the present application, the term "Fab domain" generally refers to a region comprising a heavy chain variable domain, a light chain variable domain, a constant domain of light chain and a first constant domain of heavy chain (CH1). For example, in the present application, the OX40 binding moiety may comprise a Fab domain.

In the present application, the term "fusion" generally refers to the covalent bonding between two polypeptides. The polypeptides are typically linked through peptide bonds, linked to each other directly or bound by amino acid linkers. Optionally, the peptides can be bound via non-peptide covalent bonds known to those skilled in the art.

In the present application, the term "polypeptide chain" generally refers to a macromolecule comprising two or more covalently linked peptides. The peptides within a polypeptide chain can be linked to each other through a peptide bond. Each polypeptide chain may comprise one N-terminus or amino terminus and one C-terminus or carboxyl terminus.

In the present application, the term "linker" generally refers to a synthetic amino acid sequence that joins or links two polypeptide sequences (e.g., for linking two polypeptide domains). The linker can link two amino acid sequences via a peptide bond. In some embodiments, the linker of the present invention links a biologically active moiety to a second moiety in a linear sequence. For example, the peptide linker may be nonimmunogenic and flexible, for example, a peptide linker comprising serine and glycine sequences or Ala-Ala-Ala repeats. According to the specific structure of the isolated antigen-binding protein, a peptide linker may comprise, for example, 3-30 (e.g., at least 3, at least 4, at least 5, at least 6, at least 7, at least 8, at least 9, at least 10, at least 11, at least 12, at least 13, at least 14, at least 15, at least 16, at least 17, at least 18, at least 19, at least 20, at least 21, at least 22, at least 23, at least 24, at least 25, at least 26, at least 27, at least 28, at least 29, at least 30) amino acid residues.

In the present application, the term "block" generally refers to inhibiting or reducing the binding activity between a molecule and its specific binding partner (e.g, between a ligand and its specific receptor). For example, the isolated antigen-binding protein of the present application can block the interaction between PD-L1 and its receptor PD-1, thereby blocking PD-1 signaling pathways so as to restore the functional response of T cells from dysfunctional state to antigen-stimulated state. Further for example, the isolated antigen-binding protein of the present application can block the binding of PD-L1 and CD80, thereby blocking PD-1 signaling pathways so as to restore the functional response of T cells form dysfunctional state to antigen-stimulated state.

In the present application, the term "agonist" generally refers to a substance or reagent capable of activating a target molecule and/or enhancing its activity. For example, the "agonist" in the present application may be an agonistic antibody or other substances, and the agonist can convert the target molecule from an inactive state to an active state, or from a low active state to a high active state by directly or indirectly binding to the target molecule. In the present application, "OX40 agonist" generally refers to a substance or reagent capable of activating OX40 and/or enhancing its activity. For example, the OX40 agonist can convert OX40 from an inactive state to an active state, or from a low active state to a high active state by directly or indirectly binding to OX40.

In the present application, the term "immunoconjugate" generally refers to a protein molecule formed by the conjugation of one or more immunoglobulin-related molecules or fragments thereof to one or more additional molecules. The additional molecules may be the same as immunoglobulin-related molecules or fragments thereof. In some cases, the additional molecules may be different from immunoglobulin-related molecules or fragments thereof. The one or more additional molecules may be the same as or different from each other. For example, the additional molecules may be target-binding moieties and/or effector moieties, such as toxins or signaling molecules.

In the present application, the term "cytokines" generally refers to proteins that function as intercellular regulators, comprising, for example, interleukins, interferons, chemokines, hematopoietic growth factors, tumor necrosis factors and transforming growth factors. In some instances, examples of the cytokines may be IL2, IL21 and/or IFN-γ. The cytokines in the present application comprise proteins from natural sources or from recombinant cell culture, and biologically active equivalents of native-sequence cytokines.

In the present application, the term "transcription factors" generally refers to proteins capable of binding to DNA and regulating the transcription of related genes.

In the present application, the term "immune response" generally refers to the action of, for example, lymphocytes, antigen-presenting cells, phagocytes, granulocytes, and soluble macromolecules (such as antibodies, cytokines and complements) produced from the above cells or from the liver, which can lead to selective damage, destruction or elimination from the body of invading pathogens, cells or tissues infected with pathogens, cancerous cells or (in the case of autoimmunity or pathological inflammation) normal human cells or tissues.

In the present application, the term "IL-2" or "IL2" generally refers to interleukin-2, functional variants thereof and/or functional fragments thereof. The term IL2 covers human IL-2 (hIL-2), as well as IL-2 from other species, such as other mammals (e.g., rats, mice, rabbits, non-human primates, pigs or cows). The term "IL-2" may also refer to polypeptides capable of stimulating the proliferation of hIL-2-dependent cytolytic or helper T cell lines, such as those listed in the standard determination of Gillis, S., et al., J. Immunol. (1978) 120:2027-2032 and Watson, J., J. Exp. Med. (1979) 150:1510-1519.

In the present application, the term "IL-12" or "IL12" generally refers to interleukin-12, functional variants thereof and/or functional fragments thereof. The term IL12 covers human IL-12 (hIL-12), as well as IL-12 from other species, such as other mammals (e.g., rats, mice, rabbits, non-human primates, pigs or cows).

In the present application, the term "IFN-γ" generally refers to interferon gamma and variants thereof, and sometimes refers to interferon gamma-like polypeptides. For example, human IFN-γ variants may refer to naturally occurring (i.e., allelic variants at the IFN-γ locus) or recombinantly prepared proteins, which have amino acid sequences that are identical, similar or substantially similar to those of mature, natural human IFN-γ. IFN-γ may also comprise IFN-γ fragments or truncated forms of IFN-γ which retain its activity.

In the present application, the term "T-bet" generally refers to a kind of transcription factor, which can induce the production of interferon-gamma (IFN-γ) and coordinate the migration of helper T cells by regulating the expression of chemokines and chemokine receptors.

In the present application, the term "Bcl6" generally refers to B-cell lymphoma 6 protein, a kind of transcription factor.

In the present application, the term "HBeAg" generally refers to a kind of viral protein of hepatitis B. The term "HBeAg negative" generally refers to the result based on the HBeAg assay test (e.g., ELISA assay test), wherein for samples that are considered to reproducibly contain undetectable levels of HBeAg, the result is equal to or less than the threshold or cutoff value for that assay test.

In the present application, the term "one or more isolated nucleic acid molecules" generally refers to isolated nucleotides, deoxyribonucleotides or ribonucleotides of any length, or analogues thereof isolated from its natural environment or synthesized artificially. In the present application, the one or more isolated nucleic acid molecules encode the isolated antigen-binding protein of the present application or the immunoconjugate of the present application.

In the present application, the term "vector" generally refers to a nucleic acid delivery vehicle into which a polynucleotide encoding a certain protein can be inserted so as to enable the expression of the protein. The vector can make the genetic elements it carries be expressed in a host cell by transforming, transducing or transfecting the host cell. For example, the vector comprises: plasmid; phagemid; Cosmid; artificial chromosomes, such as yeast artificial chromosomes (YAC), bacterial artificial chromosomes (BAC) or P1-derived artificial chromosomes (PAC); phages, such as lambda phages or M13 phages and animal viruses, and the like. The species of animal viruses used as the vector are retrovirus (comprising lentivirus), adenovirus, adeno-associated virus, herpes virus (e.g., herpes simplex virus), poxvirus, baculovirus, papilloma virus, papovavirus (e.g., SV40). A vector may contain various elements for controlling the expression, comprising promoter sequences, transcription initiation sequences, enhancer sequences, selective elements and reporter genes. In addition, the vector may also contain replication initiation sites. The vector may also probably comprise ingredients that help its entry into cells, such as virion, lipidosome or protein coat, but not only these substances.

In the present application, the term "cell" generally refers to a single cell, cell line, or cell culture that can be or has been a recipient of a subject's plasmid or vector, which comprises the nucleic acid molecules of the present invention or the vector of the present invention. The cells may comprise the offsprings of a single cell. Due to natural, accidental or intentional mutations, the offsprings may not necessarily be exactly the same as the original parent cells (in the form of the total DNA complement or in the genome). The cells may comprise cells transfected with the vector of the present invention in vitro. The cells may be bacterial cells (e.g., *E. coli),* yeast cells, or other eukaryotic cells, such as COS cells, Chinese Hamster Ovary (CHO) cells, HeLa cells, HEK293 cells, COS-1 cells, NS0 cells, or myeloma cells. In some embodiments, the cells are mammal cells. In some embodiments, the mammal cells are HEK293 cells.

In the present application, the term "pharmaceutically acceptable carrier" generally refers to any and all solvents, dispersion media, coatings, isotonic agents and absorption delaying agents that are compatible with the medication, which are generally safe, non-toxic and neither biologically nor otherwise undesirable.

In the present application, the term "tumor" generally refers to neoplasms or solid lesions formed by abnormal cell growth. In the present application, tumor may be solid tumor or blood tumor. For example, the tumor may be selected from leukemia, lung cancer (e.g., non-small cell lung cancer), breast cancer (e.g., triple-negative breast cancer), renal cancer (e.g., renal cell carcinoma), melanoma, cervical cancer, uterine cancer, pancreatic cancer, peritoneal cancer, ovarian cancer, colon cancer, brain cancer, bladder cancer, head and neck cancer, mesothelioma, prostate cancer, gastric cancer and medulloblastoma.

In the present application, the term "subject" generally refers to human or non-human animals, comprising but not limited to cat, dog, horse, pig, cow, sheep, rabbit, mouse, rat, or monkey.

In the present application, the term "viral infection" generally refers to a disease or condition caused by virus infection of living cells. Depending on the time of onset, viral infection can be divided into inapparent infection, acute infection, latent infection and chronic infection. Depending on different routes of transmission and sites of lesion, it can be divided into respiratory viral diseases, gastrointestinal viral diseases, liver viral diseases, skin and mucous membrane viral diseases, ocular viral diseases, central nervous system viral diseases, pro-lymphocytic viral diseases, insect-borne viral diseases, and lentiviral infections. Where, the term "chronic viral infection" generally refers to viral infectious diseases or conditions with a long incubation period.

In the present application, the term "bacterial and/or fungal infection" generally refers to a process that is normally prevented by the presence or invasion of bacteria or fungi in the host, interfering with the normal physiological functioning of the host. The bacteria or fungi causing the infection may develop an ability to block the defense mechanism of the host, e.g., disrupt the immune system (e.g., interfere with the function of T-cells). In some instances, the bacterial and/or fungal infection may comprise sepsis.

In the present application, the term "sepsis" generally refers to acute syndromes caused by microbial infections that may be life-threatening. When severe microbial infection causes a series of multifactorial, abnormal immune responses in the host, they may lead to sepsis. The body's immune responses are usually precisely regulated by the innate immune system and are significantly influenced by many endogenous factors. When sepsis occurs, the abnormal immune responses are manifested in several developmental stages, comprising early activation of hyperinflammatory and anti-inflammatory responses, followed by immunosuppression and abnormal coagulation, and ultimately leading to organ dysfunction and failure.

In the present application, the term "PBMC cells" generally refers to peripheral blood mononuclear cells, which may comprise lymphocytes (T cells, B cells, NK cells) and monocytes. These cells can be extracted from whole blood by using Ficoll (a kind of hydrophilic polysaccharide for isolating blood layer) and/or gradient centrifugation (which can separate blood into an upper plasma layer, a PBMC layer and a lower layer comprising polymorphonuclear cells (e.g., neutrophils and eosinophils) and red blodd cells).

In the present application, the term "enhancing immune effects in a subject in need thereof' generally refers to inducing, eliciting, or stimulating immune cells (e.g., T-cells or PBMC cells) to have sustained or enhanced biological function, or renewing or reactivating depleted or inactivated immune cells. Examples of enhanced immune cell functions comprise: increased secretion of IL-2 by PBMC cells, increased proliferation, enhanced antigenic reactivity (e.g., viral or pathogen clearance) relative to the corresponding levels before intervention. The degree of enhancement may be at least 50%, for example, at least 50%, at least 100%, at least 200%, at least 300%, at least 400%, at least 500%. Methods for measuring such enhancements are known to those with ordinary skills in the art.

In the present application, the term "comprise" generally refers to the inclusion of explicitly specified features, but not excluding other elements.

In the present application, the term "about" generally refers to varying in a range of 0.5%-10% above or below a specified value, for example, varying in a range of 0.5%, 1%, 1.5%, 2%, 2.5%, 3%, 3.5%, 4%, 4.5%, 5%, 5.5%, 6%, 6.5%, 7%, 7.5%, 8%, 8.5%, 9%, 9.5%, or 10% above or below a specified value.

### Isolated antigen-binding protein, antibodies, variants

In one aspect, the present application provides an isolated antigen-binding protein, comprising a PD-L1 binding moiety and an OX40 binding moiety.

### PD-L1 reference antibody

The isolated antigen-binding protein of the present application comprises a PD-L1 binding moiety. The PD-L1 binding moiety is capable of competing with a PD-L1 reference antibody for binding to human PD-L1.

In the present application, the PD-L1 reference antibody may comprise a heavy chain variable domain VH2, and the VH2 may comprise a H2CDR3, the H2CDR3 may comprise an amino acid sequence as set forth in SEQ ID NO: 62. In some instances, the H2CDR3 may comprise an amino acid sequence as set forth in any one of SEQ ID NOs: 39-45.

In the present application, the PD-L1 reference antibody may comprise a heavy chain variable domain VH2, and the VH2 may comprise a H2CDR1, the H2CDR3 may comprise an amino acid sequence as set forth in SEQ ID NO: 60. In some instances, the H2CDR1 may comprise an amino acid sequence as set forth in any one of SEQ ID NOs: 21-29.

In the present application, the PD-L1 reference antibody may comprise a heavy chain variable domain VH2, and the VH2 may comprise a H2CDR2, the H2CDR3 may comprise an amino acid sequence as set forth in SEQ ID NO: 61. In some instances, the H2CDR2 may comprise an amino acid sequence as set forth in any one of SEQ ID NOs: 30-38.

In the present application, the PD-L1 reference antibody may comprise a heavy chain variable domain VH2, and the VH2 may comprise an amino acid sequence as set forth in SEQ ID NO: 63. In some instances, the VH2 may comprise an amino acid sequence as set forth in any one of SEQ ID NOs: 46-59.

The PD-L1 reference antibody of the present application may comprise H2CDR3, H2CDR1 and/or H2CDR2. Where, the H2CDR3 may comprise an amino acid sequence as set forth in SEQ ID NO: 62, the H2CDR3 may comprise an amino acid sequence as set forth in SEQ ID NO: 60, and the H2CDR3 may comprise an amino acid sequence as set forth in SEQ ID NO: 61.

In the present application, the PD-L1 reference antibody may comprise H2CDR3, H2CDR1 and/or H2CDR2. In some instances, the H2CDR3 may comprise an amino acid sequence as set forth in any one of SEQ ID NOs: 39-45, the H2CDR1 may comprise an amino acid sequence as set forth in any one of SEQ ID NOs: 21-29, and the H2CDR2 may comprise an amino acid sequence as set forth in any one of SEQ ID NOs: 30-38.

For example, the PD-L1 reference antibody may comprise a heavy chain variable domain VH2, and the VH2 may comprise H2CDR3, H2CDR1 and/or H2CDR2. Where, the H2CDR3 may comprise an amino acid sequence as set forth in SEQ ID NO: 45, the H2CDR1 may comprise an amino acid sequence as set forth in SEQ ID NO: 29, and the H2CDR2 may comprise an amino acid sequence as set forth in SEQ ID NO: 36.

For example, the PD-L1 reference antibody may comprise a heavy chain variable domain VH2. Where, the VH2 may comprise an amino acid sequence as set forth in SEQ ID NO: 53.

For example, the PD-L1 reference antibody may comprise a heavy chain variable domain VH2, and the VH2 may comprise H2CDR3, H2CDR1 and/or H2CDR2. Where, the H2CDR3 may comprise an amino acid sequence as set forth in SEQ ID NO: 45, the H2CDR1 may comprise an amino acid sequence as set forth in SEQ ID NO: 29, and the H2CDR2 may comprise an amino acid sequence as set forth in SEQ ID NO: 36. Further for example, the VH2 may comprise an amino acid sequence as set forth in SEQ ID NO: 53. In some embodiments, the antibody or the antigen-binding fragment thereof may comprise Hu56v1 or an antibody having the same heavy chain variable domain.

### PD-L1 binding moiety

The isolated antigen-binding protein of the present application comprises a PD-L1 binding moiety. The PD-L1 binding moiety may comprise a heavy chain variable domain VH2, and the VH2 may comprise H2CDR3, the H2CDR3 may comprise an amino acid sequence as set forth in SEQ ID NO: 62: X₁X₂X₃X₄X₅X₆X₇X₈X₉X₁₀X₁₁X₁₂X₁₃X₁₄X₁₅X₁₆X₁₇X₁₈X₁₉X₂₀Y (SEQ ID NO: 62); wherein, X₁ = D or R; X₂ = S, C or L; X₃ = F, P or N; X₄ = E, C, G, P, Q or H; X₅ = D, T, K, I, Y or P; X₆ = P, C or G; X₇ = T, E or P; X₈ = C, F, V or no amino acid; X₉ = T, P, D or no amino acid; X₁₀ = L, S, G, W or no amino acid; X₁₁ = V, C, P or no amino acid; X₁₂ = T, V, S or no amino acid; X₁₃ = V, G or no amino acid; X₁₄ = A, G or no amino acid; X₁₅ = S, Y or no amino acid; X₁₆ = S, W, C or no amino acid; X₁₇ = G, P or L; X₁₈ = A, S or M; X₁₉ = F or G; X₂₀ = Q, D or P. In some instances, the H2CDR₃ may comprise an amino acid sequence as set forth in any one of SEQ ID NOs: 39-45.

In the present application, the PD-L1 binding moiety may comprise a heavy chain variable domain VH2, and the VH2 may comprise H2CDR1, the H2CDR1 may comprise an amino acid sequence as set forth in SEQ ID NO: 60: X₁X₂X₃X₄X₅X₆X₇X₈MX₉ (SEQ ID NO: 60); wherein, X₁ = G, L or R; X₂ = N, F, Y or K; X₃ = I, T or M; X₄ = S, I, F or V; X₅ = S or R; X₆ = R, S or V; X₇ = R, C or Y;X₈ = C or G; Xg = A or G. In some instances, the H2CDR1 may comprise an amino acid sequence as set forth in any one of SEQ ID NOs: 21-29.

In the present application, the PD-L1 binding moiety may comprise a heavy chain variable domain VH2, and the VH2 may comprise H2CDR2, the H2CDR2 may comprise an amino acid sequence as set forth in SEQ ID NO: 61: X₁X₂X₃X₄X₅X₆X₇X₈X₉X₁₀X₁₁X₁₂X₁₃X₁₄X₁₅KG (SEQ ID NO: 61); wherein, X₁ = K, N, T or D; X₂ = L, I or V; X₃ = L, T, D, S or N; X₄ = T, S, G or P; X₅ = T, D or N; X₆ = S, P, T or no amino acid; X₇ = G or I; X₈ = S, T, N or G; X₉ = T or S; X₁₀ = Y, R, S, K or I; X₁₁ = L or Y; X₁₂ = A, V or N; X₁₃ = D or Q; X₁₄ = S or R; X₁₅ = V, M or F. In some instances, the H2CDR2 may comprise an amino acid sequence as set forth in any one of SEQ ID NOs: 30-38.

In the present application, the PD-L1 binding moiety may comprise a heavy chain variable domain VH2, and the VH2 may comprise an amino acid sequence as set forth in SEQ ID NO: 63: QVQLX₁ESGGGX₂VQX₃GGSLRLSCAX₄X₅X₆X₇X₈X₉X₁₀X₁₁X₁₂X₁₃MX₁₄X₁₅RQAPG KX₁₆X₁₇X₁₈X₁₉X₂₀X₂₁X₂₂X₂₃X₂₄X₂₅X₂₆X₂₇X₂₈X₂₉X₃₀X₃₁X₃₂X₃₃X₃₄X₃₅X₃₆KGRFTISX₃₇ X₃₈NX₃₉KX₄₀TX₄₁X₄₂LQMNSLX₄₃X₄₄EDTAX₄₅YYCX₄₆AX₄₇X₄₈X₄₉X₅₀X₅₁X₅₂X₅₃X₅₄X_{5 5}X₅₆X₅₇X₅₈X₅₉X₆₀X₆₁X₆₂X₆₃X₆₄X₆₅X₆₆YX₆₇GQGTX₆₈VTVSS; wherein, X₁ = V or Q; X₂ = L or S; X₃ = P or A; X₄ = A or V; X₅ = S or Q; X₆ = G, L or R; X₇ = N, F, Y or K; X₈ = I, T or M; X₉ = S, I, F orV; X₁₀ = S or R; X₁₁ = R, S orV; X₁₂ = R, C orY; X₁₃ = C or G; X₁₄ = A or G; X₁₅ = F or Y; X₁₆ = E, Q or G; X₁₇ = R or L; X₁₈ = E or V; X₁₉ = R, G or L; X₂₀ = V or G; X₂₁ = A, D, P or S; X₂₂ = K, N, T, D or A; X₂₃ = I, L or V; X₂₄ = L, T, D, S or N; X₂₅ = T, G, S or P; X₂₆ = T, D or N; X₂₇ = P, T, S or no amino acid; X₂₈ = G or I; X₂₉ = N, S, T or G; X₃₀ = T, S or no amino acid; X₃₁ = Y, R, S, K or I; X₃₂ = L or Y; X₃₃ = A, V or N; X₃₄ = D or Q; X₃₅ = S or R; X₃₆ = V, M or F; X₃₇ = R, K or Q; X₃₈ = D or N; X₃₉ = S or A; X₄₀ = N or S; X₄₁ = V or L; X₄₂ = Y or D; X₄₃ = R or K; X₄₄ = A, T or P; X₄₅ = V or M; X₄₆ = A or V; X₄₇ = D or R; X₄₈ = S, C or L; X₄₉ = F, P or N; X₅₀ = E, C, G, P, Q or H; X₅₁ = D, T, K, I, Y or P; X₅₂ = P, C or G; X₅₃ = T, E or P; X₅₄ = C, F, V or no amino acid; X₅₅ = T, P, D or no amino acid; X₅₆ = L, S, G, W or no amino acid; X₅₇ = V, C, P or no amino acid; X₅₈ = T, V, S or no amino acid; X₅₉ = V, G or no amino acid; X₆₀ = A, G or no amino acid; X₆₁ = S, Y or no amino acid; X₆₂ = S, W, C or no amino acid; X₆₃ = G, P or L; X₆₄ = A, S or M; X₆₅ = F or G; X₅₅ = Q, D or P; X₆₇ = W or R; X₆₈ = L or Q. In some instances, the VH2 may comprise an amino acid sequence as set forth in any one of SEQ ID NOs: 46-59.

The PD-L1 binding moiety of the present application may comprise H2CDR3, H2CDR1 and/or H2CDR2. Where, the H2CDR3 may comprise an amino acid sequence as set forth in SEQ ID NO: 62, the H2CDR3 may comprise an amino acid sequence as set forth in SEQ ID NO: 60, and the H2CDR3 may comprise an amino acid sequence as set forth in SEQ ID NO: 61.

In the present application, the PD-L1 binding moiety may comprise H2CDR3, H2CDR1 and/or H2CDR2. In some instances, the H2CDR3 may comprise an amino acid sequence as set forth in any one of SEQ ID NOs: 39-45, the H2CDR1 may comprise an amino acid sequence as set forth in any one of SEQ ID NOs: 21-29, and the H2CDR2 may comprise an amino acid sequence as set forth in any one of SEQ ID NOs: 30-38.

For example, the PD-L1 binding moiety may comprise a heavy chain variable domain VH2, and the VH2 may comprise H2CDR3, H2CDR1 and/or H2CDR2. Where, the H2CDR3 may comprise an amino acid sequence as set forth in SEQ ID NO: 45, the H2CDR1 may comprise an amino acid sequence as set forth in SEQ ID NO: 29, and the H2CDR2 may comprise an amino acid sequence as set forth in SEQ ID NO: 36.

For example, the PD-L1 binding moiety may comprise a heavy chain variable domain VH2. Where, the VH2 may comprise an amino acid sequence as set forth in SEQ ID NO: 53.

For example, the PD-L1 binding moiety may comprise a heavy chain variable domain VH2, and the VH2 may comprise H2CDR3, H2CDR1 and/or H2CDR2. Where, the H2CDR3 may comprise an amino acid sequence as set forth in SEQ ID NO: 45, the H2CDR1 may comprise an amino acid sequence as set forth in SEQ ID NO: 29, and the H2CDR2 may comprise an amino acid sequence as set forth in SEQ ID NO: 36. Further for example, the VH2 may comprise an amino acid sequence as set forth in SEQ ID NO: 53. In some embodiments, the binding moiety may comprise Hu56v1 or an antibody having the same heavy chain variable domain. In the present application, the PD-L1 binding moiety may be a single-domain antibody.

In the present application, the isolated antigen-binding protein comprises a PD-L1 binding moiety. The PD-L1 binding moiety can recognize and/or bind to amino acid residues in an N-terminal IgV domain of human PD-L1. In some instances, the amino acid residues may be I54, Y56, E58, Q66 and/or R113. In some other instances, the amino acid residues may be D61, N63, V68, M115, S117, Y123 and/or R125. For example, the N-terminal IgV domain of human PD-L1 may comprise an amino acid sequence as set forth in SEQ ID NO: 2.

The PD-L1 binding moiety of the present application can bind to a conformational epitope of the N-terminal IgV domain of human PD-L1. In some instances, the conformational epitope may comprise I54, Y56, E58, Q66 and R113. In some other instances, the conformational epitope may comprise D61, N63, V68, M115, S117, Y123 and R125. For example, the conformational epitope may comprise I54, Y56, E58, Q66, R113, D61, N63, V68, M115, S117, Y123 and R125. For example, the N-terminal IgV domain of human PD-L1 may comprise an amino acid sequence as set forth in SEQ ID NO: 2.

### OX40 reference antibody

The isolated antigen-binding protein of the present application comprises an OX40 binding moiety. The OX40 binding moiety is capable of competing with an OX40 reference antibody for binding to human OX40.

In the present application, the OX40 reference antibody may comprise H1CDR3, the H1CDR3 may comprise an amino acid sequence as set forth in SEQ ID NO: 5. The OX40 reference antibody may comprise H1CDR2, the H1CDR2 may comprise an amino acid sequence as set forth in SEQ ID NO: 4. The OX40 reference antibody may comprise H1CDR1, the H1CDR1 may comprise an amino acid sequence as set forth in SEQ ID NO: 3.

In the present application, the OX40 reference antibody may comprise L1CDR3, the L1CDR3 may comprise an amino acid sequence as set forth in SEQ ID NO: 11. The OX40 reference antibody may comprise L1CDR2, the L1CDR2 may comprise an amino acid sequence as set forth in SEQ ID NO: 10. The OX40 reference antibody may comprise L1CDR1, the L1CDR1 may comprise an amino acid sequence as set forth in SEQ ID NO: 9.

The OX40 reference antibody of the present application may comprise a light chain variable domain VL1, the VL1 may comprise an amino acid sequence as set forth in SEQ ID NO: 12. The OX40 reference antibody of the present application may comprise a heavy chain variable domain VH1, the VH1 may comprise an amino acid sequence as set forth in SEQ ID NO: 6.

For example, the OX40 reference antibody may comprise VH1 and VL1, and the VH1 may comprise H1CDR3, H1CDR1 and/or H1CDR2. Where, the H1CDR3 may comprise an amino acid sequence as set forth in SEQ ID NO: 5, the H1CDR1 may comprise an amino acid sequence as set forth in SEQ ID NO: 3, the H1CDR2 may comprise an amino acid sequence as set forth in SEQ ID NO: 4, and the VH1 may comprise an amino acid sequence as set forth in SEQ ID NO: 6. The VL1 may comprise L1CDR3, L1CDR1 and/or L1CDR2. Where, the L1CDR3 may comprise an amino acid sequence as set forth in SEQ ID NO: 11, the L1CDR1 may comprise an amino acid sequence as set forth in SEQ ID NO: 9, the L1CDR2 may comprise an amino acid sequence as set forth in SEQ ID NO: 10, and the VL1 may comprise an amino acid sequence as set forth in SEQ ID NO: 12.

### OX40 binding moiety

The isolated antigen-binding protein of the present application comprises an OX40 binding moiety.

In the present application, the OX40 binding moiety may comprise H1CDR3, the H1CDR3 may comprise an amino acid sequence as set forth in SEQ ID NO: 5. The OX40 binding moiety may comprise H1CDR2, the H1CDR2 may comprise an amino acid sequence as set forth in SEQ ID NO: 4. The OX40 binding moiety may comprise H1CDR1, the H1CDR1 may comprise an amino acid sequence as set forth in SEQ ID NO: 3.

In the present application, the OX40 binding moiety may comprise L1CDR3, the L1CDR3 may comprise an amino acid sequence as set forth in SEQ ID NO: 11. The OX40 binding moiety may comprise L1CDR2, the L1CDR2 may comprise an amino acid sequence as set forth in SEQ ID NO: 10. The OX40 binding moiety may comprise L1CDR1, the L1CDR1 may comprise an amino acid sequence as set forth in SEQ ID NO: 9.

The OX40 binding moiety of the present application may comprise a light chain variable domain VL1, the VL1 may comprise an amino acid sequence as set forth in SEQ ID NO: 12. The OX40 binding moiety of the present application may comprise a heavy chain variable domain VH1, the VH1 may comprise an amino acid sequence as set forth in SEQ ID NO: 6. The OX40 binding moiety of the present application may also comprise a light chain constant region, the light chain constant region may be derived from Igκ or Igλ. For example, the light chain constant region may comprise an amino acid sequence as set forth in SEQ ID NO: 13. The OX40 binding moiety may also comprise a Fab domain.

The OX40 binding moiety of the present application may comprise a heavy chain constant region. The heavy chain constant region may be derived from IgG, such as IgG1 or IgG4. For example, the heavy chain constant region may comprise an amino acid sequence as set forth in SEQ ID NO: 7.

The OX40 binding moiety may comprise a heavy chain H1, the H1 may comprise an amino acid sequence as set forth in SEQ ID NO: 8. The OX40 binding moiety may comprise a light chain L1, the L1 may comprise an amino acid sequence as set forth in SEQ ID NO: 16.

For example, the OX40 binding moiety may comprise VH1 and VL1, and the VH1 may comprise H1CDR3, H1CDR1 and/or H1CDR2. Where, the H1CDR3 may comprise an amino acid sequence as set forth in SEQ ID NO: 5, the H1CDR1 may comprise an amino acid sequence as set forth in SEQ ID NO: 3, the H1CDR2 may comprise an amino acid sequence as set forth in SEQ ID NO: 4, and the VH1 may comprise an amino acid sequence as set forth in SEQ ID NO: 6. The VL1 may comprise L1CDR3, L1CDR1 and/or L1CDR2. Where, the L1CDR3 may comprise an amino acid sequence as set forth in SEQ ID NO: 11, the L1CDR1 may comprise an amino acid sequence as set forth in SEQ ID NO: 9, the L1CDR2 may comprise an amino acid sequence as set forth in SEQ ID NO: 10, and the VL1 may comprise an amino acid sequence as set forth in SEQ ID NO: 12. The OX40 binding moiety may also comprise a heavy chain constant region and a light chain constant region. Where, the heavy chain constant region may comprise an amino acid sequence as set forth in SEQ ID NO: 7, the light chain constant region may comprise an amino acid sequence as set forth in SEQ ID NO: 13. The OX40 binding moiety of the present application may comprise a heavy chain H1 and a light chain L1, wherein the H1 may comprise an amino acid sequence as set forth in SEQ ID NO: 8. The L1 may comprise an amino acid sequence as set forth in SEQ ID NO: 16.

In the present application, the isolated antigen-binding protein comprises an OX40 binding moiety. The OX40 binding moiety is capable of recognizing and/or binding to amino acid residues in a human OX40 extracellular domain. For example, the amino acid residues may be G70 and/or F71. Further for example, the human OX40 extracellular domain may comprise an amino acid sequence as set forth in SEQ ID NO: 1.

The OX40 binding moiety of the isolated antigen-binding protein of the present application can bind to a conformational epitope of the human OX40 extracellular domain. The conformational epitope may comprise amino acid residues in the human OX40 extracellular domain. For example, the amino acid residues may be G70 and/or F71. Further for example, the human OX40 extracellular domain may comprise an amino acid sequence as set forth in SEQ ID NO: 1.

The isolated antigen-binding moiety of the present application may comprise a first polypeptide chain and a second polypeptide chain. The first polypeptide chain of the isolated antigen-binding moiety may comprise the light chain variable domain VL1 of the OX40 binding moiety. In some instances, the VL1 may comprise an amino acid sequence as set forth in SEQ ID NO: 12. The first polypeptide chain may comprise a light chain L1 of the OX40 binding moiety. In some instances, the L1 may comprise an amino acid sequence as set forth in SEQ ID NO: 16. For example, the first polypeptide chain of the isolated antigen-binding moiety may comprise an amino acid sequence as set forth in SEQ ID NO: 16.

The second polypeptide chain of the isolated antigen-binding moiety of the present application may comprise the heavy chain H1 of the OX40 binding moiety and the PD-L1 binding moiety. For example, the heavy chain H1 of the OX40 binding moiety may comprise an amino acid sequence as set forth in SEQ ID NO: 8. The PD-L1 binding moiety may comprise a heavy chain variable domain VH2. In some instances, the VH2 may comprise an amino acid sequence as set forth in SEQ ID NO: 63. For example, the VH2 may comprise an amino acid sequence as set forth in any one of SEQ ID NOs: 46-59.

In the second polypeptide chain, the PD-L1 binding moiety may be linked to H1 of the OX40 binding moiety directly or indirectly. In some instances, the PD-L1 binding moiety may be located at the N-terminus of the H1 of the OX40 binding moiety. For example, the C-terminus of the PD-L1 binding moiety may be fused with the N-terminus of the H1 of the OX40 binding moiety directly or indirectly. In some other instances, the PD-L1 binding moiety may be located at the C-terminus of the H1 of the OX40 binding moiety. For example, the N-terminus of of the PD-L1 binding moiety may be fused with the C-terminus of the H1 of the OX40 binding moiety directly or indirectly. The PD-L1 binding moiety of the present application may be linked to the H1 of the OX40 binding moiety through a linker, for example, a peptide linker. The linker may comprise an amino acid sequence as set forth in any one of SEQ ID NOs: 14-15. For example, the second polypeptide chain of the present application may comprise an amino acid sequence as set forth in any one of SEQ ID NOs: 17-20.

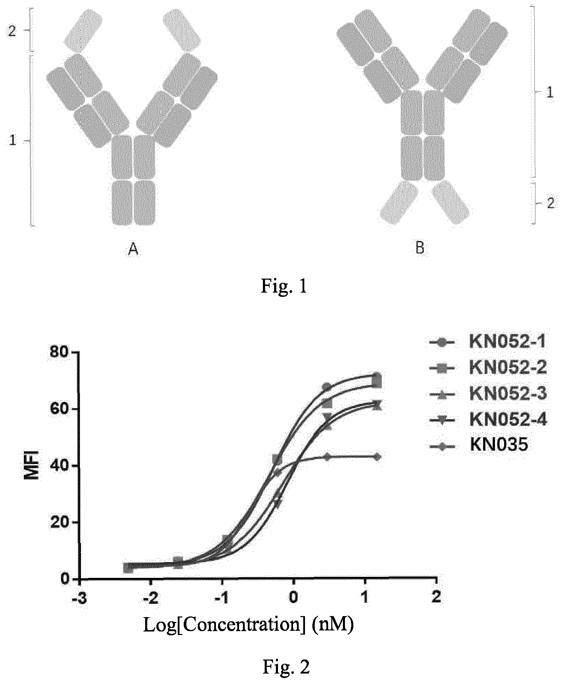

In the present application, the isolated antigen-binding protein may comprise two first polypeptide chains and two second polypeptide chains. The isolated antigen-binding protein has a symmetrical structure. In some instances, the C-terminus of the PD-L1 binding moiety may be linked to the N-terminus of the H1 of the OX40. For example, the structure of the isolated antigen-binding protein may be shown in Fig. 1A. In some other instances, the N-terminus of the PD-L1 binding moiety may be linked to the C-terminus of the H1 of the OX40. For example, the structure of the isolated antigen-binding protein may be shown in Fig. 1B. 1 or 2 of Fig. 1A and Fig. 1B may indicate the PD-L1 binding moiety and the OX40 binding moiety respectively, or alternatively, may indicate the OX40 binding moiety and the PD-L1 binding moiety respectively.

For example, with regard to the structure in Fig. 1A, the first polypeptide chain of the isolated antigen-binding protein may comprise an amino acid sequence as set forth in SEQ ID NO: 16, the second polypeptide chain may comprise an amino acid sequence as set forth in SEQ ID NO: 17, for example, the antigen-binding protein may be KN052-2. For example, with regard to the structure in Fig. 1A, the first polypeptide chain of the isolated antigen-binding protein may comprise an amino acid sequence as set forth in SEQ ID NO: 16, the second polypeptide chain may comprise an amino acid sequence as set forth in SEQ ID NO: 18, for example, the antigen-binding protein may be KN052-1. For example, with regard to the structure in Fig. 1B, the first polypeptide chain of the isolated antigen-binding protein may comprise an amino acid sequence as set forth in SEQ ID NO: 16, the second polypeptide chain may comprise an amino acid sequence as set forth in SEQ ID NO: 19, for example, the antigen-binding protein may be KN052-4. For example, with regard to the structure in Fig. 1B, the first polypeptide chain of the isolated antigen-binding protein may comprise an amino acid sequence as set forth in SEQ ID NO: 16, the second polypeptide chain may comprise an amino acid sequence as set forth in SEQ ID NO: 20, for example, the antigen-binding protein may be KN052-3.

In the present application, the isolated antigen-binding protein is capable of binding to PD-L1 expressed on the cell surface. The cells may be any cells that can express PD-L1, such as CHO cells or A375 cells. The binding effect can be detected by methods known in the art, such as ELISA, flow cytometry, immunoblotting assay. For example, in the flow cytometry assay, by using A375 cells that express hPD-L1, the isolated antigen-binding protein of the present application can bind to PD-L1, for which the EC50 value may be between about 0.1 nM and about 100 nM, e.g., between about 0.1 nM and about 50 nM, between about 0.1 nM and about 30 nM, between about 0.1 nM and about 20 nM, between about 0.1 nM and about 10 nM, between about 0.1 nM and about 5nM or between about 0.1 nM and about 1 nM.

In some instances, the isolated antigen-binding protein of the present application can bind to OX40 on the cell surface, and the cells may be any cells that can express OX40, such as HEK293 cells or T cells. The binding effect can be detected by methods known in the art, such as ELISA, flow cytometry, immunoblotting assay. For example, the antigen-binding protein of the present application is incubated with HEL293 cells capable of expressing OX40, and analyzed by flow cytometry, for which the EC50 value of binding may be between about 0.1 nM and about 100 nM, e.g., between about 0.1 nM and about 50 nM, between about 0.1 nM and about 30 nM, between about 0.1 nM and about 20 nM, between about 0.1 nM and about 15nM, between about 1 nM and about 15nM or between about 5nM and about 10 nM.

The isolated antigen-binding protein of the present application can bridge PD-L1-expressing cells to OX40-expressing cells. In some instances, the antigen-binding protein of the present application can be mixed with PD-L1-expressing CHO cells and OX40-expressing 293 cells, and then by use of different fluorescent labels, the proportion of the two cells bridged together in the overall cells can be detected. For example, the antigen-binding protein can increase the proportion of bridged cells by more than about 5% (e.g., more than about 6%, more than about 8%, more than about 10%, more than about 12%, more than about 15%, more than about 20% or more).

In the present application, the isolated antigen-binding protein is capable of blocking the binding of PD-1 and PD-L1. In some instances, the PD-L1 may comprise natural PD-L1 proteins or functional fragments, variants, isotypes and species homologues thereof, as well as analogues having at least one common epitope with PD-L1, and they may also be synthesized artificially. In some instances, the PD-L1 may be PDL1-Fc-fused protein. The blocking activity can be detected by any methods known in the art, shuch as ELISA, flow cytometry, immunoblotting assay. In some instances, the blocking activity can be detectedby ELISA. For example, when the ratio of the concentration of the antigen-binding protein to the concentration of the receptor protein (PD1 protein) that must be blocked is about 1:10, the blocking activity may be more than about 40% (more than about 50%, more than about 60%, more than about 70% or more). The antigen-binding protein of the present application can eliminate the inhibitory effect caused by PD1/PDL1.

In the present application, the isolated antigen-binding protein has an OX40 agonist activity. In some embodiments, the isolated antigen-binding protein of the present application can convert OX40 from an inactive state to an active state, or from a low active state to a high active state by directly or indirectly binding to OX40 on the surface of T cells. In some ways, the NF-κB luciferase detection system can be used. Firstly, an OX40-transfected vector is constructed, which may be a plasmid. Then a cell line that stably expresses luciferase is constructed, such as 293T cell line. And then the OX40 plasmid is transfected into cells, and after a period of incubation, the antibody to be tested is added, and the luciferase activity is finally detected. For example, the in vitro agonist activity of the antigen-binding protein is comparable to the effect of the OX40 antibody alone.

The protein, polypeptide and/or amino acid sequences involved in the present application should also be understood to comprise at least the following ranges: variants or homologues having the same or similar functions as those of the protein or polypeptide.

In the present application, the variants may be proteins or polypeptides with one or more amino acid substitutions, deletions or additions in the amino acid sequence of the protein and/or the polypeptide. For example, the functional variants may comprise proteins or polypeptides with amino acid changes by at least 1, for example, 1-30, 1-20 or 1-10, and further for example 1, 2, 3, 4 or 5 amino acid substitutions, deletions and/or insertions. The functional variants can substantially maintain the biological properties of the protein or the polypeptide before change (e.g., substitution, deletion or addition). For example, the functional variants can maintain at least 60%, 70%, 80%, 90%, or 100% of the biological activity (e.g., antigen-binding ability) of the protein or the polypeptide before change. For example, the substitution may be conservative substitution.

In the present application, the homologues may be proteins or polypeptides having at least about 85% (e.g., having at least about 85%, about 90%, about 91%, about 92%, about 93%, about 94%, about 95%, about 96%, about 97%, about 98%, about 99% or higher) sequence homology with the amino acid sequence of the protein and/or the polypeptide (e.g., antibodies or fragments thereof that specifically bind to CD38 protein).

In the present application, the homology generally refers to similarity, analogy or association between two or more sequences. The "percentage of sequence homology" can be calculated by the following way: comparing two sequences to be compared in a comparison window, determining the number of positions where the same nucleic acid bases (e.g., A, T, C, G) or the same amino acid residues (e.g., Ala, Pro, Ser, Thr, Gly, Val, Leu, Ile, Phe, Tyr, Trp, Lys, Arg, His, Asp, Glu, Asn, Gin, Cys and Met) are present in both sequences so as to obtain the number of matching positions, dividing the number of matching positions by the total number of positions in the comparison window (i.e., window size), multifying the result by 100 to generate the percentage of sequence homology. The alignment for determining the percentage of sequence homology can be achieved in a variety of ways known in the art, for example, by using publicly available computer softwares, such as BLAST, BLAST-2, ALIGN or Megalign (DNASTAR) softwares. A person skilled in the art can determine suitable parameters for aligning sequences, comprising any algorithms needed to achieve the maximal alignment over the full-length sequence range being compared or within the target sequence region. The homology can also be determined through the following methods: FASTA and BLAST. The description of FASTA algorithm can be found in "Improved tools for biological sequence comparison" to W. R. Pearson and D. J. Lipman, Proc. Natl. Acad. Sci., 85: 2444-2448, 1988; and "Rapid and Sensitive Protein Similarity Searches" to D. J. Lipman and W. R. Pearson, Science, 227: 1435-1441, 1989. The description of BLAST algorithm can be found in "Basic Local Alignment Search Tool" to S. Altschul, W. Gish, W. Miller, E. W. Myers and D. Lipman, Journal of Molecular Biology, 215: 403-410, 1990.

### Isolated nucleic acid molecules, vector and cell

In another aspect, the present application also provides one or more isolated nucleic acid molecules, the one or more nucleic acid molecules can encode the isolated antigen-binding protein of the present application. For example, each of the one or more nucleic acid molecules can encode an intact isolated antigen-binding protein, and can also encode a part of it.

The nucleic acid molecules of the present application may be isolated from each other. For example, they can comprise a nucleotide sequence encoding the isolated antigen-binding protein of the present application or a portion thereof, respectively. The nucleic acid molecules of the present application may also comprise a variety of nucleotide sequences encoding the isolated antigen-binding protein of the present application or a portion thereof simultaneously.

In the present application, the nucleic acid molecules can be synthesized by conventional methods in the art. For example, they can be produced or synthesized by the following methods: (i) amplification *in vitro,* e.g., being produced by amplification through polymerase chain reaction (PCR), (ii) being produced by cloning and recombination, (iii) being purified, for example fractionation by enzymatic digestion and gel electrophoresis, or, (iv) being synthesized, for example through chemical synthesis. In some instances, the nucleic acid molecules can be prepared by a recombinant DNA technology.

In the present application, the nucleic acid encoding the antibody or the antigen-binding fragment thereof can be prepared by a variety of methods known in the art, comprising, but not limited to, overlap extension PCR using restrictive fragment operation or using synthetic oligonucleotide. For specific operations, see Sambrook et al., Molecular Cloning, A Laboratory Manual, Cold Spring Harbor Laboratory Press, Cold Spring Harbor, N.Y., 1989; and Ausube et al. Current Protocols in Molecular Biology, Greene Publishing and Wiley-Interscience, New York N.Y., 1993.

In another aspect, the present application provides one or more vectors, comprising the one or more nucleic acid molecules of the present application. Each vector may comprise one or more of the nucleic acid molecules. Moreover, the vector may further comprise other gene(s), e.g., a marker gene that allows the selection of the vector in an appropriate host cell and under appropriate conditions. Moreover, the vector may further comprise an expression control element that allows the coding region to be properly expressed in an appropriate host. Such a control element is well known by persons skilled in the art. For example, it may comprise a promoter, a ribosome binding site, an enhancer and other control elements regulating the gene transcription or the mRNA translation, and the like. The one or more nucleic acid molecules of the present application can be operatively linked to the expression control element.

The vector may comprise, e.g., plasmid, cosmid, virus, phage, or other vectors commonly used in, e.g., genetic engineering. For example, the vector is an expression vector.

In another aspect, the present application provides a cell, which may comprise the one or more nucleic acid molecules of the present application and/or the one or more vectors of the present application. In some embodiments, each kind of or each of the host cells may comprise one or one kind of the nucleic acid molecules or vectors of the present application. In some embodiments, each kind of or each of the host cells may comprise multiple (e.g., two or more) or multiple kinds of (e.g., two or more kinds of) the nucleic acid molecules or vectors of the present application. For example, the vectors of the present application can be introduced into the cells, e.g., prokaryotic cells (e.g., bacterial cells), CHO cells, NS/0 cells, HEK293T cells or HEK293A cells, or other eukaryotic cells, such as cells from plants, fungi or yeast cells, and the like. The vectors of the present application can be introduced into the cells by methods known in the art, such as electroporation, lipofectine transfection, lipofectamin transfection, and the like. For example, the host cells may be COS, CHO, NSO, sf9, sf21, DH5a, BL21 (DE3) or TG1.

### Pharmaceutical composition, use and method

In another aspect, the present application provides an immunoconjugate, comprising the isolated antigen-binding protein.

In another aspect, the present application provides a use of the isolated antigen-binding protein or the immunoconjugate of the present application in preparing a drug, wherein the drug can be used for the treatment of tumor. The drug can be used for inhibiting the growth of tumors or tumor cells. Where, the tumor may be selected from a group consisting of: blood tumor and solid tumor, for example, melanoma, leukemia, colon cancer. For example, the drug of the present application can inhibit or delay the development or progression of a disease, reduce the tumor size (or even substantially eliminate the tumor), and/or alleviate and/or stabilize the disease state. Examples of inhibiting the growth of tumors or tumor cells comprise: reduced tumor growth volume relative to the corresponding level before intervention. The degree of reduction may be at least 50%, e.g., at least 60%, at least 70%, at least 80%, at least 90%, at least 100%, at least 120%, at least 150% or at least 200%.

In another aspect, the present application further provides a use of the isolated antigen-binding protein or the immunoconjugate in preparing a drug, the drug may be used for the treatment of viral infection in a subject. In some instances, the viral infection may be chronic viral infection. Examples of chronic viral infection are hepatitis B virus infection (HBV), influenza virus infection or Epstein-Barr virus (EBV).

In another aspect, the present application provides a use of the isolated antigen-binding protein or the immunoconjugate in preparing a drug, the drug may be used for the treatment of bacterial and/or fungal infection in a subject. Bacterial and/or fungal infection may comprise systemic inflammatory response syndrome, sepsis and/or severe sepsis. In some instances, bacterial and/or fungal infection may comprise sepsis.

The drug can enhance the immune responses of the subject, for example, immune responses of T cells. The enhancement of T cell immune responses can be demonstrated by, e.g., treated T cells or T cells from treated subjects being highly activated. The T cells may comprise CD4⁺T cells and/or CD8⁺T cells. The activation of T cells can be assessed by stimulating PBMCs to differentiate into T cells or activated T cell secretory cytokines. For example, cytokines may be IL-2, IL21 and/or IFN-γ. In some instances, PBMCs can be stimulated with viral antigens, and the secretion of cytokines can be detected by ELISA.

In another aspect, the present application provides a pharmaceutical composition, which comprises the isolated antigen-binding protein, the immunoconjugate, the isolated nucleic acid molecules, the vector or the cell, and optionally a pharmaceutically acceptable carrier. The pharmaceutically acceptable adjuvant may comprise a buffer, an antioxidant, a preservative, a low molecular weight polypeptide, a protein, a hydrophilic polymer, an amino acid, a carbohydrate, a chelating agent, a counterion, a metal complex and/or a nonionic surfactant, and the like.

In the present application, the drug can be formulated for oral administration, intravenous administration, intramuscular administration, *in situ* administration at the tumor site, inhalation, rectal administration, vaginal administration, transdermal administration or administration via subcutaneous reservoir. For example, for injection preparations, the pharmaceutical composition can be prepared into, for example, a single-dose dosage form of ampoules or, for example, a unit dosage form of multi-dose container. The pharmaceutical composition can also be prepared into solutions, suspensions, tablets, pills, capsules and long-acting preparations.

The drug of the present application may comprise a prophylactically and/or therapeutically effective amount of the antibody or the antigen-binding fragment thereof. The prophylactically and/or therapeutically effective amount is a dose required to prevent and/or treat (at least partially treat) a disease or disorder and/or any complications thereof in a subject suffering from or having a risk of developing the disease or disorder. The dosage and frequency of administration of the pharmaceutical composition of the present application are determined by the type of active ingredient and various other factors, comprising the disease to be treated, the route of administration, the age, sex and weight of the patient, and the severity of the disease, etc. In addition to the route of drug administration and frequency of treatment, various factors, comprising the patient's age, weight, health status, gender, disease severity, diet and metabolic rate, should be considered to determine the effective dosage of the drug. In view of this, the effective dosage of the pharmaceutical composition disclosed herein can be readily determined by those skilled in the art for use in a particular situation. The pharmaceutical composition disclosed herein is not particularly limited to dosage forms, routes of administration, and modes of administration, as long as it shows effectiveness.

In another aspect, the antigen-binding protein, the nucleic acid molecules, the vector, the composition or the cell of the present application can be used for the treatment of the tumor.

In another aspect, the present application provides the antigen-binding protein, the nucleic acid molecules, the vector, the composition, or the cell, which can be used for the treatment of tumor.

In another aspect, the present application also provides a method for inhibiting the growth of tumors or tumor cells, comprising a step of: administering the isolated antigen-binding protein of the present application. Where, the tumor may be selected from a group consisting of: blood tumor and solid tumor, e.g., melanoma, leukemia, lymphoma, myeloma, digestion tract cancer, digestive adenocarcinoma, lung cancer, head and neck cancer, sarcoma, gonadal carcinoma, etc.

In another aspect, the present application also provides a method for treating tumors, comprising a step of: administering the isolated antigen-binding protein, the immunoconjugate, or the pharmaceutical composition of the present application. In another aspect, the present application also provides a method for treating viral infection, comprising a step of: administering the isolated antigen-binding protein, the immunoconjugate, or the pharmaceutical composition of the present application. In another aspect, the present application also provides a method for treating bacterial and/or fungal infection, comprising a step of: administering the isolated antigen-binding protein to a subject in need thereof.

The dosage and frequency of administration of the pharmaceutical composition of the present application are determined by the type of active ingredient and various other factors, comprising the disease to be treated, the route of administration, the age, sex and weight of the patient, and the severity of the disease, etc. In addition to the route of drug administration and frequency of treatment, various factors, comprising the patient's age, weight, health status, gender, disease severity, diet and metabolic rate, should be considered to determine the effective dosage of the drug. In view of this, the effective dosage of the pharmaceutical composition disclosed herein can be readily determined by those skilled in the art for use in a particular situation. The pharmaceutical composition disclosed herein is not particularly limited to dosage forms, routes of administration, and modes of administration, as long as it shows effectiveness.

In another aspect, the present application also provides a method for enhancing immune effects in a subject in need thereof, comprising a step of: administering the isolated antigen-binding protein of the present application to the subject. The enhanced immune effects may comprise, for example, enhanced humoral immune effects (also known as, antibody-dependent) and/or cellular immune effects (also known as, antibody-independent). Enhanced humoral immune effects can be demonstrated by showing elevated antibody titers (e.g., by ELISA assay) in treated subjects. Enhanced celluar immune effects can be demonstrated by, e.g., treated T cells or T cells from treated subjects being highly activated. The activation of T cells can be assessed by stimulating PBMCs to differentiate into T cells or activated T cell secretory cytokines. For example, cytokines may be IL-2.

### Examples

The following examples are given only for illustrating the isolated antigen-binding protein and use, etc. of the present application, rather than being intended to limit the inventive scope of the present application, nor are they intended to indicate that the following experiments are the overall and exclusive experiments performed. Efforts have been made to ensure the accuracy of the numerical values used (e.g., amounts, temperature, etc.), but certain experimental errors and deviations should be accounted for. Unless otherwise stated, the number of parts is parts by weight, the molecular weight is weight average molecular weight, the temperature is in Celsius, and the pressure is at or near atmospheric pressure. Standard abbrevations can be used, for example, bp, base pair; kb, kilobase pair; pl, picoliter; s or sec, second; min, minute; h or hr, hour; aa, amino acid; nt, nucleotide; iv, intravenous injection; i.m., intramuscular; i.p., intraperitoneal; s.c., subcutaneous, etc.

### Example 1 Preparation of the isolated antigen-binding protein

As an example, the following isolated antigen-binding proteins of the present invention are produced: KN052-1, KN052-2, KN052-3 and KN052-4. The amino acid sequences are shown in Table 2 below.

**Table 2 The amino acid sequences of the antigen-binding proteins of the present application**

| | First polypeptide chain | Second polypeptide chain |
|---|---|---|
| KN052-1 | SEQ ID NO: 16 | SEQ ID NO: 18 |
| KN052-2 | | SEQ ID NO: 17 |
| KN052-3 | | SEQ ID NO: 20 |
| KN052-4 | | SEQ ID NO: 19 |

The above sequences of KN052-1, KN052-2, KN052-3, KN052-4 were cloned into an expression vector pCDNA4 (Invitrogen, Cat V86220), obtaining the corresponding recombinant expression vectors, respectively. Then, these recombinant expression vectors were used to transfect HEK293 cells for the expression of recombinant proteins. In brief, plasmids were diluted in Freestyle293 medium, into which was added a PEI (polyethyleneimine) solution. Each plasmid/PEI solution was added into HEK293 cell suspension, and cultured at 37°C, 10% CO2, 90 rpm for 5-6 days. The supernatants were collected, and purified using A protein affinity chromatography, obtaining purified isolated antigen-binding proteins, respectively.

### Example 2 Detection of binding activity

### 2.1 In vitro binding of A375-hPD-L1 cells

A375-hPD-L1 cells (A375 cell lines with constant expression of human PD-L1 protein, produced by stably transfecting an expression cassette of human PD-L1 into A374 cell lines) were adjusted to a density of 4×10⁶ cells/mL, and added into a 96-well plate. The antibodies to be tested obtained in Example 1 which have been diluted to a certain concentration were then added. Anti-PD-L antibody KN035 (Antibody sequence: SEQ ID NO. 64) was used as the positive control. After the processes of binding and washing, the samples were loaded in a flow cytometer for analysis, to measure the median fluorescence intensity (MFI).

The results are shown in Fig. 2. As can be seen, compared to KN035, the isolated antigen-binding proteins of the present application, KN052-1, KN052-2, KN052-3 and KN052-4, all show good PD-L1 binding ability, and the location and manner of attachment of the PD-L1 binding moiety to the OX-40 binding moiety do not affect the ability to bind PD-L1.

### 2.2 In vitro binding of HEK293-OX40 cells

HEK293-OX40 cells (HEK293 cell lines with transient expression of human OX40 protein, produced by transiently transfecting an expression cassette of human OX40 into HEK293 cell lines) were adjusted to a density of 4×10⁶ cells/ml, and added into a 96-well plate. The antibodies to be tested which have been diluted to a certain concentration were then added. Anti-OX-40 antibody DF004 (heavy chain: SEQ ID NO: 8, light chain: SEQ ID NO: 16) was used as the positive control, which was synthesized artificially. After the processes of binding and washing, the samples were loaded in a flow cytometer for analysis, to measure the median fluorescence intensity (MFI).

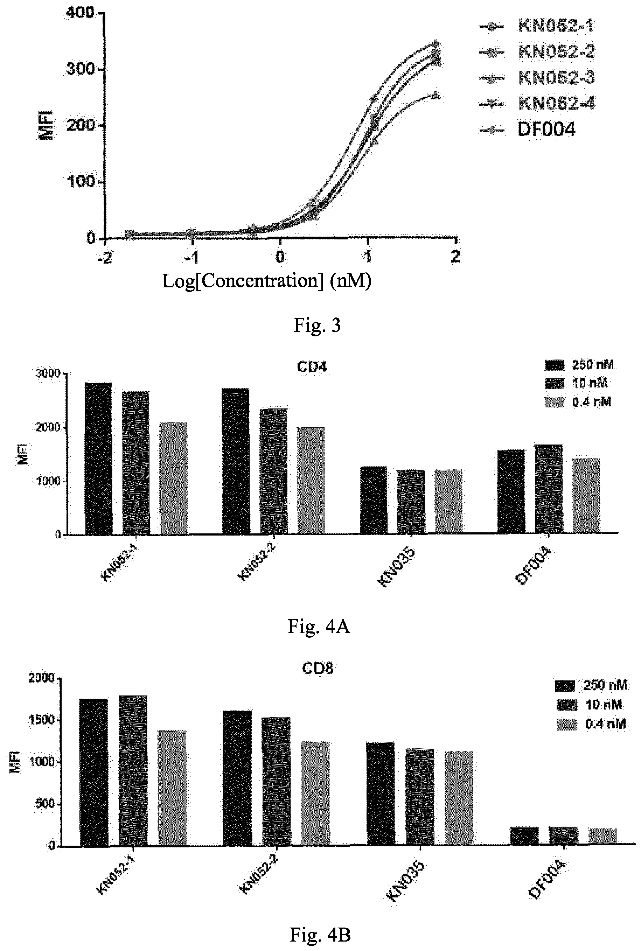

The results are shown in Fig. 3. As can be seen, compared to DF004, the isolated antigen-binding proteins of the present application all show good OX40 binding ability.

### 2.3 In vitro binding of activated T cells

Peripheral blood mononuclear cells (PBMCs) were separated from peripheral blood of healthy donors by means of density gradient centrifugation of human lymphocyte separation solution (purchased from Tianjin Haoyang) and inoculated into a RPMI complete medium. The PBMCs were activated with SEB of which the final concentration was 200 ng/ml for 4 days. The cells were resuspended in 3% PBS-BSA Buffer, into which were added different concentrations of antibodies to be tested, and incubated at room temperature for 60 min. After washing, biolegend secondary antibody APC anti-hlgG, as well as Alexa Fluor 405-anti-CD3 (life technologies, Item No. CD0326), Alexa Fluor 488-anti-CD4 (life technologies, Item No. mhcd0420) and APC-Alexa Fluor 750-anti-CD8 (life technologies, Item No. mhcd0827) were added and incubated on ice for 20 min. After washing, the cells were resuspended in 500 µl 1% PBS-BSA Buffer, and detected by flow cytometer.

The results are shown in Figs. 4A-4B. As can be seen, KN052-1 and KN052-2 can bind to activated CD4⁺T cells (Fig. 4A) and CD8⁺T cells (Fig. 4B) well, better than the control antibodies KN035 and DF004, indicating that both KN052-1 and KN052-2 can bind to OX40.

### Example 3 Cell bridging experiment

PD-L1-expressing CHO cells and OX40-expressing 293 cells were mixed, into which was then added KN052 protein and incubated at 37°C for 1 hour. An equimolar mixing group of anti-PD-L1antibody KN035 and anti-OX40 antibody DF004 was considered as a negative control group. After then, by use of different fluorescent labels, the proportion of the two cells bridged together by the antigen-binding protein of the present application in the overall cells was inspected.

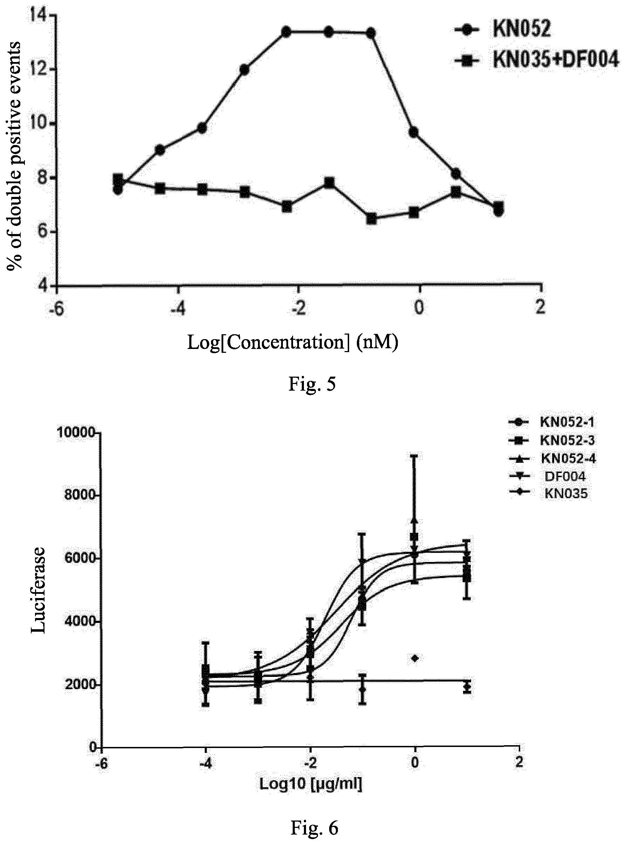

The results are shown in Fig. 5. As can be seen, KN052 protein can effectively bridge two cells which express PDL1 and OX40 respectively. However, a mixture of anti- PD-L1 antibody and anti-OX40 antibody alone cannot achieve such a result.

### Example 4. Detection of in vitro agonist activity of antibodies by NF-κB system

Construction of OX40-CD40 plasmid: according to the amino acid sequence (P43489) of human OX40 on the protein database Uniprot, the amino acid sequence of human OX40 extracellular domain and transmembrane region (i.e., residues from position 1 to position 235 in P43489); according to the amino acid sequence of human CD40 on the protein database Uniprot (P25942), the amino acid sequence of human CD40 intracellular domain (i.e., residues from position 216 to position 277 in P25942); both of them were spliced together by gene synthesis, and then subcloned into the commercial vector pcDNA4/myc-HisA (Invitrogen, V863-20) by double digestion of Hindlll and EcoRI of Fermentas Company, and the accuracy of the constructed plasmid was verified by sequencing, obtaining recombinant plasmid DNA, that was: pcDNA4-OX40-CD40 (i.e., OX40-CD40 referred hereinafter).

293T-NF-κB stable cell lines: 293T cells were seeded in 24-well plates at 1×10⁵/well, each well was added with 200 µl DMEM complete medium, and at the same time also added with 20 µl NF-κB-luciferase-lentivirus (Qiagen, cat: CLS-013L), MOI = 2. After infection for 24 h, the supernatant was discarded, and 1 ml DMEM complete medium was added to continue the culture. After 24 h, DMEM complete medium containing 0.3 µg/ml puromycin was used to continue to culture the cells, and the cells were expanded and cryopreserved. 293T-NF-κB cells screened with puromycin were seeded in 24-well plates at 1×10⁵/well. After being stimulated with 10 ng/ml TNF-α for 6 h, the lysed cells were detected for luciferase. The results show that, the luciferase value of TNF-α-stimulated cells was significantly higher than that of unstimulated cells, demonstrating that NF-κB-luciferase has been stably transfected into 293T cells.

5×10⁵ of 293T-NF-κB cells were resuspended in a medium without double antibodies and seeded in 6-well plates. After 24 h, the supernatant was discarded, the cells were washed once with PBS, and 1.8 ml of serum-free culture without double antibodies was added. At a ratio of plasmid:liposome = 1:3, 0.2 µg of OX40-CD40 plasmids were transfected. After 4 h of transfection, the supernatant was discarded, and the culture was continued by replacing with fresh complete medium. On the day after transfection, cells were seeded in 96-well plates at a density of 5×10⁴/well, a series of concentration gradients of antibodies (initial concentration 10 µg/ml, 10-fold dilution, 7 gradients) were added, and the cells were lysed after incubating at 37°C for 24 h to detect the luciferase.

As shown in Fig.6, a diagram showing the detection results of in vitro agonist activity after taking log of the concentration of antibodies to be tested. The results show that KN052 (regardless of the length of the linker and the attachment location of the two binding moieties) has good agonist activity in vitro, comparable to the effect of anti-OX-40 antibody DF004.

### Example 5 Blocking the binding of PD1 and PDL1

PDL1-Fc fusion proteins were coated at 4°C overnight, and then each well was added with 100 µL of gradient-diluted proteins to be tested, KN052-1, KN052-2, KN052-3 and KN052-4, for which the diluent contained 100 µg/mL of PD1-Fc-Biotin, and reacted at room temperature for 1 hour. After then, SA-HRP (purchased from Sigma Co.) was added and reacted at room temperature for 1 hour. After then, color developing liquid was added, and the absorbance was read at a wavelength of 405 nm.

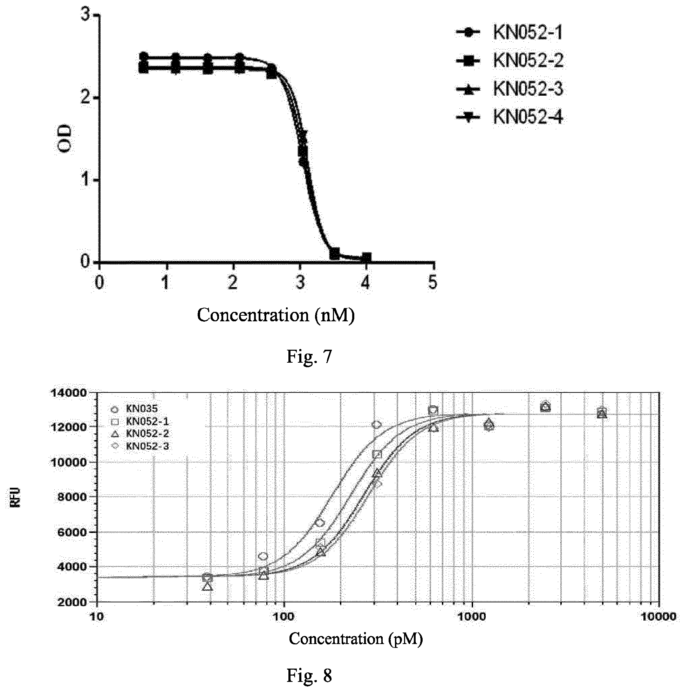

A software SotfMax Pro v5.4 was applied for data processing and graphical analysis, and the blocking curve of antibodies against PDL1-PD1 and IC50 values were obtained through four-parameter fitting. The results are shown in Fig. 7, from which it can be known that KN052-1, KN052-2, KN052-3 and KN052-4 can effectively block the interaction between PDL1 and PD1.

### Example 6 Blocking the binding of PD-L1 and CD80

CD80-Fc proteins were obtained from the expression of HEK293 cells. By utilizing the Biotinlytion kit from Thermo Co., biotinylated proteins CD80-Fc-Biotin were obtained.

PDL1-Fc fusion proteins were coated at 4°C overnight, and then each well was added with 100 µL of gradient-diluted proteins to be tested, KN052-1,2,3,4 (for which the diluent contained 300 µg/mL of CD80-Fc-Biotin), and reacted at room temperature for 1 hour. After then, SA-HRP (purchased from Sigma Co.) was added and reacted at room temperature for 1 hour. After then, color developing liquid was added, and the absorbance was read at a wavelength of 405 nm.

The software SotfMax Pro v5.4 was applied for data processing and graphical analysis, and the blocking curve of antibodies against PDL1-CD80 and IC50 values were obtained through four-parameter fitting. The results show that KN052-1, KN052-2, KN052-3 and KN052-4 can effectively block the interaction between PDL1 and CD80.

### Example 7 Effectively relieving the inhibitory effect caused by PD1/PDL1 in the NFAT system

PD-L1 aAPC/CHO-K1 cells (CHO-K1 cells, cell surface proteins that stably express human PD-L1 and are capable of activating homologous TCR in an antigen-independent manner, Promega) were resuspended with 10%FBS+F-12K+G418+HB, and the cell number was adjusted to 4×10⁵ cells/ml. Into each well was added 50 µl cell suspension, and at the same time 50 µl medium was added to culture overnight. Different concentration levels of anti-PD-L1 antibody KN035 or KN052 antibody were formulated with F-12K + 2% FBS: maximum 10 nM (final concentration: 5000 pM), 2-fold dilution, 8 concentrations. The used medium was discarded. Into each well was added 50 µl drug, and incubated in an incubator for 90 min. Jurkat-PD1-NFAT cells (Promega) were collected, and the cell number was adjusted with 1640 + 2% FB to 8 × 10⁵ cells/ml. Into each well was added 50 µl and incubated in an incubator for 6 hours. Into each well was added 10 µl of luciferase detection substrate and detected by a Fluorescence microplate reader.

The results are shown in Fig. 8, showing that KN052 can effectively relieving the inhibitory effect caused by the binding of PD1-PDL1.

### Example 8 Detection of ADCC activity of the isolated antigen-binding protein of the present application

With engineered Jurkat cells (with the addition of CD16a receptors, at the same time constructing luciferase labels on the NFAT pathway, purchased from Promega) as effector cells, of which the cell number was 1×10⁵ cells/well; with HEK293 cells expressing PD-L1 and OX40 respectively (respectively produced by transiently transfecting human PD-L1 or OX40 expression cassette into HEK293 cell lines) as target cells, of which the cell number was 2.5×10⁴ cells/well, after culturing at 37°C for 5 hours, the production of luciferase was detected with fluorescent substrate.

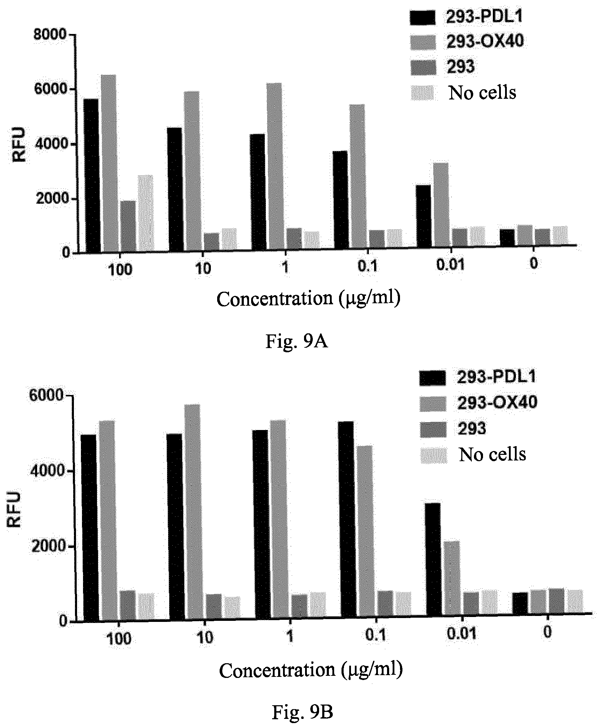

The results are shown in Fig. 9. Fig. 9A shows the effect of KN052-1 on ADCC activity, and Fig. 9B shows the effect of KN052-3 on ADCC activity. The results show that, the antibodies to be tested all have ADCC activity. It can be seen from comparison of 9A and 9B that, PD-L1 binding moiety is fused at the N-terminus or C-terminus of the OX40 binding moiety, with not very significant difference in the effects on the ADCC activity.

### Example 9 Activation effect on PBMCs

The in vitro activity of the isolated antigen-binding protein of the present application in stimulating immune responses was inspected, as shown by the secrection of IL-2 from PBMC cells. In brief, PBMCs were separated from a variety of healthy donors, and respectively cultured with Staphylococcus Aureus Enterotoxin B (SEB) at a concentration of 200 ng/mL. Different concentrations (0.015 nM, 0.15 nM, 1.5 nM, 15 nM, 0.3906 nM, 3.125 nM, 25 nM and 200 nM) of the isolated antigen-binding proteins (KN052-1, KN052-2, KN052-3 or KN052-4) or control molecules (DF004, KN035, KN035+DF004) were added. After 5 days of cultivation, the supernatant was collected, and the level of IL-2 was analyzed with human IL-2 DuoSet ELISA kit.

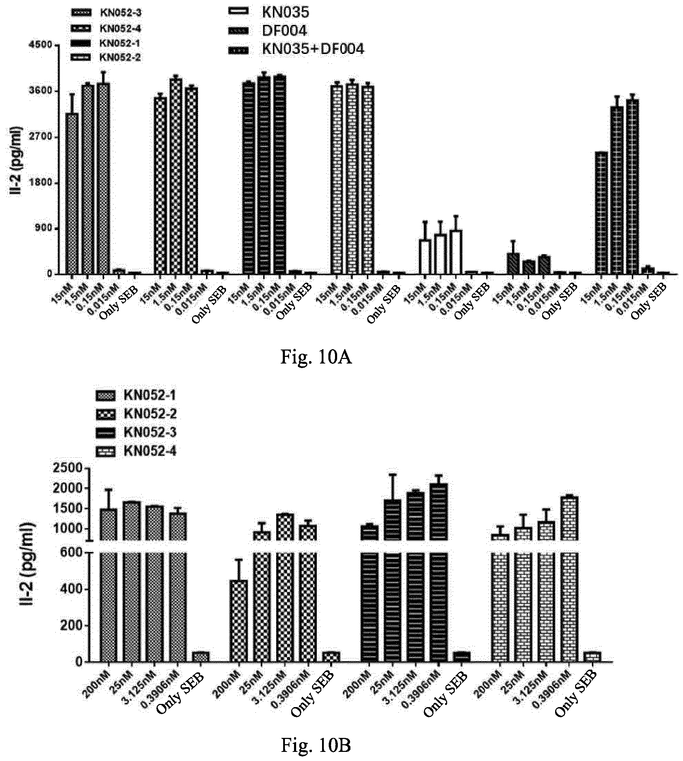

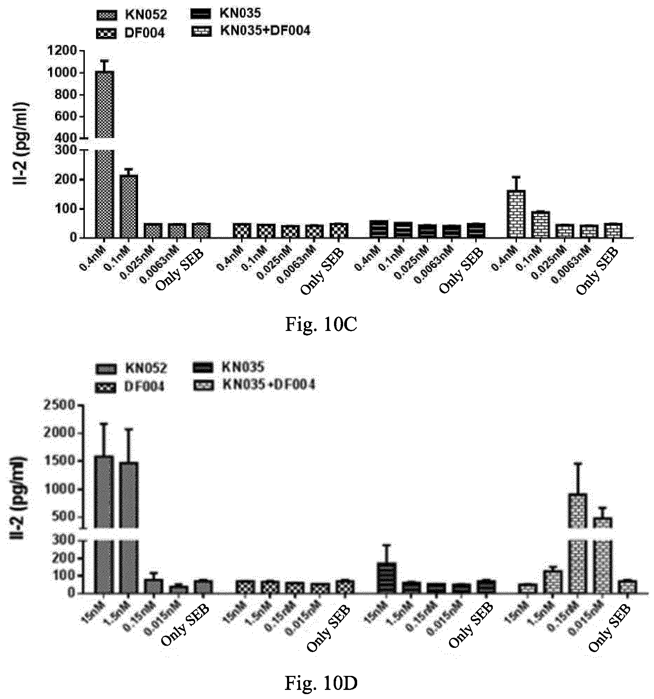

The results are shown in Fig. 10. Figs. 10A-10D show the results of PBMCs from four different donors. It can be seen that, the ability of the isolated antigen-binding proteins of the present application, KN052-1, KN052-2, KN052-3 and KN052-4, in terms of enhancing immune responses is significantly improved compared to control molecules or the combination of control molecules, indicating that there is a strong synergistic effect.

### Example 10 Evaluation of anti-tumor effects of the isolated antigen-binding protein of the present application in MC38-hPD-L1 double knock-in mouse model

Human OX40 and PD-L1 genes were knocked into C57BL/6 mice to produce humanized double knock-in (KI) mice. In addition, mouse colon cancer hPD-L1-MC38 cell lines (MC38 cell lines constantly expressing human PD-L1 protein, which were produced by stably transfecting human PD-L1 expression cassette into MC38 cell lines) were constructed with human PD-L1 instead of mouse PD-L1. MC38-hPD-L1 tumor cells (5×10⁵) were inoculated subcutaneously into right anterior ventral of the double knock-in mice, to obtain hPD-L1-MC38 double knock-in mouse models. When the average tumor size reached about 60-65 mm³, mice were grouped, with 6 mice in each group. The isolated antigen-binding protein (e.g., KN052-2) of the present application or the control solution (e.g., PBS solution) was intraperitoneally injected into the mice, for which the specific time points of administration were the day when the average tumor size reached about 60-65 mm³ (i.e., day 0), and days 4, 7, and 11 after that day. The tumor volume was measured periodically.

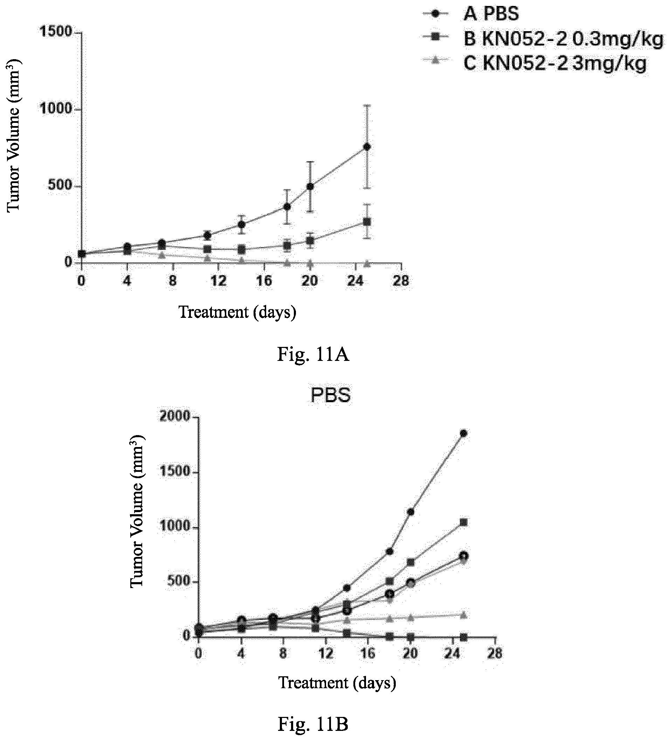

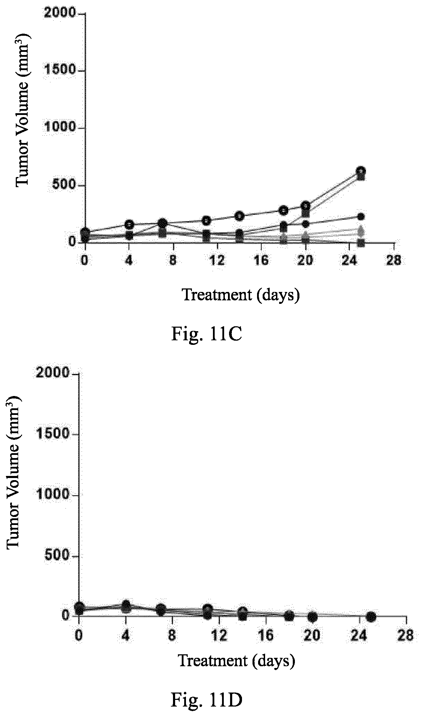

The results are shown in Figs. 11A-11D and Table 2. Where, Fig. 11A shows the average tumor volume of each group of mice at different times after administration of PBS solution, 0.3 mg/kg of KN052-2, and 3 mg/kg of KN052-2; Fig. 11B shows the tumor volume per mouse in the group administered with PBS solution; Fig. 11C shows the tumor volume per mouse in the group administered with 0.3 mg/kg of KN052-2, wherein CR = 1/6; Fig. 11D shows the tumor volume per mouse in the group administered with 3 mg/kg of KN052-2, wherein CR = 5/6. Table 3 below shows the tumor growth inhibition rate 25 days after the first dose. It can be seen from the results that, at a dose of 0.3 mg/kg or 3 mg/kg, the isolated antigen-binding protein KN052-2 of the present application has significant anti-tumor activity in a dose-dependent manner; at a dose of 3 mg/kg, KN052-2 completely inhibits the tumor growth 25 days after the first dose.

**Table 3 Tumor growth inhibition rate (TGI) on Day 25**

| Dose of KN052-2 | 0.3 mg/kg | 3 mg/kg |
|---|---|---|
| TGI (%) | 63.98 | 99.83 |

### Example 11 Evaluation of anti-tumor effects of the isolated antigen-binding protein of the present application in wild-type MC38 (not expressing human PD-L1) double knock-in mouse model

Human OX40 and PD-L1 genes were knocked into C57BL/6 mice to produce humanized double knock-in (KI) mice. Wild-type MC38 tumor cells were inoculated subcutaneously into right anterior ventral of the double knock-in mice (3×10⁵), to obtain MC38 double knock-in mouse models not expressing PD-L1. When the average tumor size reached about 60-65 mm³, mice were grouped, with 6 mice in each group. The isolated antigen-binding protein (e.g., KN052-2) of the present application or the control solution (e.g., PBS solution) was intravenously injected into the mice, for which the specific time points of administration were the day when the average tumor size reached about 60-65 mm³ (i.e., day 0), and days 4, 7, and 11 after that day. The tumor volume was measured periodically.

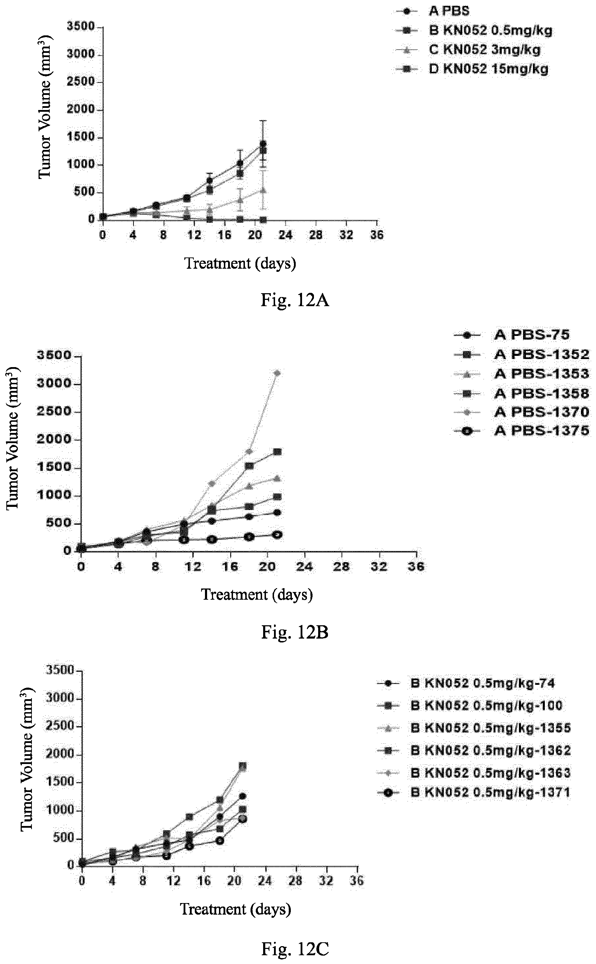

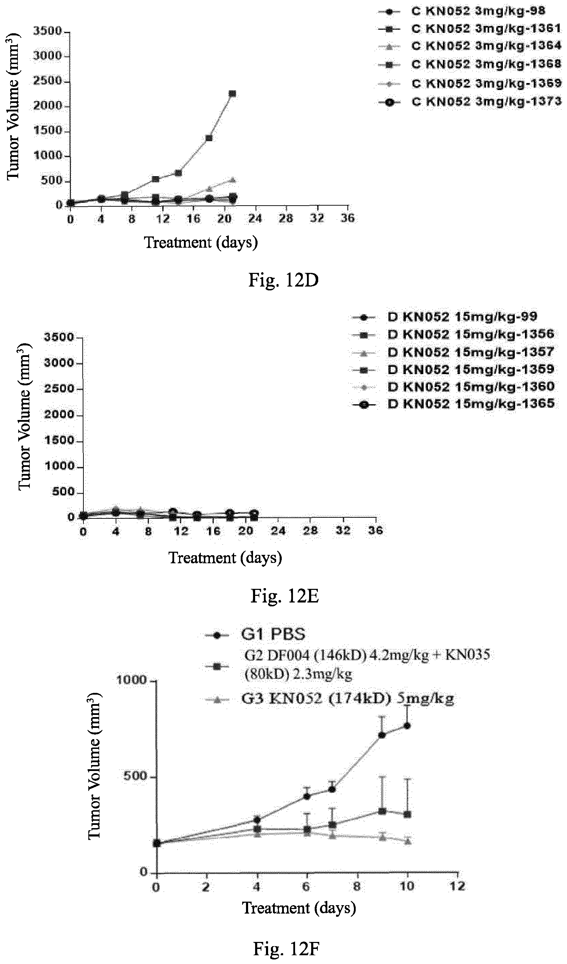

The results are shown in Figs. 12A-12E. Where, Fig. 12A shows the average tumor volume of each group of mice at different times after administration of PBS solution, 0.5 mg/kg of KN052-2, 3 mg/kg of KN052-2 and 15 mg/kg of KN052-2; Fig. 12B shows the tumor volume per mouse of the 6 mice in the group administered with PBS solution; Fig. 12C shows the tumor volume per mouse of the 6 mice in the group administered with 0.5 mg/kg of KN052-2; Fig. 12D shows the tumor volume per mouse of the 6 mice in the group administered with 3 mg/kg of KN052-2; Fig. 12E shows the tumor volume per mouse of the 6 mice in the group administered with 15 mg/kg of KN052-2. It can be seen from the results that, the isolated antigen-binding protein KN052-2 of the present application also has anti-tumor activity in tumors not expressing human PD-L1 in a dose-dependent manner.

Human OX40 and PD-L1 genes were knocked into C57BL/6 mice to produce humanized double knock-in (KI) mice. Wild-type MC38 tumor cells were inoculated subcutaneously into right anterior ventral of the double knock-in mice (3×10⁵), to obtain MC38 double knock-in mouse models not expressing PD-L1. When the average tumor size reached about 50-70 mm³, mice were grouped, with 6 mice in each group. The isolated antigen-binding protein (e.g., 5 mg/kg of KN052-2, G3) of the present application or the control solution (e.g., PBS solution, G1) or the control drug combination (e.g., a combination of 2.3 mg/kg KN035 and 4.2mg/kg DF004, G2) was intravenously injected into the mice, for which the specific time points of administration were the day when the average tumor size reached about 50-70 mm³ (i.e., day 0), and days 4, 6, 7, 9, and 10 after that day. The tumor volume was measured periodically.

The results are shown in Fig. 12F, from which it can be seen that at equimolar dose of administration, the antitumor effect of the bispecific isolated antigen-binding protein KN052-2 of the present application is better than that of the combination of its parent monospecific antibodies KN035+DF004.

### Example 12 Pharmacokinetic test

Cynomolgus monkeys (Suzhou Xishan Zhongke) were intravenously administered with KN052-1 1 mg/kg, 10 mg/kg in a single dose. There were two animals in each dose group, one male and one female. The required blood samples were collected via the femoral vein before administration and 0.083 h, 2 h, 4 h, 8 h, 24 h, 48 h, 72 h (3 days), 120 h (5 days), 168 h (7 days), 216 h (9 days), 264 h (11 days), 336 h (14 days), 504 h (21 days), 672 h (28 days) after the intravenous injection. Sera were separated. The concentration of KN052-1 in the sera of Cynomolgus monkeys was determined by an Elisa method, and the pharmacokinetic parameters were calculated with DAS (3.2.8) software.

The results are shown in Table 4, from which it can be known that the half life of KN052-1 in Cynomolgus monkeys is 69 to 99 hours.

**Table 4 PK Analysis**

| | Dose: 1mg/kg | | Dose: 10mg/kg | |
|---|---|---|---|---|
| | Female | Male | Female | Male |
| Half life t_{1/2} (h) | 99 | 71 | 69 | 95 |
| Cmax (µg/L) | 29942 | 26045 | 318177 | 224148 |
| AUC (0-t) (µg/L*h) | 1622882 | 1088052 | 13021571 | 13825759 |

### Example 13 Detection of antiviral infection activity

Chronic hepatitis B (CHB) patients, aged between 20 and 60 years old, were selected, whose complete clinical information had been recorded and informed consents had been signed. 10 mL of peripheral blood was collected from each of the above CHB patients, from which PBMCs were separated, and then the cells were preserved in liquid nitrogen.

A polypeptide library containing different subtypes of hepatitis B surface antigen sequences (sAg polypeptide library), as well as a hepatitis B c antigen polypeptide library (cAg polypeptides library) were synthesized with reference to a method known in the art (see Chen L, et al. J Immuol, 2007), respectively.

ELISpot kits of R&D were used to further investigate the secrection of IFNγ, IL2 and TNFα from the above PBMCs under the stimulation of KN052. The kits used were: Human IFN-gamma ELISpot Kit (Lot#: EL285); Human IL-2 ELISpot Kit (Lot#: EL202); Human TNF-alpha ELISpot Development Module (Lot#: SEL210), in turn.

PBMCs from different patients were pre-stimulatd with sAg polypeptide library, cAg polypeptide library, non-specific Phorbol-12-Myristate-13-Acetate (phorbol myristate acetate, PMA) or a simple medium, respectively; KN052 protein was then added, or an isotype control was added for further stimulation. After that, experimental operations were conducted according to R&D instruction. The secrections of IFNγ, IL2 and TNFα under different stimulating conditions were investigated by means of ELISpot.

The results show that, the secrection of cytokines increases after the stimulation of KN052.

### Example 14 Detection of drug efficacy in sepsis model

The purpose of this experiment was to investigate the 7-day survival rate of cecal ligation-perforation (CLP) sepsis model mice by KN052. 6-8-week-old PDL1/OX40 humanized mice were used in this experiment. The mice were randomly divided into 4 groups according the body weight: Sham group, CLP model control group (CLP group), experimental group of CLP model + injection of KN052 (KN035 dosing group) and group of CLP model + injection of isotype control antibody (Isotype dosing group). CLP modeling was as follows: the cecum was ligated at a length of 1.5 cm. After ligation, the ligated cecum was punctured with a sterile 25G needle for modeling. 3 h after modeling, through intraperitoneal injection, mice in the sham group and CLP group were each given 200 ul of saline, and mice in the KN052 dosing group and Isotype dosing group were given 50 µg of KN035 and Isotype, respectively. The 7-day survival rate of mice was observed after administration. The observation process started from the day of surgery and was recorded as day 0, and subsequently, every 24 h was an observation time point. The surviving mice in each group were recorded, and the 7-day survival rate (%) of mice was calculated for each group and the survival curve was plotted.

The results show that, there is no significant difference between the survival rate of mice in the Isotype group and that of mice in the CLP group; while compared with mice in the CLP model group, the survival rate of mice in the KN052 dosing group is improved greatly.

The foregoing detailed description is provided by way of explanation and examples, and is not intended to limit the scope of the appended claims. Various changes of the embodiments listed herein are obvious to those of ordinary skills in the art, and are reserved within the scope of the appended claims and their equivalents.

## Claims

1. An isolated antigen-binding protein, comprising a PD-L1 binding moiety and an OX40 binding moiety, wherein:
said OX40 binding moiety is capable of recognizing and/or binding amino acid residues G70 and/or F71 in a human OX40 extracellular domain, wherein said human OX40 extracellular domain comprises an amino acid sequence as set forth in SEQ ID NO: 1;
said PD-L1 binding moiety is capable of recognizing and/or binding amino acid residues I54, Y56, E58, Q66 and/or R113 in an N-terminal IgV domain of human PD-L1, and wherein said N-terminal IgV domain of human PD-L1 comprises an amino acid sequence as set forth in SEQ ID NO: 2.

2. The isolated antigen-binding protein according to claim 1, wherein said PD-L1 binding moiety is also capable of binding amino acid residues D61, N63, V68, M115, S117, Y123 and/or R125 in an N-terminal IgV domain of human PD-L1, and wherein said N-terminal IgV domain of human PD-L1 comprises an amino acid sequence as set forth in SEQ ID NO: 2.

3. The isolated antigen-binding protein according to any one of claims 1-2, wherein said PD-L1 binding moiety is capable of binding a conformational epitope of the N-terminal IgV domain of human PD-L1, said conformational epitope comprises amino acid residues I54, Y56, E58, Q66 and R113 in the N-terminal IgV domain of human PD-L1, and wherein said N-terminal IgV domain of human PD-L1 comprises an amino acid sequence as set forth in SEQ ID NO: 2.

4. The isolated antigen-binding protein according to any one of claims 1-3, wherein said PD-L1 binding moiety is capable of binding the conformational epitope of the N-terminal IgV domain of human PD-L1, said conformational epitope comprises amino acid residues I54, Y56, E58, Q66, R113, D61, N63, V68, M115, S117, Y123 and R125 in the N-terminal IgV domain of human PD-L1, and wherein said N-terminal IgV domain of human PD-L1 comprises an amino acid sequence as set forth in SEQ ID NO: 2.

5. The isolated antigen-binding protein according to any one of claims 1-4, wherein said OX40 binding moiety is capable of binding a conformational epitope of the human OX40 extracellular domain, said conformational epitope comprises amino acid residues G70 and F71 in the human OX40 extracellular domain, and wherein said human OX40 extracellular domain comprises an amino acid sequence as set forth in SEQ ID NO: 1.

6. The isolated antigen-binding protein according to any one of claims 1-5, wherein said PD-L1 binding moiety is capable of competing with a PD-L1 reference antibody for binding to human PD-L1, wherein said PD-L1 reference antibody comprises a heavy chain variable domain VH2, and said VH2 comprises an H2CDR3, said H2CDR3 comprises an amino acid sequence as set forth in SEQ ID NO: 62.

7. The isolated antigen-binding protein according to claim 6, wherein the H2CDR3 of said PD-L1 reference antibody comprises an amino acid sequence as set forth in any one of SEQ ID NOs: 39-45.

8. The isolated antigen-binding protein according to any one of claims 6-7, wherein the VH2 of said PD-L1 reference antibody comprises an H2CDR1, said H2CDR1 comprises an amino acid sequence as set forth in SEQ ID NO: 60.

9. The isolated antigen-binding protein according to claim 8, wherein the H2CDR1 of said PD-L1 reference antibody comprises an amino acid sequence as set forth in any one of SEQ ID NOs: 21-29.

10. The isolated antigen-binding protein according to any one of claims 6-9, wherein the VH2 of said PD-L1 reference antibody comprises an H2CDR2, said H2CDR2 comprises an amino acid sequence as set forth in SEQ ID NO: 61.

11. The isolated antigen-binding protein according to claim 10, wherein the H2CDR2 of said PD-L1 reference antibody comprises an amino acid sequence as set forth in any one of SEQ ID NOs: 30-38.

12. The isolated antigen-binding protein according to any one of claims 6-11, wherein the VH2 of said PD-L1 reference antibody comprises an amino acid sequence as set forth in SEQ ID NO: 63.

13. The isolated antigen-binding protein according to any one of claims 6-12, wherein the VH2 of said PD-L1 reference antibody comprises an amino acid sequence as set forth in any one of SEQ ID NOs: 46-59.

14. The isolated antigen-binding protein according to any one of claims 1-13, wherein said OX40 binding moiety competes with an OX40 reference antibody for binding to a human OX40, wherein said OX40 reference antibody comprises a heavy chain H1CDR3, said heavy chain H1CDR3 comprises an amino acid sequence as set forth in SEQ ID NO: 5.

15. The isolated antigen-binding protein according to claim 14, wherein said OX40 reference antibody comprises a heavy chain H1CDR1, said heavy chain H1CDR1 comprises an amino acid sequence as set forth in SEQ ID NO: 3.

16. The isolated antigen-binding protein according to any one of claims 14-15, wherein said OX40 reference antibody comprises a heavy chain H1CDR2, said heavy chain H1CDR2 comprises an amino acid sequence as set forth in SEQ ID NO: 4.

17. The isolated antigen-binding protein according to any one of claims 14-16, wherein said OX40 reference antibody comprises a heavy chain variable domain VH1, said VH1 comprises an amino acid sequence as set forth in SEQ ID NO: 6.

18. The isolated antigen-binding protein according to any one of claims 14-17, wherein said OX40 reference antibody comprises a light chain L1CDR1, said L1CDR1 comprises an amino acid sequence as set forth in SEQ ID NO: 9.

19. The isolated antigen-binding protein according to any one of claims 14-18, wherein said OX40 reference antibody comprises a light chain L1CDR2, said L1CDR2 comprises an amino acid sequence as set forth in SEQ ID NO: 10.

20. The isolated antigen-binding protein according to any one of claims 14-19, wherein said OX40 reference antibody comprises a light chain L1CDR3, said L1CDR3 comprises an amino acid sequence as set forth in SEQ ID NO: 11.

21. The isolated antigen-binding protein according to any one of claims 14-20, wherein said OX40 reference antibody comprises a light chain variable domain VL1, said VL1 comprises an amino acid sequence as set forth in SEQ ID NO: 12.

22. The isolated antigen-binding protein according to any one of claims 1-21, wherein said PD-L1 binding moiety comprises a heavy chain variable domain VH2, and said VH2 comprises an H2CDR3, and said H2CDR3 comprises an amino acid sequence as set forth in SEQ ID NO: 62.

23. The isolated antigen-binding protein according to claim 22, wherein the H2CDR3 of said PD-L1 binding moiety comprises an amino acid sequence as set forth in any one of SEQ ID NOs: 39-45.

24. The isolated antigen-binding protein according to any one of claims 22-23, wherein the VH2 of said PD-L1 binding moiety comprises an H2CDR1, said H2CDR1 comprises an amino acid sequence as set forth in SEQ ID NO: 60.

25. The isolated antigen-binding protein according to claim 24, wherein the H2CDR1 of said PD-L1 binding moiety comprises an amino acid sequence as set forth in any one of SEQ ID NOs: 21-29.

26. The isolated antigen-binding protein according to any one of claims 22-25, wherein the VH2 of said PD-L1 binding moiety comprises an H2CDR2, said H2CDR2 comprises an amino acid sequence as set forth in SEQ ID NO: 61.

27. The isolated antigen-binding protein according to claim 26, wherein the H2CDR2 of said PD-L1 binding moiety comprises an amino acid sequence as set forth in any one of SEQ ID NOs: 30-38.

28. The isolated antigen-binding protein according to any one of claims 22-27, wherein the VH2 of said PD-L1 binding moiety comprises an amino acid sequence as set forth in SEQ ID NO: 63.

29. The isolated antigen-binding protein according to any one of claims 22-28, wherein the VH2 of said PD-L1 binding moiety comprises an amino acid sequence as set forth in any one of SEQ ID NOs: 46-59.

30. The isolated antigen-binding protein according to any one of claims 1-29, wherein said PD-L1 binding moiety is a single-domain antibody.

31. The isolated antigen-binding protein according to any one of claims 1-30, wherein said PD-L1 binding moiety comprises an amino acid sequence as set forth in SEQ ID NO: 63.

32. The isolated antigen-binding protein according to any one of claims 1-31, wherein said PD-L1 binding moiety comprises an amino acid sequence as set forth in any one of SEQ ID NOs: 46-59.

33. The isolated antigen-binding protein according to any one of claims 1-32, wherein said OX40 binding moiety comprises a heavy chain H1CDR3, said heavy chain H1CDR3 comprises an amino acid sequence as set forth in SEQ ID NO: 5.

34. The isolated antigen-binding protein according to any one of claims 1-33, wherein said OX40 binding moiety comprises a heavy chain H1CDR1, said heavy chain H1CDR1 comprises an amino acid sequence as set forth in SEQ ID NO: 3.

35. The isolated antigen-binding protein according to any one of claims 1-34, wherein said OX40 binding moiety comprises a heavy chain H1CDR2, said heavy chain H1CDR2 comprises an amino acid sequence as set forth in SEQ ID NO: 4.

36. The isolated antigen-binding protein according to any one of claims 1-35, wherein said OX40 binding moiety comprises a heavy chain variable domain VH1, said VH1 comprises an amino acid sequence as set forth in SEQ ID NO: 6.

37. The isolated antigen-binding protein according to any one of claims 1-36, wherein said OX40 binding moiety comprises a light chain L1CDR1, said L1CDR1 comprises an amino acid sequence as set forth in SEQ ID NO: 9.

38. The isolated antigen-binding protein according to any one of claims 1-37, wherein said OX40 binding moiety comprises a light chain L1CDR2, said L1CDR2 comprises an amino acid sequence as set forth in SEQ ID NO: 10.

39. The isolated antigen-binding protein according to any one of claims 1-38, wherein said OX40 binding moiety comprises a light chain L1CDR3, said L1CDR3 comprises an amino acid sequence as set forth in SEQ ID NO: 11.

40. The isolated antigen-binding protein according to any one of claims 1-39, wherein said OX40 binding moiety comprises a light chain variable domain VL1, said VL1 comprises an amino acid sequence as set forth in SEQ ID NO: 12.

41. The isolated antigen-binding protein according to any one of claims 1-40, wherein said OX40 binding moiety comprises a light chain constant region, said light chain constant region is derived from Igκ or Igλ.

42. The isolated antigen-binding protein according to claim 41, wherein said light chain constant region comprises an amino acid sequence as set forth in SEQ ID NO: 13.

43. The isolated antigen-binding protein according to any one of claims 1-42, wherein said OX40 binding moiety comprises a Fab domain.

44. The isolated antigen-binding protein according to any one of claims 1-43, which further comprises a heavy chain constant region, and said heavy chain constant region is derived from IgG.

45. The isolated antigen-binding protein according to claim 44, wherein said heavy chain constant region is derived from human IgG1 or human IgG4.

46. The isolated antigen-binding protein according to any one of claims 44-45, wherein said heavy chain constant region comprises an amino acid sequence as set forth in SEQ ID NO: 7.

47. The isolated antigen-binding protein according to any one of claims 1-46, wherein said PD-L1 binding moiety is located at N-terminus of said OX40 binding moiety.

48. The isolated antigen-binding protein according to any one of claims 1-46, wherein said PD-L1 binding moiety is located at C-terminus of said OX40 binding moiety.

49. The isolated antigen-binding protein according to any one of claims 1-47, wherein said OX40 binding moiety comprises an antibody heavy chain H1, and said PD-L1 binding moiety is fused with N-terminus of said H1 directly or indirectly.

50. The isolated antigen-binding protein according to any one of claims 1-48, wherein said OX40 binding moiety comprises an antibody heavy chain H1, and said PD-L1 binding moiety is fused with C-terminus of said H1 directly or indirectly.

51. The isolated antigen-binding protein according to any one of claims 1-50, comprising a first polypeptide chain and a second polypeptide chain, wherein, said first polypeptide chain comprises the light chain variable domain VL1 of said OX40 binding moiety, said second polypeptide chain comprises the heavy chain variable domain VH1 of said OX40 binding moiety and said PD-L1 binding moiety.

52. The isolated antigen-binding protein according to claim 51, wherein said first polypeptide chain comprises a light chain L1 of said OX40 binding moiety.

53. The isolated antigen-binding protein according to any one of claims 51-52, wherein said first polypeptide chain comprises an amino acid sequence as set forth in SEQ ID NO: 16.

54. The isolated antigen-binding protein according to any one of claims 51-53, wherein said second polypeptide chain comprises the heavy chain H1 of said OX40 binding moiety and said PD-L1 binding moiety.

55. The isolated antigen-binding protein according to claim 54, wherein in said second polypeptide chain, the N-terminus of said heavy chain H1 is linked to the C-terminus of said PD-L1 binding moiety directly or indirectly.

56. The isolated antigen-binding protein according to claim 54, wherein in said second polypeptide chain, the C-terminus of said heavy chain H1 is linked to the N-terminus of said PD-L1 binding moiety directly or indirectly.

57. The isolated antigen-binding protein according to any one of claims 54-56, wherein said heavy chain H1 is linked to said PD-L1 binding moiety through a linker.

58. The isolated antigen-binding protein according to claim 57, wherein said linker is a peptide linker.

59. The isolated antigen-binding protein according to any one of claims 57-58, wherein said linker comprises an amino acid sequence as set forth in any one of SEQ ID NOs: 14-15.

60. The isolated antigen-binding protein according to any one of claims 51-59, wherein said second polypeptide chain comprises an amino acid sequence as set forth in any one of SEQ ID NOs: 17-20.

61. The isolated antigen-binding protein according to any one of claims 51-60, wherein said isolated antigen-binding protein comprises two first polypeptide chains and two second polypeptide chains.

62. The isolated antigen-binding protein according to any one of claims 1-61, which has a symmetrical structure.

63. The isolated antigen-binding protein according to any one of claims 1-62, which is capable of blocking the binding of PD-1 and PD-L1.

64. The isolated antigen-binding protein according to any one of claims 1-63, which is capable of blocking the binding of PD-L1 and CD80.

65. The isolated antigen-binding protein according to any one of claims 1-64, which has an OX40 agonist activity.

66. An immunoconjugate, comprising the isolated antigen-binding protein according to any one of claims 1-65.

67. One or more isolated nucleic acid molecules, encoding the isolated antigen-binding protein according to any one of claims 1-65 or the immunoconjugate according to claims 65.

68. A vector, comprising the one or more isolated nucleic acid molecules according to claim 67.

69. A cell, comprising the one or more isolated nucleic acid molecules according to claim 67 or the vector according to claim 68.

70. A pharmaceutical composition, comprising the isolated antigen-binding protein according to any one of claims 1-65, the immunoconjugate according to claim 66, the one or more isolated nucleic acid molecules according to claim 67, the vector according to claim 68, and/or the cell according to claim 69, and optionally a pharmaceutically acceptable carrier.

71. A use of the isolated antigen-binding protein according to any one of claims 1-65 in preparing a drug, wherein said drug is used for the treatment of tumor.

72. The use according to claim 71, wherein said tumor is selected from a group consisting of: blood tumor and solid tumor.

73. A use of the isolated antigen-binding protein according to any one of claims 1-65 or the immunoconjugate according to claim 66 in preparing a drug, said drug is used for the treatment of viral infection in a subject.

74. The use according to claim 73, wherein said viral infection comprises chronic viral infection.

75. The use according to any one of claims 73-74, wherein said viral infection is infection caused by a virus selected from a group consisting of: hepatitis B virus (HBV), influenza virus and EBV.

76. A use of the isolated antigen-binding protein according to any one of claims 1-65 or the immunoconjugate according to claim 66 in preparing a drug, said drug is used for the treatment of bacterial and/or fungal infection in a subject.

77. The use according to claim 76, wherein said bacterial and/or fungal infection comprises sepsis.

78. The use according to any one of claims 71-77, wherein said drug increases the production of cytokines in the subject.

79. The use according to any one of claims 71-78, wherein said drug increases the production of cytokines from CD4⁺ T cells and/or CD8⁺ T cells in the subject.

80. The use according to any one of claims 78-79, wherein said cytokines comprise interleukins and/or interferons.

81. The use according to any one of claims 78-80, wherein said cytokines comprise IL2, IL21 and/or IFN-γ.

82. The use according to any one of claims 71-81, wherein said drug enhances immune responses of T cells in the subject.

83. The use according to claim 82, wherein said immune responses comprise increased production of cytokines.

84. The use according to any one of claims 82-83, wherein said T cells comprise CD4⁺T cells and/or CD8⁺T cells.

85. The use according to any one of claims 71-84, wherein said drug upregulates the expression level of transcription factors in said subject.

86. The use according to claim 85, wherein said transcription factors comprise T-bet and/or Bcl6.

87. The use according to any one of claims 71-86, wherein said subject is an HBeAg negative subject.

88. A pharmaceutical composition for treating tumors, comprising the isolated antigen-binding protein according to any one of claims 1-65 and optionally a pharmaceutically acceptable carrier.

89. A pharmaceutical composition for treating viral infection, comprising the isolated antigen-binding protein according to any one of claims 1-65 and optionally a pharmaceutically acceptable carrier.

90. A pharmaceutical composition for treating bacterial and/or fungal infection, comprising the isolated antigen-binding protein according to any one of claims 1-65 and optionally a pharmaceutically acceptable carrier.

91. A method for treating viral infection, comprising a step of: administering the isolated antigen-binding protein according to any one of claims 1-65 to a subject in need thereof.

92. A method for treating bacterial and/or fungal infection, comprising a step of: administering the isolated antigen-binding protein according to any one of claims 1-65 to a subject in need thereof.

93. A method for inhibiting the growth of tumors or tumor cells, comprising a step of: administering the isolated antigen-binding protein according to any one of claims 1-65.

94. A method for enhancing immune effects in a subject in need thereof, comprising a step of: administering the isolated antigen-binding protein according to any one of claims 1-65 to said subject.


Sequence Listing

<110>  JIANGSU ALPHAMAB BIOPHARMACEUTICALS CO., LTD.

<120>  OX40/PD-L1 BISPECIFIC ANTIBODY

<130>  203521PEP-WO

<160>  64

<170>  PatentIn version 3.5

<210>  1
<211>  216
<212>  PRT
<213>  Artificial Sequence

<220>
<223>  Human OX40 extracellular domain

<400>  1

Met Cys Val Gly Ala Arg Arg Leu Gly Arg Gly Pro Cys Ala Ala Leu
1               5                   10                  15

Leu Leu Leu Gly Leu Gly Leu Ser Thr Val Thr Gly Leu His Cys Val
            20                  25                  30

Gly Asp Thr Tyr Pro Ser Asn Asp Arg Cys Cys His Glu Cys Arg Pro
        35                  40                  45

Gly Asn Gly Met Val Ser Arg Cys Ser Arg Ser Gln Asn Thr Val Cys
    50                  55                  60

Arg Pro Cys Gly Pro Gly Phe Tyr Asn Asp Val Val Ser Ser Lys Pro
65                  70                  75                  80

Cys Lys Pro Cys Thr Trp Cys Asn Leu Arg Ser Gly Ser Glu Arg Lys
                85                  90                  95

Gln Leu Cys Thr Ala Thr Gln Asp Thr Val Cys Arg Cys Arg Ala Gly
            100                 105                 110

Thr Gln Pro Leu Asp Ser Tyr Lys Pro Gly Val Asp Cys Ala Pro Cys
        115                 120                 125

Pro Pro Gly His Phe Ser Pro Gly Asp Asn Gln Ala Cys Lys Pro Trp
    130                 135                 140

Thr Asn Cys Thr Leu Ala Gly Lys His Thr Leu Gln Pro Ala Ser Asn
145                 150                 155                 160

Ser Ser Asp Ala Ile Cys Glu Asp Arg Asp Pro Pro Ala Thr Gln Pro

165                 170                 175

Gln Glu Thr Gln Gly Pro Pro Ala Arg Pro Ile Thr Val Gln Pro Thr
            180                 185                 190

Glu Ala Trp Pro Arg Thr Ser Gln Gly Pro Ser Thr Arg Pro Val Glu
        195                 200                 205

Val Pro Gly Gly Arg Ala Val Ala
    210                 215

<210>  2
<211>  127
<212>  PRT
<213>  Artificial Sequence

<220>
<223>  N-terminal IgV domain of human PD-L1

<400>  2

Met Arg Ile Phe Ala Val Phe Ile Phe Met Thr Tyr Trp His Leu Leu
1               5                   10                  15

Asn Ala Phe Thr Val Thr Val Pro Lys Asp Leu Tyr Val Val Glu Tyr
            20                  25                  30

Gly Ser Asn Met Thr Ile Glu Cys Lys Phe Pro Val Glu Lys Gln Leu
        35                  40                  45

Asp Leu Ala Ala Leu Ile Val Tyr Trp Glu Met Glu Asp Lys Asn Ile
    50                  55                  60

Ile Gln Phe Val His Gly Glu Glu Asp Leu Lys Val Gln His Ser Ser
65                  70                  75                  80

Tyr Arg Gln Arg Ala Arg Leu Leu Lys Asp Gln Leu Ser Leu Gly Asn
                85                  90                  95

Ala Ala Leu Gln Ile Thr Asp Val Lys Leu Gln Asp Ala Gly Val Tyr
            100                 105                 110

Arg Cys Met Ile Ser Tyr Gly Gly Ala Asp Tyr Lys Arg Ile Thr
        115                 120                 125

<210>  3
<211>  7
<212>  PRT
<213>  Artificial Sequence

<220>

<223>  anti-OX40          H1CDR1

<400>  3

Ser Asn Ser Val Ser Trp Asp
1               5

<210>  4
<211>  18
<212>  PRT
<213>  Artificial Sequence

<220>
<223>  anti-OX40          H1CDR2

<400>  4

Arg Thr Tyr Tyr Arg Ser Lys Trp Tyr Asn Glu Tyr Ala Val Ser Val
1               5                   10                  15

Glu Ser

<210>  5
<211>  7
<212>  PRT
<213>  Artificial Sequence

<220>
<223>  anti-OX40         H1CDR3

<400>  5

Asn Asp Tyr Phe Phe Asp Leu
1               5

<210>  6
<211>  119
<212>  PRT
<213>  Artificial Sequence

<220>
<223>  anti-OX40  VH1

<400>  6

Gln Val Gln Leu Gln Gln Ser Gly Pro Gly Leu Val Lys Pro Ser Gln
1               5                   10                  15

Thr Leu Ser Leu Thr Cys Ala Ile Ser Gly Asp Ser Val Ser Ser Asn
            20                  25                  30

Ser Val Ser Trp Asp Trp Ile Arg Gln Ser Pro Ser Arg Gly Leu Glu
        35                  40                  45

Trp Leu Gly Arg Thr Tyr Tyr Arg Ser Lys Trp Tyr Asn Glu Tyr Ala

50                  55                  60

Val Ser Val Glu Ser Arg Ile Thr Ile Asn Pro Asp Thr Ser Lys Asn
65                  70                  75                  80

Gln Phe Ser Leu Gln Leu Asn Ser Val Thr Pro Glu Asp Thr Ala Ile
                85                  90                  95

Tyr Phe Cys Val Arg Asn Asp Tyr Phe Phe Asp Leu Trp Gly Arg Gly
            100                 105                 110

Thr Leu Val Thr Val Ser Ser
        115

<210>  7
<211>  330
<212>  PRT
<213>  Artificial Sequence

<220>
<223>  anti-OX40        heavy chain constant region

<400>  7

<210>  8
<211>  449
<212>  PRT
<213>  Artificial Sequence

<220>
<223>  anti-OX40         heavy chain H1

<400>  8

Gln Val Gln Leu Gln Gln Ser Gly Pro Gly Leu Val Lys Pro Ser Gln
1               5                   10                  15

Thr Leu Ser Leu Thr Cys Ala Ile Ser Gly Asp Ser Val Ser Ser Asn
            20                  25                  30

Ser Val Ser Trp Asp Trp Ile Arg Gln Ser Pro Ser Arg Gly Leu Glu
        35                  40                  45

Trp Leu Gly Arg Thr Tyr Tyr Arg Ser Lys Trp Tyr Asn Glu Tyr Ala
    50                  55                  60

Val Ser Val Glu Ser Arg Ile Thr Ile Asn Pro Asp Thr Ser Lys Asn
65                  70                  75                  80

Gln Phe Ser Leu Gln Leu Asn Ser Val Thr Pro Glu Asp Thr Ala Ile
                85                  90                  95

Tyr Phe Cys Val Arg Asn Asp Tyr Phe Phe Asp Leu Trp Gly Arg Gly
            100                 105                 110

Thr Leu Val Thr Val Ser Ser Ala Ser Thr Lys Gly Pro Ser Val Phe
        115                 120                 125

Pro Leu Ala Pro Ser Ser Lys Ser Thr Ser Gly Gly Thr Ala Ala Leu
    130                 135                 140

Gly Cys Leu Val Lys Asp Tyr Phe Pro Glu Pro Val Thr Val Ser Trp
145                 150                 155                 160

Asn Ser Gly Ala Leu Thr Ser Gly Val His Thr Phe Pro Ala Val Leu
                165                 170                 175

Gln Ser Ser Gly Leu Tyr Ser Leu Ser Ser Val Val Thr Val Pro Ser
            180                 185                 190

Ser Ser Leu Gly Thr Gln Thr Tyr Ile Cys Asn Val Asn His Lys Pro
        195                 200                 205

Ser Asn Thr Lys Val Asp Lys Lys Val Glu Pro Lys Ser Cys Asp Lys
    210                 215                 220

Thr His Thr Cys Pro Pro Cys Pro Ala Pro Glu Leu Leu Gly Gly Pro
225                 230                 235                 240

Ser Val Phe Leu Phe Pro Pro Lys Pro Lys Asp Thr Leu Met Ile Ser
                245                 250                 255

Arg Thr Pro Glu Val Thr Cys Val Val Val Asp Val Ser His Glu Asp
            260                 265                 270

Pro Glu Val Lys Phe Asn Trp Tyr Val Asp Gly Val Glu Val His Asn
        275                 280                 285

Ala Lys Thr Lys Pro Arg Glu Glu Gln Tyr Asn Ser Thr Tyr Arg Val
    290                 295                 300

Val Ser Val Leu Thr Val Leu His Gln Asp Trp Leu Asn Gly Lys Glu
305                 310                 315                 320

Tyr Lys Cys Lys Val Ser Asn Lys Ala Leu Pro Ala Pro Ile Glu Lys
                325                 330                 335

Thr Ile Ser Lys Ala Lys Gly Gln Pro Arg Glu Pro Gln Val Tyr Thr
            340                 345                 350

Leu Pro Pro Ser Arg Asp Glu Leu Thr Lys Asn Gln Val Ser Leu Thr
        355                 360                 365

Cys Leu Val Lys Gly Phe Tyr Pro Ser Asp Ile Ala Val Glu Trp Glu
    370                 375                 380

Ser Asn Gly Gln Pro Glu Asn Asn Tyr Lys Thr Thr Pro Pro Val Leu
385                 390                 395                 400

Asp Ser Asp Gly Ser Phe Phe Leu Tyr Ser Lys Leu Thr Val Asp Lys
                405                 410                 415

Ser Arg Trp Gln Gln Gly Asn Val Phe Ser Cys Ser Val Met His Glu
            420                 425                 430

Ala Leu His Asn His Tyr Thr Gln Lys Ser Leu Ser Leu Ser Pro Gly
        435                 440                 445

Lys

<210>  9
<211>  11
<212>  PRT
<213>  Artificial Sequence

<220>
<223>  anti-OX40 L1CDR1

<400>  9

Arg Ala Ser Gln Ser Val Gly Ser Tyr Leu Ala
1               5                   10

<210>  10
<211>  7
<212>  PRT
<213>  Artificial Sequence

<220>
<223>  anti-OX40 L1CDR2

<400>  10

Asp Ala Ser Asn Arg Ala Thr
1               5


<210>  11
<211>  11
<212>  PRT
<213>  Artificial Sequence

<220>
<223>  anti-OX40         L1CDR3

<400>  11

Gln Gln Arg Ser Asn Trp Pro Pro Gly Tyr Thr
1               5                   10


<210>  12
<211>  109
<212>  PRT
<213>  Artificial Sequence

<220>
<223>  anti-OX40 VL1

<400>  12

Glu Ile Val Leu Thr Gln Ser Pro Ala Thr Leu Ser Leu Ser Pro Gly
1               5                   10                  15


Glu Arg Ala Thr Leu Ser Cys Arg Ala Ser Gln Ser Val Gly Ser Tyr
            20                  25                  30


Leu Ala Trp Tyr Gln Gln Lys Pro Gly Gln Ala Pro Arg Leu Leu Ile
        35                  40                  45


Tyr Asp Ala Ser Asn Arg Ala Thr Gly Ile Pro Ala Arg Phe Ser Gly
    50                  55                  60


Ser Gly Ser Gly Thr Asp Phe Thr Leu Thr Ile Ser Ser Leu Glu Pro
65                  70                  75                  80


Glu Asp Phe Ala Val Tyr Tyr Cys Gln Gln Arg Ser Asn Trp Pro Pro
                85                  90                  95

Gly Tyr Thr Phe Gly Gln Gly Thr Lys Leu Glu Ile Lys
            100                 105

<210>  13
<211>  107
<212>  PRT
<213>  Artificial Sequence

<220>
<223>  anti-OX40 light chain constant region

<400>  13

<210>  14
<211>  15
<212>  PRT
<213>  Artificial Sequence

<220>
<223>  linker1

<400>  14

Ala Ser Thr Lys Gly Pro Ser Val Phe Pro Leu Ala Gly Ala Pro
1               5                   10                  15

<210>  15
<211>  3
<212>  PRT
<213>  Artificial Sequence

<220>
<223>  linker2

<400>  15

Gly Ala Pro
1



<210>  16
<211>  216
<212>  PRT
<213>  Artificial Sequence

<220>
<223>  KN052 light chain first polypeptide chain (light chain L1)

<400>  16

Glu Ile Val Leu Thr Gln Ser Pro Ala Thr Leu Ser Leu Ser Pro Gly
1               5                   10                  15

Glu Arg Ala Thr Leu Ser Cys Arg Ala Ser Gln Ser Val Gly Ser Tyr
            20                  25                  30

Leu Ala Trp Tyr Gln Gln Lys Pro Gly Gln Ala Pro Arg Leu Leu Ile
        35                  40                  45

Tyr Asp Ala Ser Asn Arg Ala Thr Gly Ile Pro Ala Arg Phe Ser Gly
    50                  55                  60

Ser Gly Ser Gly Thr Asp Phe Thr Leu Thr Ile Ser Ser Leu Glu Pro
65                  70                  75                  80

Glu Asp Phe Ala Val Tyr Tyr Cys Gln Gln Arg Ser Asn Trp Pro Pro
                85                  90                  95

Gly Tyr Thr Phe Gly Gln Gly Thr Lys Leu Glu Ile Lys Arg Thr Val
            100                 105                 110

Ala Ala Pro Ser Val Phe Ile Phe Pro Pro Ser Asp Glu Gln Leu Lys
        115                 120                 125

Ser Gly Thr Ala Ser Val Val Cys Leu Leu Asn Asn Phe Tyr Pro Arg
    130                 135                 140

Glu Ala Lys Val Gln Trp Lys Val Asp Asn Ala Leu Gln Ser Gly Asn
145                 150                 155                 160

Ser Gln Glu Ser Val Thr Glu Gln Asp Ser Lys Asp Ser Thr Tyr Ser
                165                 170                 175

Leu Ser Ser Thr Leu Thr Leu Ser Lys Ala Asp Tyr Glu Lys His Lys
            180                 185                 190

Val Tyr Ala Cys Glu Val Thr His Gln Gly Leu Ser Ser Pro Val Thr
        195                 200                 205

Lys Ser Phe Asn Arg Gly Glu Cys
    210                 215

<210>  17
<211>  592
<212>  PRT
<213>  Artificial Sequence

<220>
<223>  KN052-2 heavy chain Second polypeptide chain 1 (PDL1-linker 1-OX40)

<400>  17

Gln Val Gln Leu Val Glu Ser Gly Gly Gly Leu Val Gln Pro Gly Gly
1               5                   10                  15

Ser Leu Arg Leu Ser Cys Ala Ala Ser Gly Lys Met Ser Ser Arg Arg
            20                  25                  30

Cys Met Ala Trp Phe Arg Gln Ala Pro Gly Lys Glu Arg Glu Arg Val
        35                  40                  45

Ala Lys Leu Leu Thr Thr Ser Gly Ser Thr Tyr Leu Ala Asp Ser Val
    50                  55                  60

Lys Gly Arg Phe Thr Ile Ser Arg Asp Asn Ser Lys Asn Thr Val Tyr
65                  70                  75                  80

Leu Gln Met Asn Ser Leu Arg Ala Glu Asp Thr Ala Val Tyr Tyr Cys
                85                  90                  95

Ala Ala Asp Ser Phe Glu Asp Pro Thr Cys Thr Leu Val Thr Ser Ser
            100                 105                 110

Gly Ala Phe Gln Tyr Trp Gly Gln Gly Thr Leu Val Thr Val Ser Ser
        115                 120                 125

Ala Ser Thr Lys Gly Pro Ser Val Phe Pro Leu Ala Gly Ala Pro Gln
    130                 135                 140

Val Gln Leu Gln Gln Ser Gly Pro Gly Leu Val Lys Pro Ser Gln Thr
145                 150                 155                 160

Leu Ser Leu Thr Cys Ala Ile Ser Gly Asp Ser Val Ser Ser Asn Ser
                165                 170                 175

Val Ser Trp Asp Trp Ile Arg Gln Ser Pro Ser Arg Gly Leu Glu Trp
            180                 185                 190

Leu Gly Arg Thr Tyr Tyr Arg Ser Lys Trp Tyr Asn Glu Tyr Ala Val
        195                 200                 205

Ser Val Glu Ser Arg Ile Thr Ile Asn Pro Asp Thr Ser Lys Asn Gln
    210                 215                 220

Phe Ser Leu Gln Leu Asn Ser Val Thr Pro Glu Asp Thr Ala Ile Tyr
225                 230                 235                 240

Phe Cys Val Arg Asn Asp Tyr Phe Phe Asp Leu Trp Gly Arg Gly Thr
                245                 250                 255

Leu Val Thr Val Ser Ser Ala Ser Thr Lys Gly Pro Ser Val Phe Pro
            260                 265                 270

Leu Ala Pro Ser Ser Lys Ser Thr Ser Gly Gly Thr Ala Ala Leu Gly
        275                 280                 285

Cys Leu Val Lys Asp Tyr Phe Pro Glu Pro Val Thr Val Ser Trp Asn
    290                 295                 300

Ser Gly Ala Leu Thr Ser Gly Val His Thr Phe Pro Ala Val Leu Gln
305                 310                 315                 320

Ser Ser Gly Leu Tyr Ser Leu Ser Ser Val Val Thr Val Pro Ser Ser
                325                 330                 335

Ser Leu Gly Thr Gln Thr Tyr Ile Cys Asn Val Asn His Lys Pro Ser
            340                 345                 350

Asn Thr Lys Val Asp Lys Lys Val Glu Pro Lys Ser Cys Asp Lys Thr
        355                 360                 365

His Thr Cys Pro Pro Cys Pro Ala Pro Glu Leu Leu Gly Gly Pro Ser
    370                 375                 380

Val Phe Leu Phe Pro Pro Lys Pro Lys Asp Thr Leu Met Ile Ser Arg
385                 390                 395                 400

Thr Pro Glu Val Thr Cys Val Val Val Asp Val Ser His Glu Asp Pro
                405                 410                 415

Glu Val Lys Phe Asn Trp Tyr Val Asp Gly Val Glu Val His Asn Ala
            420                 425                 430

Lys Thr Lys Pro Arg Glu Glu Gln Tyr Asn Ser Thr Tyr Arg Val Val
        435                 440                 445

Ser Val Leu Thr Val Leu His Gln Asp Trp Leu Asn Gly Lys Glu Tyr
    450                 455                 460

Lys Cys Lys Val Ser Asn Lys Ala Leu Pro Ala Pro Ile Glu Lys Thr
465                 470                 475                 480

Ile Ser Lys Ala Lys Gly Gln Pro Arg Glu Pro Gln Val Tyr Thr Leu
                485                 490                 495

Pro Pro Ser Arg Asp Glu Leu Thr Lys Asn Gln Val Ser Leu Thr Cys
            500                 505                 510

Leu Val Lys Gly Phe Tyr Pro Ser Asp Ile Ala Val Glu Trp Glu Ser
        515                 520                 525

Asn Gly Gln Pro Glu Asn Asn Tyr Lys Thr Thr Pro Pro Val Leu Asp
    530                 535                 540

Ser Asp Gly Ser Phe Phe Leu Tyr Ser Lys Leu Thr Val Asp Lys Ser
545                 550                 555                 560

Arg Trp Gln Gln Gly Asn Val Phe Ser Cys Ser Val Met His Glu Ala
                565                 570                 575

Leu His Asn His Tyr Thr Gln Lys Ser Leu Ser Leu Ser Pro Gly Lys
            580                 585                 590

<210>  18
<211>  580
<212>  PRT
<213>  Artificial Sequence

<220>
<223>  KN052-1 heavy chain Second polypeptide chain 2 (PDL1-linker 2-OX40)

<400>  18

Gln Val Gln Leu Val Glu Ser Gly Gly Gly Leu Val Gln Pro Gly Gly
1               5                   10                  15

Ser Leu Arg Leu Ser Cys Ala Ala Ser Gly Lys Met Ser Ser Arg Arg
            20                  25                  30

Cys Met Ala Trp Phe Arg Gln Ala Pro Gly Lys Glu Arg Glu Arg Val
        35                  40                  45

Ala Lys Leu Leu Thr Thr Ser Gly Ser Thr Tyr Leu Ala Asp Ser Val
    50                  55                  60

Lys Gly Arg Phe Thr Ile Ser Arg Asp Asn Ser Lys Asn Thr Val Tyr
65                  70                  75                  80

Leu Gln Met Asn Ser Leu Arg Ala Glu Asp Thr Ala Val Tyr Tyr Cys
                85                  90                  95

Ala Ala Asp Ser Phe Glu Asp Pro Thr Cys Thr Leu Val Thr Ser Ser
            100                 105                 110

Gly Ala Phe Gln Tyr Trp Gly Gln Gly Thr Leu Val Thr Val Ser Ser
        115                 120                 125

Gly Ala Pro Gln Val Gln Leu Gln Gln Ser Gly Pro Gly Leu Val Lys
    130                 135                 140

Pro Ser Gln Thr Leu Ser Leu Thr Cys Ala Ile Ser Gly Asp Ser Val
145                 150                 155                 160

Ser Ser Asn Ser Val Ser Trp Asp Trp Ile Arg Gln Ser Pro Ser Arg
                165                 170                 175

Gly Leu Glu Trp Leu Gly Arg Thr Tyr Tyr Arg Ser Lys Trp Tyr Asn
            180                 185                 190

Glu Tyr Ala Val Ser Val Glu Ser Arg Ile Thr Ile Asn Pro Asp Thr
        195                 200                 205

Ser Lys Asn Gln Phe Ser Leu Gln Leu Asn Ser Val Thr Pro Glu Asp
    210                 215                 220

Thr Ala Ile Tyr Phe Cys Val Arg Asn Asp Tyr Phe Phe Asp Leu Trp
225                 230                 235                 240

Gly Arg Gly Thr Leu Val Thr Val Ser Ser Ala Ser Thr Lys Gly Pro
                245                 250                 255

Ser Val Phe Pro Leu Ala Pro Ser Ser Lys Ser Thr Ser Gly Gly Thr
            260                 265                 270

Ala Ala Leu Gly Cys Leu Val Lys Asp Tyr Phe Pro Glu Pro Val Thr
        275                 280                 285

Val Ser Trp Asn Ser Gly Ala Leu Thr Ser Gly Val His Thr Phe Pro
    290                 295                 300

Ala Val Leu Gln Ser Ser Gly Leu Tyr Ser Leu Ser Ser Val Val Thr
305                 310                 315                 320

Val Pro Ser Ser Ser Leu Gly Thr Gln Thr Tyr Ile Cys Asn Val Asn
                325                 330                 335

His Lys Pro Ser Asn Thr Lys Val Asp Lys Lys Val Glu Pro Lys Ser
            340                 345                 350

Cys Asp Lys Thr His Thr Cys Pro Pro Cys Pro Ala Pro Glu Leu Leu
        355                 360                 365

Gly Gly Pro Ser Val Phe Leu Phe Pro Pro Lys Pro Lys Asp Thr Leu
    370                 375                 380

Met Ile Ser Arg Thr Pro Glu Val Thr Cys Val Val Val Asp Val Ser
385                 390                 395                 400

His Glu Asp Pro Glu Val Lys Phe Asn Trp Tyr Val Asp Gly Val Glu
                405                 410                 415

Val His Asn Ala Lys Thr Lys Pro Arg Glu Glu Gln Tyr Asn Ser Thr
            420                 425                 430

Tyr Arg Val Val Ser Val Leu Thr Val Leu His Gln Asp Trp Leu Asn
        435                 440                 445

Gly Lys Glu Tyr Lys Cys Lys Val Ser Asn Lys Ala Leu Pro Ala Pro
    450                 455                 460

Ile Glu Lys Thr Ile Ser Lys Ala Lys Gly Gln Pro Arg Glu Pro Gln
465                 470                 475                 480

Val Tyr Thr Leu Pro Pro Ser Arg Asp Glu Leu Thr Lys Asn Gln Val
                485                 490                 495

Ser Leu Thr Cys Leu Val Lys Gly Phe Tyr Pro Ser Asp Ile Ala Val
            500                 505                 510

Glu Trp Glu Ser Asn Gly Gln Pro Glu Asn Asn Tyr Lys Thr Thr Pro
        515                 520                 525

Pro Val Leu Asp Ser Asp Gly Ser Phe Phe Leu Tyr Ser Lys Leu Thr
    530                 535                 540

Val Asp Lys Ser Arg Trp Gln Gln Gly Asn Val Phe Ser Cys Ser Val

545                 550                 555                 560

Met His Glu Ala Leu His Asn His Tyr Thr Gln Lys Ser Leu Ser Leu
                565                 570                 575

Ser Pro Gly Lys
            580

<210>  19
<211>  591
<212>  PRT
<213>  Artificial Sequence

<220>
<223>  KN052-4 heavy chain Second polypeptide chain 3 (OX40-linker 1-PDL1)

<400>  19

Gln Val Gln Leu Gln Gln Ser Gly Pro Gly Leu Val Lys Pro Ser Gln
1               5                   10                  15

Thr Leu Ser Leu Thr Cys Ala Ile Ser Gly Asp Ser Val Ser Ser Asn
            20                  25                  30

Ser Val Ser Trp Asp Trp Ile Arg Gln Ser Pro Ser Arg Gly Leu Glu
        35                  40                  45

Trp Leu Gly Arg Thr Tyr Tyr Arg Ser Lys Trp Tyr Asn Glu Tyr Ala
    50                  55                  60

Val Ser Val Glu Ser Arg Ile Thr Ile Asn Pro Asp Thr Ser Lys Asn
65                  70                  75                  80

Gln Phe Ser Leu Gln Leu Asn Ser Val Thr Pro Glu Asp Thr Ala Ile
                85                  90                  95

Tyr Phe Cys Val Arg Asn Asp Tyr Phe Phe Asp Leu Trp Gly Arg Gly
            100                 105                 110

Thr Leu Val Thr Val Ser Ser Ala Ser Thr Lys Gly Pro Ser Val Phe
        115                 120                 125

Pro Leu Ala Pro Ser Ser Lys Ser Thr Ser Gly Gly Thr Ala Ala Leu
    130                 135                 140

Gly Cys Leu Val Lys Asp Tyr Phe Pro Glu Pro Val Thr Val Ser Trp
145                 150                 155                 160

Asn Ser Gly Ala Leu Thr Ser Gly Val His Thr Phe Pro Ala Val Leu
                165                 170                 175

Gln Ser Ser Gly Leu Tyr Ser Leu Ser Ser Val Val Thr Val Pro Ser
        180                 185                 190

Ser Ser Leu Gly Thr Gln Thr Tyr Ile Cys Asn Val Asn His Lys Pro
    195                 200                 205

Ser Asn Thr Lys Val Asp Lys Lys Val Glu Pro Lys Ser Cys Asp Lys
    210                 215                 220

Thr His Thr Cys Pro Pro Cys Pro Ala Pro Glu Leu Leu Gly Gly Pro
225                 230                 235                 240

Ser Val Phe Leu Phe Pro Pro Lys Pro Lys Asp Thr Leu Met Ile Ser
                245                 250                 255

Arg Thr Pro Glu Val Thr Cys Val Val Val Asp Val Ser His Glu Asp
            260                 265                 270

Pro Glu Val Lys Phe Asn Trp Tyr Val Asp Gly Val Glu Val His Asn
        275                 280                 285

Ala Lys Thr Lys Pro Arg Glu Glu Gln Tyr Asn Ser Thr Tyr Arg Val
    290                 295                 300

Val Ser Val Leu Thr Val Leu His Gln Asp Trp Leu Asn Gly Lys Glu
305                 310                 315                 320

Tyr Lys Cys Lys Val Ser Asn Lys Ala Leu Pro Ala Pro Ile Glu Lys
                325                 330                 335

Thr Ile Ser Lys Ala Lys Gly Gln Pro Arg Glu Pro Gln Val Tyr Thr
            340                 345                 350

Leu Pro Pro Ser Arg Asp Glu Leu Thr Lys Asn Gln Val Ser Leu Thr
        355                 360                 365

Cys Leu Val Lys Gly Phe Tyr Pro Ser Asp Ile Ala Val Glu Trp Glu
    370                 375                 380

Ser Asn Gly Gln Pro Glu Asn Asn Tyr Lys Thr Thr Pro Pro Val Leu
385                 390                 395                 400

Asp Ser Asp Gly Ser Phe Phe Leu Tyr Ser Lys Leu Thr Val Asp Lys
                405                 410                 415

Ser Arg Trp Gln Gln Gly Asn Val Phe Ser Cys Ser Val Met His Glu

420                 425                 430

Ala Leu His Asn His Tyr Thr Gln Lys Ser Leu Ser Leu Ser Pro Gly
        435                 440                 445

Ala Ser Thr Lys Gly Pro Ser Val Phe Pro Leu Ala Gly Ala Pro Gln
    450                 455                 460

Val Gln Leu Val Glu Ser Gly Gly Gly Leu Val Gln Pro Gly Gly Ser
465                 470                 475                 480

Leu Arg Leu Ser Cys Ala Ala Ser Gly Lys Met Ser Ser Arg Arg Cys
                485                 490                 495

Met Ala Trp Phe Arg Gln Ala Pro Gly Lys Glu Arg Glu Arg Val Ala
            500                 505                 510

Lys Leu Leu Thr Thr Ser Gly Ser Thr Tyr Leu Ala Asp Ser Val Lys
        515                 520                 525

Gly Arg Phe Thr Ile Ser Arg Asp Asn Ser Lys Asn Thr Val Tyr Leu
    530                 535                 540

Gln Met Asn Ser Leu Arg Ala Glu Asp Thr Ala Val Tyr Tyr Cys Ala
545                 550                 555                 560

Ala Asp Ser Phe Glu Asp Pro Thr Cys Thr Leu Val Thr Ser Ser Gly
                565                 570                 575

Ala Phe Gln Tyr Trp Gly Gln Gly Thr Leu Val Thr Val Ser Ser
            580                 585                 590

<210>  20
<211>  579
<212>  PRT
<213>  Artificial Sequence

<220>
<223>  KN052-3 heavy chain Second polypeptide chain 4 (OX40-linker 2-PDL1)

<400>  20

Gln Val Gln Leu Gln Gln Ser Gly Pro Gly Leu Val Lys Pro Ser Gln
1               5                   10                  15

Thr Leu Ser Leu Thr Cys Ala Ile Ser Gly Asp Ser Val Ser Ser Asn
            20                  25                  30

Ser Val Ser Trp Asp Trp Ile Arg Gln Ser Pro Ser Arg Gly Leu Glu
        35                  40                  45

Trp Leu Gly Arg Thr Tyr Tyr Arg Ser Lys Trp Tyr Asn Glu Tyr Ala
    50                  55                  60

Val Ser Val Glu Ser Arg Ile Thr Ile Asn Pro Asp Thr Ser Lys Asn
65                  70                  75                  80

Gln Phe Ser Leu Gln Leu Asn Ser Val Thr Pro Glu Asp Thr Ala Ile
                85                  90                  95

Tyr Phe Cys Val Arg Asn Asp Tyr Phe Phe Asp Leu Trp Gly Arg Gly
            100                 105                 110

Thr Leu Val Thr Val Ser Ser Ala Ser Thr Lys Gly Pro Ser Val Phe
        115                 120                 125

Pro Leu Ala Pro Ser Ser Lys Ser Thr Ser Gly Gly Thr Ala Ala Leu
    130                 135                 140

Gly Cys Leu Val Lys Asp Tyr Phe Pro Glu Pro Val Thr Val Ser Trp
145                 150                 155                 160

Asn Ser Gly Ala Leu Thr Ser Gly Val His Thr Phe Pro Ala Val Leu
                165                 170                 175

Gln Ser Ser Gly Leu Tyr Ser Leu Ser Ser Val Val Thr Val Pro Ser
            180                 185                 190

Ser Ser Leu Gly Thr Gln Thr Tyr Ile Cys Asn Val Asn His Lys Pro
        195                 200                 205

Ser Asn Thr Lys Val Asp Lys Lys Val Glu Pro Lys Ser Cys Asp Lys
    210                 215                 220

Thr His Thr Cys Pro Pro Cys Pro Ala Pro Glu Leu Leu Gly Gly Pro
225                 230                 235                 240

Ser Val Phe Leu Phe Pro Pro Lys Pro Lys Asp Thr Leu Met Ile Ser
                245                 250                 255

Arg Thr Pro Glu Val Thr Cys Val Val Val Asp Val Ser His Glu Asp
            260                 265                 270

Pro Glu Val Lys Phe Asn Trp Tyr Val Asp Gly Val Glu Val His Asn
        275                 280                 285

Ala Lys Thr Lys Pro Arg Glu Glu Gln Tyr Asn Ser Thr Tyr Arg Val

290                 295                 300

Val Ser Val Leu Thr Val Leu His Gln Asp Trp Leu Asn Gly Lys Glu
305                 310                 315                 320

Tyr Lys Cys Lys Val Ser Asn Lys Ala Leu Pro Ala Pro Ile Glu Lys
                325                 330                 335

Thr Ile Ser Lys Ala Lys Gly Gln Pro Arg Glu Pro Gln Val Tyr Thr
            340                 345                 350

Leu Pro Pro Ser Arg Asp Glu Leu Thr Lys Asn Gln Val Ser Leu Thr
        355                 360                 365

Cys Leu Val Lys Gly Phe Tyr Pro Ser Asp Ile Ala Val Glu Trp Glu
    370                 375                 380

Ser Asn Gly Gln Pro Glu Asn Asn Tyr Lys Thr Thr Pro Pro Val Leu
385                 390                 395                 400

Asp Ser Asp Gly Ser Phe Phe Leu Tyr Ser Lys Leu Thr Val Asp Lys
                405                 410                 415

Ser Arg Trp Gln Gln Gly Asn Val Phe Ser Cys Ser Val Met His Glu
            420                 425                 430

Ala Leu His Asn His Tyr Thr Gln Lys Ser Leu Ser Leu Ser Pro Gly
        435                 440                 445

Gly Ala Pro Gln Val Gln Leu Val Glu Ser Gly Gly Gly Leu Val Gln
    450                 455                 460

Pro Gly Gly Ser Leu Arg Leu Ser Cys Ala Ala Ser Gly Lys Met Ser
465                 470                 475                 480

Ser Arg Arg Cys Met Ala Trp Phe Arg Gln Ala Pro Gly Lys Glu Arg
                485                 490                 495

Glu Arg Val Ala Lys Leu Leu Thr Thr Ser Gly Ser Thr Tyr Leu Ala
            500                 505                 510

Asp Ser Val Lys Gly Arg Phe Thr Ile Ser Arg Asp Asn Ser Lys Asn
        515                 520                 525

Thr Val Tyr Leu Gln Met Asn Ser Leu Arg Ala Glu Asp Thr Ala Val
    530                 535                 540

Tyr Tyr Cys Ala Ala Asp Ser Phe Glu Asp Pro Thr Cys Thr Leu Val
545                 550                 555                 560

Thr Ser Ser Gly Ala Phe Gln Tyr Trp Gly Gln Gly Thr Leu Val Thr
                565                 570                 575

Val Ser Ser

<210>  21
<211>  10
<212>  PRT
<213>  Artificial Sequence

<220>
<223>  anti-PD-L1 27 H2CDR1

<400>  21

Gly Asn Ile Ser Ser Arg Arg Cys Met Ala
1               5                   10

<210>  22
<211>  10
<212>  PRT
<213>  Artificial Sequence

<220>
<223>  anti-PD-L1 38 H2CDR1

<400>  22

Gly Phe Ile Ser Ser Arg Arg Cys Met Gly
1               5                   10

<210>  23
<211>  10
<212>  PRT
<213>  Artificial Sequence

<220>
<223>  anti-PD-L1 81 H2CDR1

<400>  23

Gly Asn Ile Ile Arg Val Arg Cys Met Ala
1               5                   10

<210>  24
<211>  10
<212>  PRT
<213>  Artificial Sequence

<220>
<223>  anti-PD-L1 87 H2CDR1

<400>  24

Gly Tyr Ile Phe Ser Ser Cys Gly Met Gly
1               5                   10

<210>  25
<211>  10
<212>  PRT
<213>  Artificial Sequence

<220>
<223>  anti-PD-L1 94 H2CDR1

<400>  25

Leu Asn Ile Phe Ser Ser Tyr Cys Met Gly
1               5                   10

<210>  26
<211>  10
<212>  PRT
<213>  Artificial Sequence

<220>
<223>  anti-PD-L1 69 H2CDR1

<400>  26

Arg Asn Ile Ser Ser Ser Tyr Cys Met Ala
1               5                   10

<210>  27
<211>  10
<212>  PRT
<213>  Artificial Sequence

<220>
<223>  Hu56V2   H2CDR1
Hu56V4  H2CDR1

<400>  27

Gly Phe Thr Phe Ser Arg Arg Cys Met Ala
1               5                   10

<210>  28
<211>  10
<212>  PRT
<213>  Artificial Sequence

<220>
<223>  anti-PD-L1 10 H2CDR1

<400>  28

Gly Asn Ile Val Ser Ser Tyr Cys Met Gly
1               5                   10

<210>  29

<211>  10
<212>  PRT
<213>  Artificial Sequence

<220>
<223>  Hu56V1¡¢anti-PD-L1 56¡¢Hu56V3¡¢Hu56V5¡¢anti-PD-L1 m3 H2CDR1

<400>  29

Gly Lys Met Ser Ser Arg Arg Cys Met Ala
1               5                   10


<210>  30
<211>  17
<212>  PRT
<213>  Artificial Sequence

<220>
<223>  anti-PD-L1 69    H2CDR2

<400>  30

Lys Ile Leu Thr Thr Pro Gly Asn Thr Tyr Leu Ala Asp Ser Val Lys
1               5                   10                  15


Gly



<210>  31
<211>  17
<212>  PRT
<213>  Artificial Sequence

<220>
<223>  anti-PD-L1 27 H2CDR2

<400>  31

Asn Ile Leu Thr Thr Thr Gly Asn Thr Tyr Leu Ala Asp Ser Val Lys
1               5                   10                  15


Gly



<210>  32
<211>  17
<212>  PRT
<213>  Artificial Sequence

<220>
<223>  anti-PD-L1 81 H2CDR2

<400>  32

Asn Ile Leu Thr Thr Thr Ile Ser Thr Tyr Leu Ala Asp Ser Val Lys
1               5                   10                  15

Gly

<210>  33
<211>  16
<212>  PRT
<213>  Artificial Sequence

<220>
<223>  anti-PD-L1 38 H2CDR2

<400>  33

Asn Ile Thr Gly Thr Gly Asn Thr Tyr Leu Ala Asp Ser Val Lys Gly
1               5                   10                  15

<210>  34
<211>  16
<212>  PRT
<213>  Artificial Sequence

<220>
<223>  anti-PD-L1 94 H2CDR2

<400>  34

Thr Ile Asp Ser Asp Gly Thr Thr Arg Tyr Val Asp Ser Val Lys Gly
1               5                   10                  15

<210>  35
<211>  16
<212>  PRT
<213>  Artificial Sequence

<220>
<223>  anti-PD-L1 87 H2CDR2

<400>  35

Thr Ile Ser Ser Asp Gly Thr Thr Ser Tyr Ala Asp Ser Val Lys Gly
1               5                   10                  15

<210>  36
<211>  17
<212>  PRT
<213>  Artificial Sequence

<220>
<223>  anti-PD-L1 56¡¢Hu56V1¡¢Hu56V2¡¢Hu56V3¡¢Hu56V4¡¢Hu56V5 H2CDR2

<400>  36

Lys Leu Leu Thr Thr Ser Gly Ser Thr Tyr Leu Ala Asp Ser Val Lys
1               5                   10                  15

Gly

<210>  37
<211>  16
<212>  PRT
<213>  Artificial Sequence

<220>
<223>  anti-PD-L1 10 H2CDR2

<400>  37

Ala Ile Asp Ser Asp Gly Thr Thr Lys Tyr Ala Asp Ser Met Lys Gly
1               5                   10                  15


<210>  38
<211>  17
<212>  PRT
<213>  Artificial Sequence

<220>
<223>  anti-PD-L1 m3 H2CDR2

<400>  38

Asp Val Asn Pro Asn Ser Gly Gly Ser Ile Tyr Asn Gln Arg Phe Lys
1               5                   10                  15


Gly




<210>  39
<211>  19
<212>  PRT
<213>  Artificial Sequence

<220>
<223>  anti-PD-L1 27 H2CDR3

<400>  39

Asp Ser Phe His Asp Pro Thr Cys Thr Val Val Ala Ser Ser Gly Ala
1               5                   10                  15


Phe Gln Tyr




<210>  40
<211>  17
<212>  PRT
<213>  Artificial Sequence

<220>
<223>  anti-PD-L1 38 H2CDR3

<400>  40

Asp Ser Phe Pro Thr Cys Thr Val Val Ala Ser Ser Gly Ala Phe Gln
1               5                   10                  15

Tyr

<210> 41
<211> 19
<212> PRT
<213> Artificial Sequence

<220>
<223> anti-PD-L1 69 H2CDR3

<400> 41

Asp Ser Phe Gln Lys Pro Thr Cys Thr Val Val Ala Ser Trp Pro Ala
1               5                   10                  15

Phe Gln Tyr

<210> 42
<211> 21
<212> PRT
<213> Artificial Sequence

<220>
<223> anti-PD-L1 87 H2CDR3

<400> 42

Asp Cys Pro Pro Ile Pro Glu Phe Thr Ser Cys Ser Gly Gly Tyr Cys
1               5                   10                  15

Leu Ser Gly Asp Tyr
            20

<210> 43
<211> 19
<212> PRT
<213> Artificial Sequence

<220>
<223> ANTI-PD-L1 81 H2CDR3

<400> 43

Asp Ser Phe Gly Tyr Pro Thr Cys Pro Gly Pro Ala Ser Ser Gly Ala
1               5                   10                  15

Phe Gln Tyr

<210> 44
<211> 16
<212> PRT

<213>  Artificial Sequence

<220>
<223>  anti-PD-L1 94¡¢anti-PD-L1 10 H2CDR3

<400>  44

Arg Leu Asn Cys Pro Gly Pro Val Asp Trp Val Pro Met Phe Pro Tyr
1               5                   10                  15


<210>  45
<211>  19
<212>  PRT
<213>  Artificial Sequence

<220>
<223>  anti-PD-L1 56¡¢Hu56V1¡¢Hu56V2¡¢Hu56V3¡¢Hu56V4¡¢Hu56V5¡¢anti-PD-L1
       m3 H2CDR3

<400>  45

Asp Ser Phe Glu Asp Pro Thr Cys Thr Leu Val Thr Ser Ser Gly Ala
1               5                   10                  15


Phe Gln Tyr



<210>  46
<211>  128
<212>  PRT
<213>  Artificial Sequence

<220>
<223>  anti-PD-L1 27 VH2

<400>  46

Gln Val Gln Leu Gln Glu Ser Gly Gly Gly Ser Val Gln Ala Gly Gly
1               5                   10                  15


Ser Leu Arg Leu Ser Cys Ala Ala Ser Gly Asn Ile Ser Ser Arg Arg
            20                  25                  30


Cys Met Ala Trp Phe Arg Gln Ala Pro Gly Lys Glu Arg Glu Arg Val
        35                  40                  45


Ala Asn Ile Leu Thr Thr Thr Gly Asn Thr Tyr Leu Ala Asp Ser Val
    50                  55                  60


Lys Gly Arg Phe Thr Ile Ser Gln Asn Asn Ala Lys Ser Thr Val Tyr
65                  70                  75                  80


Leu Gln Met Asn Ser Leu Lys Pro Glu Asp Thr Ala Met Tyr Tyr Cys
                85                  90                  95

Ala Ala Asp Ser Phe His Asp Pro Thr Cys Thr Val Val Ala Ser Ser
            100                 105                 110

Gly Ala Phe Gln Tyr Trp Gly Gln Gly Thr Gln Val Thr Val Ser Ser
        115                 120                 125

<210> 47
<211> 125
<212> PRT
<213> Artificial Sequence

<220>
<223> anti-PD-L1 38 VH2

<400> 47

Gln Val Gln Leu Gln Glu Ser Gly Gly Gly Ser Val Gln Ala Gly Gly
1               5                   10                  15

Ser Leu Arg Leu Ser Cys Ala Val Ser Gly Phe Ile Ser Ser Arg Arg
            20                  25                  30

Cys Met Gly Trp Phe Arg Gln Ala Pro Gly Lys Glu Arg Val Gly Val
        35                  40                  45

Ala Asn Ile Thr Gly Thr Gly Asn Thr Tyr Leu Ala Asp Ser Val Lys
    50                  55                  60

Gly Arg Phe Thr Ile Ser Gln Asn Asn Ala Lys Ser Thr Val Tyr Leu
65                  70                  75                  80

Gln Met Asn Ser Leu Lys Pro Glu Asp Thr Ala Met Tyr Tyr Cys Ala
                85                  90                  95

Ala Asp Ser Phe Pro Thr Cys Thr Val Val Ala Ser Ser Gly Ala Phe
            100                 105                 110

Gln Tyr Trp Gly Gln Gly Thr Gln Val Thr Val Ser Ser
        115                 120                 125

<210> 48
<211> 128
<212> PRT
<213> Artificial Sequence

<220>
<223> anti-PD-L1 56 VH2

<400> 48

Gln Val Gln Leu Gln Glu Ser Gly Gly Gly Leu Val Gln Pro Gly Gly
1               5                   10                  15

Ser Leu Arg Leu Ser Cys Ala Ala Ser Gly Lys Met Ser Ser Arg Arg
            20                  25                  30

Cys Met Ala Trp Phe Arg Gln Ala Pro Gly Lys Glu Arg Glu Arg Val
        35                  40                  45

Ala Lys Leu Leu Thr Thr Ser Gly Ser Thr Tyr Leu Ala Asp Ser Val
    50                  55                  60

Lys Gly Arg Phe Thr Ile Ser Gln Asn Asn Ala Lys Ser Thr Val Tyr
65                  70                  75                  80

Leu Gln Met Asn Ser Leu Lys Pro Glu Asp Thr Ala Met Tyr Tyr Cys
                85                  90                  95

Ala Ala Asp Ser Phe Glu Asp Pro Thr Cys Thr Leu Val Thr Ser Ser
            100                 105                 110

Gly Ala Phe Gln Tyr Trp Gly Gln Gly Thr Gln Val Thr Val Ser Ser
        115                 120                 125

<210> 49
<211> 128
<212> PRT
<213> Artificial Sequence

<220>
<223> anti-PD-L1 69 VH2

<400> 49

Gln Val Gln Leu Gln Glu Ser Gly Gly Gly Ser Val Gln Ala Gly Gly
1               5                   10                  15

Ser Leu Arg Leu Ser Cys Ala Val Gln Arg Asn Ile Ser Ser Ser Tyr
            20                  25                  30

Cys Met Ala Trp Phe Arg Gln Ala Pro Gly Lys Gln Arg Glu Arg Val
        35                  40                  45

Asp Lys Ile Leu Thr Thr Pro Gly Asn Thr Tyr Leu Ala Asp Ser Val
    50                  55                  60

Lys Gly Arg Phe Thr Ile Ser Gln Asn Asn Ala Lys Ser Thr Val Tyr
65                  70                  75                  80

Leu Gln Met Asn Ser Leu Lys Pro Glu Asp Thr Ala Met Tyr Tyr Cys
                85                  90                  95

Ala Ala Asp Ser Phe Gln Lys Pro Thr Cys Thr Val Val Ala Ser Trp
            100                 105                 110

Pro Ala Phe Gln Tyr Trp Gly Gln Gly Thr Gln Val Thr Val Ser Ser
        115                 120                 125

<210>  50
<211>  128
<212>  PRT
<213>  Artificial Sequence

<220>
<223>  anti-PD-L1 81 VH2

<400>  50

Gln Val Gln Leu Gln Glu Ser Gly Gly Gly Ser Val Gln Ala Gly Gly
1               5                   10                  15

Ser Leu Arg Leu Ser Cys Ala Ala Ser Gly Asn Ile Ile Arg Val Arg
            20                  25                  30

Cys Met Ala Trp Phe Arg Gln Ala Pro Gly Lys Glu Arg Glu Arg Gly
        35                  40                  45

Pro Asn Ile Leu Thr Thr Thr Ile Ser Thr Tyr Leu Ala Asp Ser Val
    50                  55                  60

Lys Gly Arg Phe Thr Ile Ser Gln Asn Asn Ala Lys Ser Thr Val Tyr
65                  70                  75                  80

Leu Gln Met Asn Ser Leu Lys Pro Glu Asp Thr Ala Met Tyr Tyr Cys
                85                  90                  95

Ala Ala Asp Ser Phe Gly Tyr Pro Thr Cys Pro Gly Pro Ala Ser Ser
            100                 105                 110

Gly Ala Phe Gln Tyr Trp Gly Gln Gly Thr Gln Val Thr Val Ser Ser
        115                 120                 125

<210>  51
<211>  129
<212>  PRT
<213>  Artificial Sequence

<220>
<223>  anti-PD-L1 87 VH2

<400>  51

Gln Val Gln Leu Gln Glu Ser Gly Gly Gly Ser Val Gln Ala Gly Gly
1               5                   10                  15

Ser Leu Lys Leu Ser Cys Ala Ala Ser Gly Tyr Ile Phe Ser Ser Cys
            20                  25                  30

Gly Met Gly Trp Tyr Arg Gln Ala Pro Gly Lys Glu Arg Glu Leu Val
        35                  40                  45

Ser Thr Ile Ser Ser Asp Gly Thr Thr Ser Tyr Ala Asp Ser Val Lys
    50                  55                  60

Gly Arg Phe Thr Ile Ser Gln Asp Asn Ala Lys Asn Thr Leu Tyr Leu
65                  70                  75                  80

Gln Met Asn Ser Leu Lys Thr Glu Asp Thr Ala Val Tyr Tyr Cys Val
                85                  90                  95

Ala Asp Cys Pro Pro Ile Pro Glu Phe Thr Ser Cys Ser Gly Gly Tyr
            100                 105                 110

Cys Leu Ser Gly Asp Tyr Trp Gly Gln Gly Thr Gln Val Thr Val Ser
        115                 120                 125

Ser

<210>  52
<211>  124
<212>  PRT
<213>  Artificial Sequence

<220>
<223>  anti-PD-L1 94 VH2

<400>  52

Gln Val Gln Leu Gln Glu Ser Gly Gly Gly Ser Val Gln Ala Gly Gly
1               5                   10                  15

Ser Leu Arg Leu Ser Cys Ala Ala Ser Leu Asn Ile Phe Ser Ser Tyr
            20                  25                  30

Cys Met Gly Trp Phe Arg Gln Ala Pro Gly Lys Gln Arg Val Gly Val
        35                  40                  45

Ala Thr Ile Asp Ser Asp Gly Thr Thr Arg Tyr Val Asp Ser Val Lys
    50                  55                  60

Gly Arg Phe Thr Ile Ser Lys Asp Asn Ala Lys Asn Thr Leu Asp Leu
65                  70                  75                  80

Gln Met Asn Ser Leu Lys Pro Glu Asp Thr Ala Met Tyr Tyr Cys Ala
                85                  90                  95

Ala Arg Leu Asn Cys Pro Gly Pro Val Asp Trp Val Pro Met Phe Pro
            100                 105                 110

Tyr Arg Gly Gln Gly Thr Gln Val Thr Val Ser Ser
        115                 120

<210>  53
<211>  128
<212>  PRT
<213>  Artificial Sequence

<220>
<223>  Hu56V1 VH2

<400>  53

Gln Val Gln Leu Val Glu Ser Gly Gly Gly Leu Val Gln Pro Gly Gly
1               5                   10                  15

Ser Leu Arg Leu Ser Cys Ala Ala Ser Gly Lys Met Ser Ser Arg Arg
            20                  25                  30

Cys Met Ala Trp Phe Arg Gln Ala Pro Gly Lys Glu Arg Glu Arg Val
        35                  40                  45

Ala Lys Leu Leu Thr Thr Ser Gly Ser Thr Tyr Leu Ala Asp Ser Val
    50                  55                  60

Lys Gly Arg Phe Thr Ile Ser Arg Asp Asn Ser Lys Asn Thr Val Tyr
65                  70                  75                  80

Leu Gln Met Asn Ser Leu Arg Ala Glu Asp Thr Ala Val Tyr Tyr Cys
                85                  90                  95

Ala Ala Asp Ser Phe Glu Asp Pro Thr Cys Thr Leu Val Thr Ser Ser
            100                 105                 110

Gly Ala Phe Gln Tyr Trp Gly Gln Gly Thr Leu Val Thr Val Ser Ser
        115                 120                 125

<210>  54
<211>  128
<212>  PRT
<213>  Artificial Sequence

<220>
<223>  Hu56V2 VH2

<400>  54

Gln Val Gln Leu Val Glu Ser Gly Gly Gly Leu Val Gln Pro Gly Gly
1               5                   10                  15

Ser Leu Arg Leu Ser Cys Ala Ala Ser Gly Phe Thr Phe Ser Arg Arg
            20                  25                  30

Cys Met Ala Trp Phe Arg Gln Ala Pro Gly Lys Glu Arg Glu Arg Val
        35                  40                  45

Ala Lys Leu Leu Thr Thr Ser Gly Ser Thr Tyr Leu Ala Asp Ser Val
    50                  55                  60

Lys Gly Arg Phe Thr Ile Ser Arg Asp Asn Ser Lys Asn Thr Val Tyr
65                  70                  75                  80

Leu Gln Met Asn Ser Leu Arg Ala Glu Asp Thr Ala Val Tyr Tyr Cys
                85                  90                  95

Ala Ala Asp Ser Phe Glu Asp Pro Thr Cys Thr Leu Val Thr Ser Ser
            100                 105                 110

Gly Ala Phe Gln Tyr Trp Gly Gln Gly Thr Leu Val Thr Val Ser Ser
        115                 120                 125


<210>  55
<211>  128
<212>  PRT
<213>  Artificial Sequence

<220>
<223>  Hu56V3 VH2

<400>  55

Gln Val Gln Leu Val Glu Ser Gly Gly Gly Leu Val Gln Pro Gly Gly
1               5                   10                  15

Ser Leu Arg Leu Ser Cys Ala Ala Ser Gly Lys Met Ser Ser Arg Arg
            20                  25                  30

Cys Met Ala Trp Phe Arg Gln Ala Pro Gly Lys Gly Leu Glu Arg Val
        35                  40                  45

Ala Lys Leu Leu Thr Thr Ser Gly Ser Thr Tyr Leu Ala Asp Ser Val
    50                  55                  60

Lys Gly Arg Phe Thr Ile Ser Arg Asp Asn Ser Lys Asn Thr Val Tyr
65                  70                  75                  80

Leu Gln Met Asn Ser Leu Arg Ala Glu Asp Thr Ala Val Tyr Tyr Cys
                85                  90                  95

Ala Ala Asp Ser Phe Glu Asp Pro Thr Cys Thr Leu Val Thr Ser Ser
            100                 105                 110

Gly Ala Phe Gln Tyr Trp Gly Gln Gly Thr Leu Val Thr Val Ser Ser
        115                 120                 125

<210>  56
<211>  128
<212>  PRT
<213>  Artificial Sequence

<220>
<223>  Hu56V4 VH2

<400>  56

Gln Val Gln Leu Val Glu Ser Gly Gly Gly Leu Val Gln Pro Gly Gly
1               5                   10                  15

Ser Leu Arg Leu Ser Cys Ala Ala Ser Gly Phe Thr Phe Ser Arg Arg
            20                  25                  30

Cys Met Ala Trp Phe Arg Gln Ala Pro Gly Lys Gly Leu Glu Arg Val
        35                  40                  45

Ala Lys Leu Leu Thr Thr Ser Gly Ser Thr Tyr Leu Ala Asp Ser Val
    50                  55                  60

Lys Gly Arg Phe Thr Ile Ser Arg Asp Asn Ser Lys Asn Thr Val Tyr
65                  70                  75                  80

Leu Gln Met Asn Ser Leu Arg Ala Glu Asp Thr Ala Val Tyr Tyr Cys
                85                  90                  95

Ala Ala Asp Ser Phe Glu Asp Pro Thr Cys Thr Leu Val Thr Ser Ser
            100                 105                 110

Gly Ala Phe Gln Tyr Trp Gly Gln Gly Thr Leu Val Thr Val Ser Ser
        115                 120                 125

<210>  57
<211>  128
<212>  PRT
<213>  Artificial Sequence

<220>
<223>  Hu56V5 VH2

<400>  57

Gln Val Gln Leu Val Glu Ser Gly Gly Gly Leu Val Gln Pro Gly Gly
1               5                   10                  15

Ser Leu Arg Leu Ser Cys Ala Ala Ser Gly Lys Met Ser Ser Arg Arg
            20                  25                  30

Cys Met Ala Trp Phe Arg Gln Ala Pro Gly Lys Glu Arg Glu Arg Val
        35                  40                  45

Ala Lys Leu Leu Thr Thr Ser Gly Ser Thr Tyr Leu Ala Asp Ser Val
    50                  55                  60

Lys Gly Arg Phe Thr Ile Ser Arg Asp Asn Ser Lys Asn Thr Val Tyr
65                  70                  75                  80

Leu Gln Met Asn Ser Leu Lys Ala Glu Asp Thr Ala Val Tyr Tyr Cys
                85                  90                  95

Ala Ala Asp Ser Phe Glu Asp Pro Thr Cys Thr Leu Val Thr Ser Ser
            100                 105                 110

Gly Ala Phe Gln Tyr Trp Gly Gln Gly Thr Leu Val Thr Val Ser Ser
        115                 120                 125


<210>  58
<211>  124
<212>  PRT
<213>  Artificial Sequence

<220>
<223>  anti-PD-L1 10 VH2

<400>  58

Gln Val Gln Leu Gln Glu Ser Gly Gly Gly Ser Val Gln Ala Gly Gly
1               5                   10                  15

Ser Leu Arg Leu Ser Cys Ala Ala Ser Gly Asn Ile Val Ser Ser Tyr
            20                  25                  30

Cys Met Gly Trp Phe Arg Gln Ala Pro Gly Lys Glu Arg Val Gly Val
        35                  40                  45

Ala Ala Ile Asp Ser Asp Gly Thr Thr Lys Tyr Ala Asp Ser Met Lys
    50                  55                  60

Gly Arg Phe Thr Ile Ser Lys Asp Asn Ala Lys Asn Thr Leu Asp Leu
65                  70                  75                  80

Gln Met Asn Ser Leu Lys Pro Glu Asp Thr Ala Met Tyr Tyr Cys Val
                85                  90                  95

Ala Arg Leu Asn Cys Pro Gly Pro Val Asp Trp Val Pro Met Phe Pro
            100                 105                 110

Tyr Arg Gly Gln Gly Thr Gln Val Thr Val Ser Ser
        115                 120

<210>  59
<211>  128
<212>  PRT
<213>  Artificial Sequence

<220>
<223>  anti-PD-L1 m3 VH2

<400>  59

Gln Val Gln Leu Gln Glu Ser Gly Gly Gly Leu Val Gln Pro Gly Gly
1               5                   10                  15

Ser Leu Arg Leu Ser Cys Ala Ala Ser Gly Lys Met Ser Ser Arg Arg
            20                  25                  30

Cys Met Ala Trp Phe Arg Gln Ala Pro Gly Lys Glu Arg Glu Arg Val
        35                  40                  45

Ala Asp Val Asn Pro Asn Ser Gly Gly Ser Ile Tyr Asn Gln Arg Phe
    50                  55                  60

Lys Gly Arg Phe Thr Ile Ser Gln Asn Asn Ala Lys Ser Thr Val Tyr
65                  70                  75                  80

Leu Gln Met Asn Ser Leu Lys Pro Glu Asp Thr Ala Met Tyr Tyr Cys
                85                  90                  95

Ala Ala Asp Ser Phe Glu Asp Pro Thr Cys Thr Leu Val Thr Ser Ser
            100                 105                 110

Gly Ala Phe Gln Tyr Trp Gly Gln Gly Thr Gln Val Thr Val Ser Ser
        115                 120                 125

<210>  60
<211>  10
<212>  PRT
<213>  Artificial Sequence

<220>
<223>  H2CDR1 general formula

<220>
<221> X
<222> (1)..(1)
<223> X=G,L or R

<220>
<221> X
<222> (2)..(2)
<223> X=N,F,Y or K

<220>
<221> X
<222> (3)..(3)
<223> X=I,T or M

<220>
<221> X
<222> (4)..(4)
<223> X=S,I,F or V

<220>
<221> X
<222> (5)..(5)
<223> X=S or R

<220>
<221> X
<222> (6)..(6)
<223> X=R,S or V

<220>
<221> X
<222> (7)..(7)
<223> X=R,C or Y

<220>
<221> X
<222> (8)..(8)
<223> X=C or G

<220>
<221> X
<222> (10)..(10)
<223> X=A or G

<400> 60

Xaa Xaa Xaa Xaa Xaa Xaa Xaa Xaa Met Xaa
1               5                   10


<210> 61
<211> 17
<212> PRT
<213> Artificial Sequence

<220>
<223> H2CDR2 general formula


<220>
<221> X

<222>  (1)..(1)
<223>  X=K,N,T,D or A

<220>
<221>  X
<222>  (2)..(2)
<223>  X=I, L or V

<220>
<221>  X
<222>  (3)..(3)
<223>  X=L,T,D,S or N

<220>
<221>  X
<222>  (4)..(4)
<223>  X=T£¬G£¬S or P

<220>
<221>  X
<222>  (5)..(5)
<223>  X=T,D or N

<220>
<221>  X
<222>  (6)..(6)
<223>  X=P,T,S or on amino acid

<220>
<221>  X
<222>  (7)..(7)
<223>  X=G or I

<220>
<221>  X
<222>  (8)..(8)
<223>  X=N,S,T or G

<220>
<221>  X
<222>  (9)..(9)
<223>  X=T,S or no amino acid

<220>
<221>  X
<222>  (10)..(10)
<223>  X=Y,R,S,K or I

<220>
<221>  X
<222>  (11)..(11)
<223>  X=L or Y

<220>
<221>  X
<222>  (12)..(12)
<223>  X=A,V or N

<220>
<221>  X
<222>  (13)..(13)
<223>  X=D or Q

<220>
<221>  X
<222>  (14)..(14)
<223>  X=S or R

<220>
<221>  X
<222>  (15)..(15)
<223>  X=V,M or F

<400>  61

Xaa Xaa Xaa Xaa Xaa Xaa Xaa Xaa Xaa Xaa Xaa Xaa Xaa Xaa Xaa Lys
1               5                   10                  15

Gly

<210>  62
<211>  21
<212>  PRT
<213>  Artificial Sequence

<220>
<223>  H2CDR3 general formula

<220>
<221>  X
<222>  (1)..(1)
<223>  X=D or R

<220>
<221>  X
<222>  (2)..(2)
<223>  X=S£¬C or L

<220>
<221>  X
<222>  (3)..(3)
<223>  X=F£¬P or N

<220>
<221>  X
<222>  (4)..(4)
<223>  X=E£¬C£¬G£¬P£¬Q or H

<220>
<221>  X
<222>  (5)..(5)
<223>  X=D£¬T£¬K£¬I£¬Y or P

<220>
<221>  X
<222>  (6)..(6)
<223>  X=P£¬C or G

<220>
<221>  X
<222>  (7)..(7)
<223>  X=T£¬E or P

<220>
<221>  X
<222>  (8)..(8)
<223>  X=C£¬F£¬V or no amino acid

<220>
<221>  X
<222>  (9)..(9)
<223>  X=T£¬P£¬D or no amino acid

<220>
<221>  X
<222>  (10)..(10)
<223>  X=L£¬S£¬G£¬W or no amino acid

<220>
<221>  X
<222>  (11)..(11)
<223>  X=V£¬C£¬P or no amino acid

<220>
<221>  X
<222>  (12)..(12)
<223>  X=T£¬V£¬S or no amino acid

<220>
<221>  X
<222>  (13)..(13)
<223>  X=V£¬G or no amino acid

<220>
<221>  X
<222>  (14)..(14)
<223>  X=A£¬G or no amino acid

<220>
<221>  X
<222>  (15)..(15)
<223>  X=S£¬Y or no amino acid

<220>
<221>  X
<222>  (16)..(16)
<223>  X=S£¬W£¬C or no amino acid

<220>
<221>  X
<222>  (17)..(17)
<223>  X=G£¬P or L

<220>
<221>  X
<222>  (18)..(18)
<223>  X=A£¬S or M

<220>
<221>  X
<222>  (19)..(19)
<223>  X=F or G

<220>
<221>  X

<222>  (20)..(20)
<223>  X=Q£¬D or P

<400>  62

Xaa Xaa Xaa Xaa Xaa Xaa Xaa Xaa Xaa Xaa Xaa Xaa Xaa Xaa Xaa Xaa
1               5                   10                  15

Xaa Xaa Xaa Xaa Tyr
            20

<210>  63
<211>  129
<212>  PRT
<213>  Artificial Sequence

<220>
<223>  VH2 general formula

<220>
<221>  X
<222>  (5)..(5)
<223>  X=V or Q

<220>
<221>  X
<222>  (11)..(11)
<223>  X=L or S

<220>
<221>  X
<222>  (14)..(14)
<223>  X=P or A

<220>
<221>  X
<222>  (24)..(24)
<223>  X=A or V

<220>
<221>  X
<222>  (25)..(25)
<223>  X=S or Q

<220>
<221>  X
<222>  (26)..(26)
<223>  X=G£¬L or R

<220>
<221>  X
<222>  (27)..(27)
<223>  X=N£¬F£¬Y or K

<220>
<221>  X
<222>  (28)..(28)
<223>  X=I£¬T or M

<220>

<221>  X
<222>  (29)..(29)
<223>  X=S£¬I£¬F or V

<220>
<221>  X
<222>  (30)..(30)
<223>  X=S or R

<220>
<221>  X
<222>  (31)..(31)
<223>  X=R£¬S or V

<220>
<221>  X
<222>  (32)..(32)
<223>  X=R£¬C or Y

<220>
<221>  X
<222>  (33)..(33)
<223>  X=C or G

<220>
<221>  X
<222>  (35)..(35)
<223>  X=A or G

<220>
<221>  X
<222>  (36)..(36)
<223>  X=F or Y

<220>
<221>  X
<222>  (43)..(43)
<223>  X=E£¬Q or G

<220>
<221>  X
<222>  (44)..(44)
<223>  X=R or L

<220>
<221>  X
<222>  (45)..(45)
<223>  X=E or V

<220>
<221>  X
<222>  (46)..(46)
<223>  X=R£¬G or L

<220>
<221>  X
<222>  (47)..(47)
<223>  X=V or G

<220>
<221>  X
<222>  (48)..(48)
<223>  X=A£¬D£¬P or S

<220>
<221>  X
<222>  (49)..(49)
<223>  X=K£¬N£¬T£¬D or A

<220>
<221>  X
<222>  (50)..(50)
<223>  X=I£¬L or V

<220>
<221>  X
<222>  (51)..(51)
<223>  X=L£¬T£¬D£¬S or N

<220>
<221>  X
<222>  (52)..(52)
<223>  X=T£¬G£¬S or P

<220>
<221>  X
<222>  (53)..(53)
<223>  X=T£¬D or N

<220>
<221>  X
<222>  (54)..(54)
<223>  X=P£¬T£¬S or no amino acid

<220>
<221>  X
<222>  (55)..(55)
<223>  X=G or I£»X29=N£¬S£¬T or G

<220>
<221>  X
<222>  (56)..(56)
<223>  X=N£¬S£¬T or G

<220>
<221>  X
<222>  (57)..(57)
<223>  X=T£¬S or no amino acid

<220>
<221>  X
<222>  (58)..(58)
<223>  X=Y£¬R£¬S£¬K or I£»

<220>
<221>  X
<222>  (59)..(59)
<223>  X=L or Y

<220>
<221>  X
<222>  (60)..(60)
<223>  X=A£¬V or N

<220>
<221>  X

<222>  (61)..(61)
<223>  X=D or Q

<220>
<221>  X
<222>  (62)..(62)
<223>  X=S or R

<220>
<221>  X
<222>  (63)..(63)
<223>  X=V£¬M or F

<220>
<221>  X
<222>  (71)..(71)
<223>  X=R£¬K or Q

<220>
<221>  X
<222>  (72)..(72)
<223>  X=D or N

<220>
<221>  X
<222>  (74)..(74)
<223>  X=S or A

<220>
<221>  X
<222>  (76)..(76)
<223>  X=N or S

<220>
<221>  X
<222>  (78)..(78)
<223>  X=V or L

<220>
<221>  X
<222>  (79)..(79)
<223>  X=Y or D

<220>
<221>  X
<222>  (86)..(86)
<223>  X=R or K

<220>
<221>  X
<222>  (87)..(87)
<223>  X=A£¬T or P

<220>
<221>  X
<222>  (92)..(92)
<223>  X=V or M

<220>
<221>  X
<222>  (96)..(96)
<223>  X=A or V

<220>
<221>  X
<222>  (98)..(98)
<223>  X=D or R

<220>
<221>  X
<222>  (99)..(99)
<223>  X=S£¬C or L

<220>
<221>  X
<222>  (100)..(100)
<223>  X=F£¬P or N

<220>
<221>  X
<222>  (101)..(101)
<223>  X=E£¬C£¬G£¬P£¬Q or H

<220>
<221>  X
<222>  (102)..(102)
<223>  X=D£¬T£¬K£¬I£¬Y or P

<220>
<221>  X
<222>  (103)..(103)
<223>  X=P£¬C or G

<220>
<221>  X
<222>  (104)..(104)
<223>  X=T£¬E or P

<220>
<221>  X
<222>  (105)..(105)
<223>  X=C£¬F£¬V or no amino acid

<220>
<221>  X
<222>  (106)..(106)
<223>  X=T£¬P£¬D or no amino acid

<220>
<221>  X
<222>  (107)..(107)
<223>  X=L£¬S£¬G£¬W or no amino acid

<220>
<221>  X
<222>  (108)..(108)
<223>  X=V£¬C£¬P or no amino acid

<220>
<221>  X
<222>  (109)..(109)
<223>  X=T£¬V£¬S or no amino acid

<220>
<221>  X
<222>  (110)..(110)

<223>  X=V£¬G or no amino acid

<220>
<221>  X
<222>  (111)..(111)
<223>  X=A£¬G or no amino acid

<220>
<221>  X
<222>  (112)..(112)
<223>  X=S£¬Y or no amino acid

<220>
<221>  X
<222>  (113)..(113)
<223>  X=S£¬W£¬C or no amino acid

<220>
<221>  X
<222>  (114)..(114)
<223>  X=G£¬P or L

<220>
<221>  X
<222>  (115)..(115)
<223>  X=A£¬S or M

<220>
<221>  X
<222>  (116)..(116)
<223>  X=F or G

<220>
<221>  X
<222>  (117)..(117)
<223>  X=Q£¬D or P

<220>
<221>  X
<222>  (119)..(119)
<223>  X=W or R

<220>
<221>  X
<222>  (124)..(124)

<220>
<221>  X
<222>  (124)..(124)
<223>  X=L or Q

<400>  63

Gln Val Gln Leu Xaa Glu Ser Gly Gly Gly Xaa Val Gln Xaa Gly Gly
1               5                   10                  15

Ser Leu Arg Leu Ser Cys Ala Xaa Xaa Xaa Xaa Xaa Xaa Xaa Xaa Xaa
            20                  25                  30

Xaa Met Xaa Xaa Arg Gln Ala Pro Gly Lys Xaa Xaa Xaa Xaa Xaa Xaa
        35                  40                  45

Xaa Xaa Xaa Xaa Xaa Xaa Xaa Xaa Xaa Xaa Xaa Xaa Xaa Xaa Xaa Lys
    50                  55                  60

Gly Arg Phe Thr Ile Ser Xaa Xaa Asn Xaa Lys Xaa Thr Xaa Xaa Leu
65                  70                  75                  80

Gln Met Asn Ser Leu Xaa Xaa Glu Asp Thr Ala Xaa Tyr Tyr Cys Xaa
                85                  90                  95

Ala Xaa Xaa Xaa Xaa Xaa Xaa Xaa Xaa Xaa Xaa Xaa Xaa Xaa Xaa Xaa
            100                 105                 110

Xaa Xaa Xaa Xaa Xaa Tyr Xaa Gly Gln Gly Thr Xaa Val Thr Val Ser
        115                 120                 125

Ser

<210>  64
<211>  360
<212>  PRT
<213>  Artificial Sequence

<220>
<223>  KN035

<400>  64

Gln Val Gln Leu Val Glu Ser Gly Gly Gly Leu Val Gln Pro Gly Gly
1               5                   10                  15

Ser Leu Arg Leu Ser Cys Ala Ala Ser Gly Lys Met Ser Ser Arg Arg
            20                  25                  30

Cys Met Ala Trp Phe Arg Gln Ala Pro Gly Lys Glu Arg Glu Arg Val
        35                  40                  45

Ala Lys Leu Leu Thr Thr Ser Gly Ser Thr Tyr Leu Ala Asp Ser Val
    50                  55                  60

Lys Gly Arg Phe Thr Ile Ser Arg Asp Asn Ser Lys Asn Thr Val Tyr
65                  70                  75                  80

Leu Gln Met Asn Ser Leu Arg Ala Glu Asp Thr Ala Val Tyr Tyr Cys
                85                  90                  95

Ala Ala Asp Ser Phe Glu Asp Pro Thr Cys Thr Leu Val Thr Ser Ser
            100                 105                 110

Gly Ala Phe Gln Tyr Trp Gly Gln Gly Thr Leu Val Thr Val Ser Ser
        115                 120                 125

Glu Pro Lys Ser Ser Asp Lys Thr His Thr Cys Pro Pro Cys Pro Ala
    130                 135                 140

Pro Glu Leu Leu Gly Gly Pro Ser Val Phe Leu Phe Pro Pro Lys Pro
145                 150                 155                 160

Lys Asp Thr Leu Met Ile Ser Arg Thr Pro Glu Val Thr Cys Val Val
                165                 170                 175

Val Asp Val Ser His Glu Asp Pro Glu Val Lys Phe Asn Trp Tyr Val
            180                 185                 190

Asp Gly Val Glu Val His Asn Ala Lys Thr Lys Pro Arg Glu Glu Gln
        195                 200                 205

Tyr Asn Ser Thr Tyr Arg Val Val Ser Val Leu Thr Val Leu His Gln
    210                 215                 220

Asp Trp Leu Asn Gly Lys Glu Tyr Lys Cys Lys Val Ser Asn Lys Ala
225                 230                 235                 240

Leu Pro Ala Pro Ile Glu Lys Thr Ile Ser Lys Ala Lys Gly Gln Pro
                245                 250                 255

Arg Glu Pro Gln Val Tyr Thr Leu Pro Pro Ser Arg Asp Glu Leu Thr
            260                 265                 270

Lys Asn Gln Val Ser Leu Thr Cys Leu Val Lys Gly Phe Tyr Pro Ser
        275                 280                 285

Asp Ile Ala Val Glu Trp Glu Ser Asn Gly Gln Pro Glu Asn Asn Tyr
    290                 295                 300

Lys Thr Thr Pro Pro Val Leu Asp Ser Asp Gly Ser Phe Phe Leu Tyr
305                 310                 315                 320

Ser Lys Leu Thr Val Asp Lys Ser Arg Trp Gln Gln Gly Asn Val Phe
                325                 330                 335

Ser Cys Ser Val Met His Glu Ala Leu His Asn His Tyr Thr Gln Lys
            340                 345                 350

Ser Leu Ser Leu Ser Pro Gly Lys
        355                 360